(19) Europäisches Patentamt
European Patent Office
Office européen des brevets

(11) **EP 4 736 896 A1**

(12) **EUROPEAN PATENT APPLICATION**
published in accordance with Art. 153(4) EPC

(43) Date of publication:
**06.05.2026 Bulletin 2026/19**

(21) Application number: **24832076.4**

(22) Date of filing: **28.06.2024**

(51) International Patent Classification (IPC):
***A61L 27/40*** (2006.01) ***A61K 9/70*** (2006.01)
***A61K 35/39*** (2015.01) ***A61K 47/32*** (2006.01)
***A61K 47/38*** (2006.01) ***A61L 27/20*** (2006.01)
***A61L 27/38*** (2006.01) ***A61L 27/52*** (2006.01)
***A61L 27/54*** (2006.01) ***A61P 3/10*** (2006.01)

(52) Cooperative Patent Classification (CPC):
**A61K 9/70; A61K 35/39; A61K 47/32; A61K 47/38; A61L 27/20; A61L 27/38; A61L 27/40; A61L 27/52; A61L 27/54; A61P 3/10**

(86) International application number:
**PCT/JP2024/023458**

(87) International publication number:
**WO 2025/005218 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.06.2023 JP 2023107390**

(71) Applicant: **Mochida Pharmaceutical Co., Ltd.**
**Tokyo 160-8515 (JP)**

(72) Inventors:
- **TSUDA Naoto**
  **Tokyo 160-8515 (JP)**
- **NAKATSUKA Shuji**
  **Tokyo 108-8230 (JP)**

(74) Representative: **Grünecker Patent- und Rechtsanwälte PartG mbB**
**Leopoldstraße 4**
**80802 München (DE)**

(54) **BIOCOMPATIBLE DEVICE**

(57) The present invention is a biocompatible device comprising an internal space configured to accommodate cells or tissues that secrete a biologically active substance, wherein the internal space is formed by disposing a biocompatible composite membrane including a semi-permeable membrane layer and a non-woven fabric layer in the device such that the semi-permeable membrane layer is on the outer side and the non-woven fabric layer is on the internal-space side, the semi-permeable membrane layer comprises a cellulose derivative laminated on a non-woven fabric using the non-woven fabric as a support, and the non-woven fabric layer comprises a thermoplastic non-woven fabric.

Fig. 3

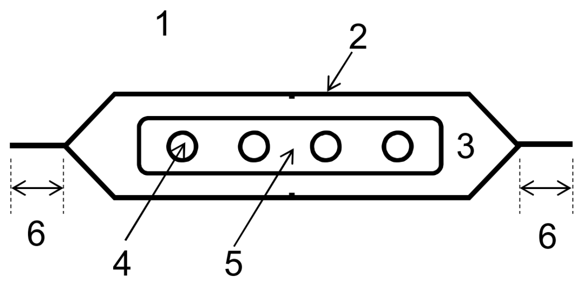


EP 4 736 896 A1

## Description

### Technical Field

The present invention relates to a biocompatible device for transplanting a cell or the like into a living body and a production method therefor, and a kit for living-donor transplantation.

### Background Art

A therapeutic method of transplanting a cell or tissue into a living body is known as one of regenerative therapies for organs with functional disorder, and devices to be used for the therapy have been developed. For example, a technique of a bioartificial pancreas (BAP, also referred to as a bioartificial pancreatic islet), which includes a pancreatic islet covered with (encapsulated in) a polymeric gel, semi-permeable membrane, or the like that enables isolation from immunocytes and the like of a recipient and is permeable to nutritional components, insulin, and others, and is transplanted into a living body, has been examined as a device for treating type 1 diabetes mellitus for some time, and various devices for transplantation have been reported (Patent Literatures 1 to 4).

In addition, biocompatible devices for transplantation that are used in therapeutic applications for a disease other than type 1 diabetes mellitus have been reported (Patent Literatures 5 to 7).

An immunoisolation device with a membrane produced with polypropylene fiber and ethylene-vinyl alcohol copolymer has been reported (Patent Literature 8).

Devices for transplantation with chemically cross-linked alginic acid have been reported (Patent Literatures 9, 10).

### Citation List

#### Patent Literature

Patent Literature 1: Japanese Translation of PCT International Application Publication No. 2022-502458

Patent Literature 2: Japanese Patent Laid-Open No. 2019-097442

Patent Literature 3: International Publication No. WO 2016/184872

Patent Literature 4: Japanese Translation of PCT International Application Publication No. 2017-502008

Patent Literature 5: Japanese Translation of PCT International Application Publication No. 2018-515582

Patent Literature 6: Japanese Patent Laid-Open No. 2014-200199

Patent Literature 7: Japanese Patent Laid-Open No. 2004-209228

Patent Literature 8: International Publication No. WO 2022/092198

Patent Literature 9: International Publication No. WO 2020/262642

Patent Literature 10: International Publication No. WO 2022/137345

### Summary of Invention

#### Technical Problem

In the circumstances as described above, a novel, practical biocompatible device or the like has been needed.

#### Solution to Problem

The present invention has the following features of (1) to (5).

(1) The present invention enables production of a biocompatible composite membrane including a semi-permeable membrane layer and a non-woven fabric layer, wherein the semi-permeable membrane layer comprises a cellulose derivative laminated on a non-woven fabric using the non-woven fabric as a support, and the non-woven fabric layer comprises a thermoplastic non-woven fabric.
(2) Use of the composite membrane enables enhancement of membrane strength in covering a hydrogel configured to accommodate a cell, tissue, or the like that secretes a biologically active substance.
(3) The configuration in which the non-woven fabric surface of the composite membrane can be fused enables production of a device by heat sealing in a simple manner.
(4) In transplanting a device produced with the composite membrane, improved adhesion rates in living bodies and improved cure rates in transplantation subjects are expected.
(5) Disposing the non-woven fabric surface of the composite membrane on the inner side allows a hydrogel that is included in an internal space of the device and configured to accommodate a cell, tissue, or the like that secretes a biologically active substance to be fixed on the non-woven fabric surface of the composite membrane, preventing reduction in the survival rate of the cell or the like.

More specifically, the present invention relates to: a biocompatible device comprising an internal space configured to accommodate a cell or tissue that secretes a biologically active substance, wherein the internal space is formed by disposing a biocompatible composite membrane including a semi-permeable membrane layer and a non-woven fabric layer in the device such that the semi-permeable membrane layer is on an outer side and the non-woven fabric layer is on an internal-space side, the semi-permeable membrane layer comprises a cellulose derivative laminated on a non-woven fabric using the non-woven fabric as a support, and the non-woven fabric layer comprises a thermoplastic non-woven fabric; a production method therefor; and others.

Exemplary embodiments of the present invention are shown as [1-1] to [4A-3] in the following.

[1-1] A biocompatible device having an internal space formed by a biocompatible composite membrane including a semi-permeable membrane layer and a non-woven fabric layer, wherein

the semi-permeable membrane layer comprises a cellulose derivative formed on a non-woven fabric serving as a support and the non-woven fabric layer comprises the thermoplastic non-woven fabric,
the semi-permeable membrane layer is disposed on an outer side and the non-woven fabric layer is disposed on an internal-space side in the device, and
the internal space includes a cell or tissue that secretes a biologically active substance.

[1-2] The device according to [1-1], having immunoisolatability.
[1-3] The device according to [1-1] or [1-2], wherein the cellulose derivative is cellulose acetate.
[1-4] The device according to any one of [1-1] to [1-3], wherein the non-woven fabric comprises one or more resins selected from the group consisting of polyethylene, polypropylene, and polyethylene terephthalate.
[1-5] The device according to any one of [1-1] to [1-4], wherein the internal space is formed by partial fusion of the non-woven fabric layer.
[1-6] The device according to any one of [1-1] to [1-5], wherein the composite membrane has a structure with partial intrusion of the cellulose derivative into the non-woven fabric.
[1-7] The device according to any one of [1-1] to [1-6], wherein the cell or tissue is enclosed in the internal space with a hydrogel serving as a carrier.
[1-8] The device according to any one of [1-1] to [1-7], wherein the cell is an insulin-secreting cell or a pancreatic islet.
[1-9] The device according to any one of [1-1] to [1-8], wherein the composite membrane has a thickness of 10 to 1500 μm.
[1-10] The device according to any one of [1-1] to [1-9], wherein the composite membrane has a strength of 1 MPa or more as measured in tensile test in accordance with JIS K 7127.
[1-11] The device according to any one of [1-1] to [1-10], for use in living-donor transplantation.
[1-12] A biocompatible composite membrane for producing the device according to any one of [1-1] to [1-11].
[1-13] A kit for living-donor transplantation, the kit comprising: a biocompatible device having an internal space formed by a biocompatible composite membrane including a semi-permeable membrane layer and a non-woven fabric layer; and a container including a cell or tissue that secretes a biologically active substance, wherein the kit is used in such a manner that the cell or tissue is enclosed in the internal space.
[1-14] A method of producing a biocompatible device, the method comprising a step of covering a hydrogel embedding a cell or tissue that secretes a biologically active substance with a biocompatible composite membrane including a semi-permeable membrane layer and a non-woven fabric layer, wherein, in the step, the semi-permeable membrane layer is disposed on an outer side and the non-woven fabric layer is disposed on a hydrogel side.

[1-15] A method of producing a biocompatible device, the method comprising a step of injecting a solution comprising a cell or tissue that secretes a biologically active substance and is mixed with a solution capable of hydrogelation into an internal space formed by a biocompatible composite membrane including a semi-permeable membrane layer and a non-woven fabric layer and gelling the solution capable of hydrogelation, wherein, in the step, the semi-permeable membrane layer is on an outer side and the non-woven fabric layer is on an internal-space side in the composite membrane.

[1A-1-1] A biocompatible device comprising an internal space configured to accommodate a cell or tissue that secretes a biologically active substance, wherein

the internal space is formed by disposing a biocompatible composite membrane including a semi-permeable membrane layer and a non-woven fabric layer in the device such that the semi-permeable membrane layer is on an outer side and the non-woven fabric layer is on an internal-space side,
the semi-permeable membrane layer comprises a cellulose derivative laminated on a non-woven fabric using the non-woven fabric as a support, and
the non-woven fabric layer comprises a thermoplastic non-woven fabric.

[1A-1-2] A biocompatible device comprising an internal space configured to accommodate a cell or tissue that secretes a biologically active substance, wherein

the internal space is formed by disposing a biocompatible composite membrane consisting of a semi-permeable membrane layer and a non-woven fabric layer in the device such that the semi-permeable membrane layer is on an outer side and the non-woven fabric layer is on an internal-space side,
the semi-permeable membrane layer comprises a cellulose derivative laminated on a non-woven fabric using the non-woven fabric as a support, and
the non-woven fabric layer comprises a thermoplastic non-woven fabric.

[1A-1-3] A biocompatible device comprising an internal space configured to accommodate a cell or tissue that secretes a biologically active substance, wherein
the internal space is formed by disposing a biocompatible composite membrane having a cellulose derivative laminated on one surface of a non-woven fabric in the device such that the surface of the composite membrane with the cellulose derivative laminated thereon is on an outer side.

[1A-2] The device according to any one of [1A-1-1] to [1A-1-3], having immunoisolatability.

[1A-3] The device according to any one of [1A-1-1] to [1A-2], wherein the cellulose derivative is cellulose acetate.

[1A-4] The device according to any one of [1A-1-1] to [1A-3], wherein the non-woven fabric comprises one or more resins selected from the group consisting of polyethylene (PE), polypropylene (PP), and polyethylene terephthalate (PET).

[1A-5] The device according to any one of [1A-1-1] to [1A-4], wherein the internal space is formed by partial fusion of a surface of the non-woven fabric layer that is free of cellulose derivative.

[1A-6] The device according to any one of [1A-1-1] to [1A-5], wherein the composite membrane has a structure with partial intrusion of the cellulose derivative into the non-woven fabric.

[1A-7] The biocompatible device according to any one of [1A-1-1] to [1A-6], wherein the cell that secretes a biologically active substance is an insulin-secreting cell or a pancreatic islet.

[1A-8] The device according to any one of [1A-1-1] to [1A-7], wherein the cell or tissue that secretes a biologically active substance is enclosed in the internal space with a hydrogel serving as a carrier.

[1A-9] The biocompatible device according to [1A-8], wherein the hydrogel is a hydrogel formed by gelation of one or more selected from the group consisting of alginic acid, collagen, hyaluronan, gelatin, fibronectin, elastin, tenascin, laminin, vitronectin, polypeptide, heparan sulfate, chondroitin, chondroitin sulfate, keratan, keratan sulfate, dermatan sulfate, carrageenan, heparin, chitin, chitosan, agarose, agar, cellulose, methylcellulose, carboxylmethylcellulose, glycogen, and derivatives of these, and fibrin, fibrinogen, thrombin, polyglutamic acid, polylactic acid, polyglycolic acid, lactic acid-glycolic acid copolymer, gellan gum, xanthan gum, galactomannan, guar gum, locust bean gum, and tara gum.

[1A-9-1] The biocompatible device according to [1A-8], wherein the hydrogel is a hydrogel formed by gelation of one or more selected from the group consisting of alginic acid, collagen, hyaluronan, gelatin, fibronectin, elastin, tenascin, laminin, vitronectin, polypeptide, heparan sulfate, chondroitin, chondroitin sulfate, keratan, keratan sulfate, dermatan sulfate, carrageenan, heparin, chitin, chitosan, agarose, agar, cellulose, methylcellulose, carboxylmethylcellulose, glycogen, and derivatives of these.

[1A-9-2] The biocompatible device according to [1A-8], wherein the hydrogel is a hydrogel formed by gelation of alginic acid or a hydrogel formed by gelation through cross-linking with alginic acid derivatives represented by the following

formula (I-A) and formula (II-A) (cross-linked alginic acid gel).

[Alginic acid derivative represented by formula (I-A)]

A chemically modified alginic acid derivative represented by the following formula (I-A):

[Chem. 1]

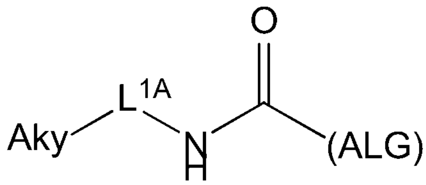

(I-A)

wherein (ALG) represents alginic acid; -NHCO- represents an amide bond via any carboxyl group of alginic acid; and $-L^{1A}-$ represents the following partial structural formula:

[Chem. 2]

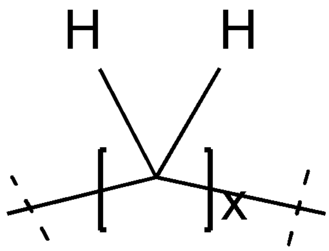

wherein x = 1 to 50 is satisfied;

1 to 15 of the $-CH_2-$ groups in the formula are each optionally replaced with such a group as -C(=O)-, -O-, -NH-, $-N(C_{1-3}$ alkyl group)-, -S-, a $C_{3-8}$ cycloalkyl ring, a benzene ring, a five- or six-membered aromatic heterocycle, or a five- or six-membered nonaromatic heterocycle;
1 to 10 of the hydrogen atoms of the $-CH_2-$ groups in the formula are each optionally replaced with such a group as a halogen atom, a hydroxy group, an amino group, a $C_{1-3}$ alkyl group, an $-O-C_{1-3}$ alkyl group, $-NH(C_{1-3}$ alkyl group), $-N(C_{1-3}$ alkyl group$)_2$, -COO-M (M = Na, K, 1/2 Ca, a hydrogen atom, or a $C_{1-3}$ alkyl group), a hydroxy $C_{1-3}$ alkyl group, a $C_{2-4}$ alkanoyl group, an $-S-C_{1-3}$ alkyl group, an $-SO_2-C_{1-3}$ alkyl group, a phenyl group, a benzyl group, a five- or six-membered aromatic heterocycle, or a five- or six-membered nonaromatic heterocycle; and
Aky is a cyclic alkyne group (in the formula, the outsides of the dashed lines at the two ends are not included).

[Alginic acid derivative represented by formula (II-A)]

A chemically modified alginic acid derivative represented by the following formula (II-A):

[Chem. 3]

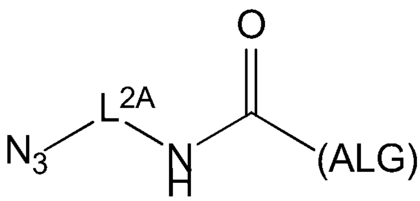

(II-A)

wherein (ALG) represents alginic acid; -NHCO- represents an amide bond via any carboxyl group of alginic acid; and $-L^{2A}-$ represents the following partial structural formula:

[Chem. 4]

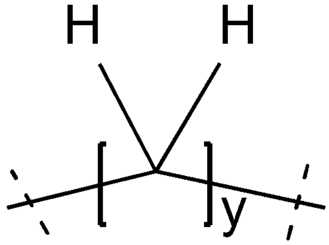

wherein y = 1 to 50 is satisfied;

1 to 15 of the $-CH_2-$ groups in the formula are each optionally replaced with such a group as -C(=O)-, -O-, -NH-, -N($C_{1-3}$ alkyl group)-, -S-, a $C_{3-8}$ cycloalkyl ring, a benzene ring, a five- or six-membered aromatic heterocycle, or a five- or six-membered nonaromatic heterocycle; and

1 to 10 of the hydrogen atoms of the $-CH_2-$ groups in the formula are each optionally replaced with such a group as a halogen atom, a hydroxy group, an amino group, a $C_{1-3}$ alkyl group, an -O-$C_{1-3}$ alkyl group, -NH($C_{1-3}$ alkyl group), -N($C_{1-3}$ alkyl group)$_2$, -COO-M (M = Na, K, 1/2 Ca, a hydrogen atom, or a $C_{1-3}$ alkyl group), a hydroxy $C_{1-3}$ alkyl group, a $C_{2-4}$ alkanoyl group, an -S-$C_{1-3}$ alkyl group, an -$SO_2$-$C_{1-3}$ alkyl group, a phenyl group, a benzyl group, a five- or six-membered aromatic heterocycle, or a five- or six-membered nonaromatic heterocycle (in the formula, the outsides of the dashed lines at the two ends are not included).

[1A-9-3] In [1A-9-2], $-L^{1A}-$ in the chemically modified alginic acid derivative represented by the formula (I-A) is such a linker that:

preferably, x = 2 to 45 is satisfied, 1 to 15 of the $-CH_2-$ groups in $-L^{1A}-$ are each optionally replaced with such a group as -C(=O)-, -O-, -NH-, -N($C_{1-3}$ alkyl group)-, a cyclohexane ring, a six-membered aromatic heterocycle, a six-membered nonaromatic heterocycle, or a benzene ring, and 1 to 10 of the hydrogen atoms of the $-CH_2-$ groups in $-L^{1A}-$ are each optionally replaced with such a group as a hydroxy group, an amino group, a $C_{1-3}$ alkyl group, an -O-$C_{1-3}$ alkyl group, -NH($C_{1-3}$ alkyl group), -N($C_{1-3}$ alkyl group)$_2$, or -COO-M (M = Na, K, 1/2 Ca, a hydrogen atom, or a $C_{1-3}$ alkyl group);

more preferably, x = 2 to 45 is satisfied, and 1 to 15 of the $-CH_2-$ groups in $-L^{1A}-$ are each optionally replaced with such a group as -C(=O)-, -O-, -NH-, -N($C_{1-3}$ alkyl group)-, a cyclohexane ring, or a benzene ring;

further preferably, x = 2 to 45 is satisfied, and 1 to 15 of the $-CH_2-$ groups in $-L^{1A}-$are each optionally replaced with such a group as -C(=O)-, -O-, -NH-, or a benzene ring;

particularly preferably, x = 3 to 25 is satisfied, and 1 to 15 of the $-CH_2-$ groups in $- L^{1A}-$ are each optionally replaced with such a group as -C(=O)-, -O-, -NH-, or a benzene ring; and

most preferably, x = 3 to 15 is satisfied, and 1 to 10 of the $-CH_2-$ groups in $-L^{1A}-$ are each optionally replaced with such a group as -C(=O)-, -O-, -NH-, or a benzene ring.

[1A-9-4] In [1A-9-2], $-L^{1A}-$ in the chemically modified alginic acid derivative represented by the formula (I-A) is a linker preferably selected from the following tables:

[Table 1-1]

| No. | $-L^{1A}-$ | |
|---|---|---|
| (LT1A-1) | 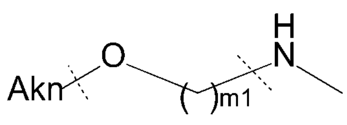 | m1 = 2-6 |
| (L1A-2) | 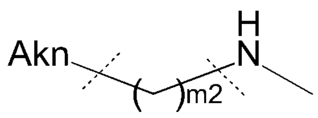 | m2 = 1-6 |
| (L1A-3) | 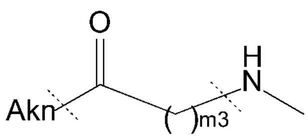 | m3 = 1-6 |

(continued)

| No. | $-L^{1A}-$ | |
|---|---|---|
| (L1A-4) | 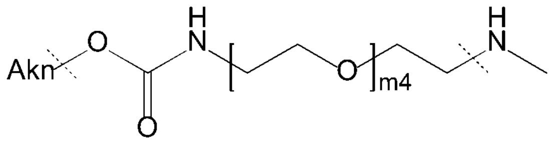 | m4 = 1-6 |
| (L1A-5) | 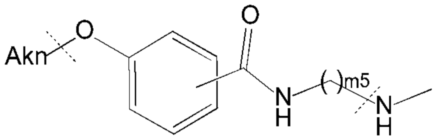 | m5 = 2-6 |
| (L1A-6) | 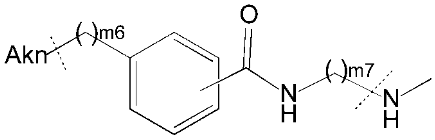 | m6 = 1-6, m7 = 2-6 |
| (L1A-7) | 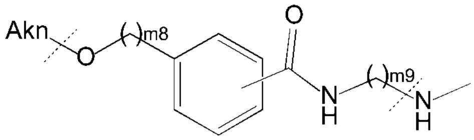 | m8 = 1-6, m9 = 2-6 |
| (L1A-8) | 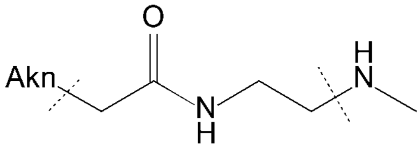 | |
| (L1A-9) | 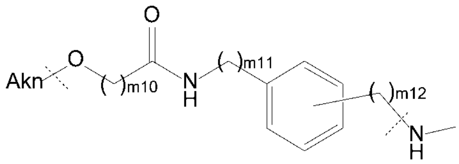 | m10 = 1-6, m11 = 0-6, m12 = 1-6 |
| (L1AB-1) | 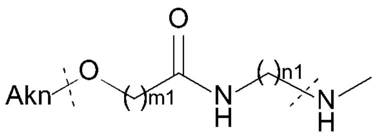 | ml = 1-6, n1 = 2-6 |
| (L1AB-2a) | 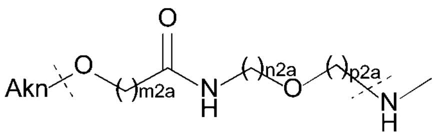 | m2a = 1-6, n2a = 2-6, p2a = 2-6 |

[Table 1-2]

| No. | $-L^{1A}-$ | |
|---|---|---|
| (L1AB-2b) | 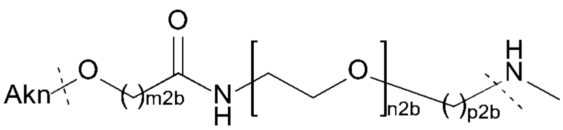 | m2b = 1-6, n2b = 1-6, p2b = 2-6 |

(continued)

| No. | $-L^{1A}-$ | |
|---|---|---|
| (L1AB-3) | 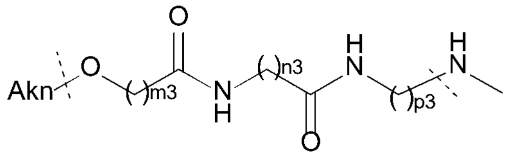 | m3 = 1-6, n3 = 1-6, p3 = 2-6 |
| (L1AB-4a) | 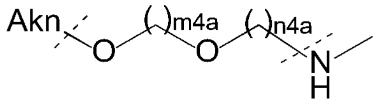 | m4a = 2-6, n4a = 2-6 |
| (L1AB-4b) | 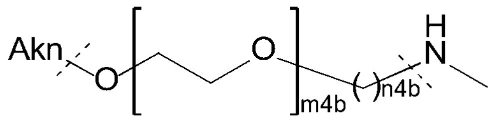 | m4b = 0-6, n4b = 2-6 |
| (L1AB-5a) | 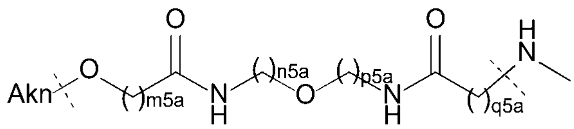 | m5a = 1-6, n5a = 2-6, p5a = 2-6, q5a = 1-6 |
| (L1AB-5b) | 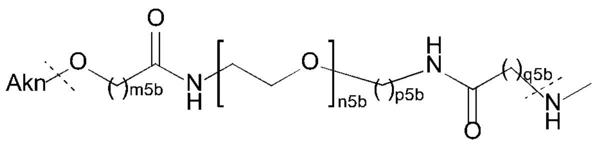 | m5b = 1-6, n5b = 1-6, p5b = 2-6, q5b = 1-6 |
| (L1AB-6a) | 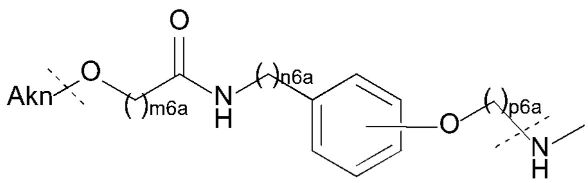 | m6a = 1-6, n6a = 0-6, p6a = 2-6 |
| (L1AB-6b) | 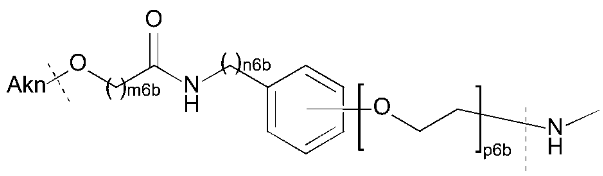 | m6b = 1-6, n6b = 0-6, p6b = 2-6 |
| (L1AB-7) | 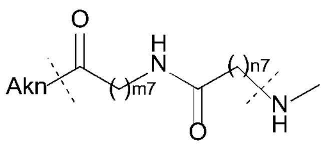 | m7 = 1-6, n7 = 1-6 |
| (L1AB-8a) | 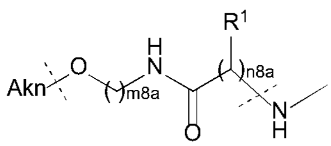 | m8a = 2-6, n8a = 1-6, $R^1$ = H,Me,Et,Bn |

[Table 1-3]

| No. | $-L^{1A}-$ | |
|---|---|---|
| (L1AB-8b) | 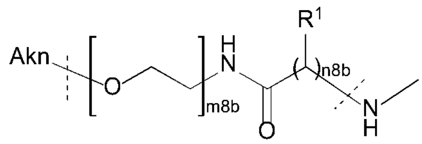 | m8b = 1-6, n8b = 1-6, $R^1$ = H,Me,Et,Bn |
| (L1AB-9a) | 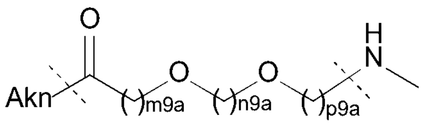 | m9a = 1-6, n9a = 2-6, p9a = 2-6 |
| (L1AB-9b) | 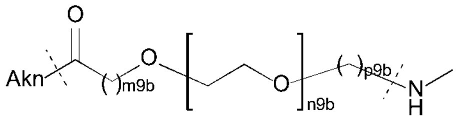 | m9b = 1-6, n9b = 1-6, p9b = 2-6 |
| (L1AB-10) | 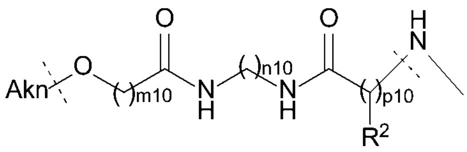 | m10 = 1-6, n10 = 2-6, p10 = 1-6, $R^2$ = H,Me,Et,Bn |

(in each of the formulas, the outsides of the dashed lines at the two ends are not included); more preferably a linker selected from the following tables:

[Table 2-1]

| No. | $-L^{1A}-$ | |
|---|---|---|
| (L1A-1) | 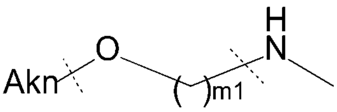 | m1 = 2-6 |
| (L1A-2-1) | 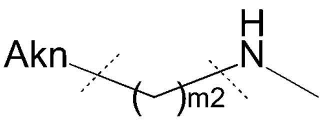 | m2 = 2-6 |
| (L1A-3-1) | 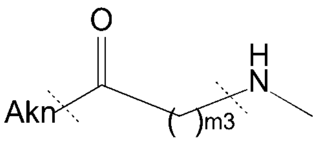 | m3 = 2-6 |
| (L1A-4) | 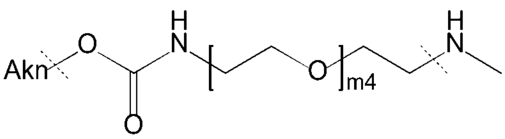 | m4 = 1-6 |
| (L1A-5-p) | 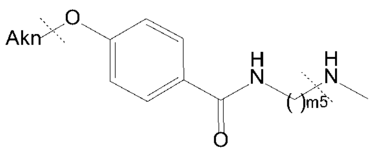 | m5 = 2-6 |

(continued)

| No. | -$L^{1A}$- | |
|---|---|---|
| (L1A-6-p) | 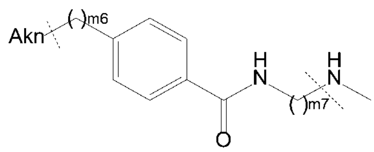 | m6 = 1-6, m7 = 2-6 |
| (L1A-7-p) | 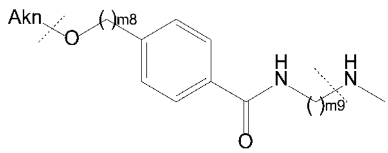 | m8 = 1-6, m9 = 2-6 |
| (L1A-8) | 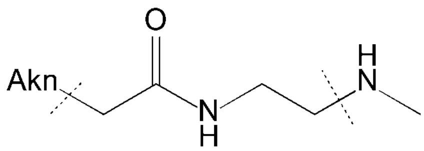 | |
| (L1A-9-p) | 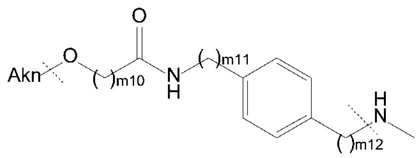 | m10 = 1-6, m11 = 0-6, m12 = 1-6 |
| (L1AB-1) | 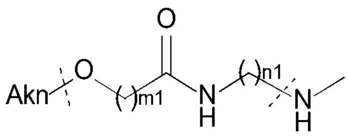 | m1 = 1-6<br>n1 = 2-6 |
| (L1AB-2a) | 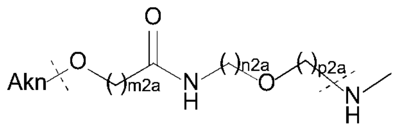 | m2a = 1-6<br>n2a = 2-6<br>p2a = 2-6 |

[Table 2-2]

| No. | -$L^{1A}$- | |
|---|---|---|
| (L1AB-2b) | 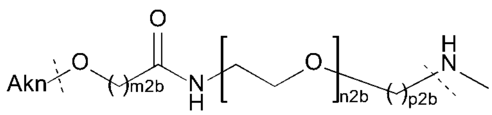 | m2b = 1-6<br>n2b = 1-6<br>p2b = 2-6 |
| (L1AB-3) | 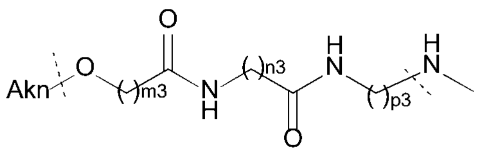 | m3 = 1-6<br>n3 = 1-6<br>p3 = 2-6 |
| (L1AB-4a) | 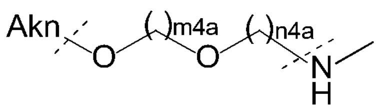 | m4a = 2-6<br>n4a = 2-6 |
| (L1AB-4b) | 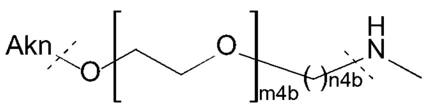 | m4b = 0-6<br>n4b = 2-6 |

(continued)

| No. | $-L^{1A}-$ | |
|---|---|---|
| (L1AB-5a) | 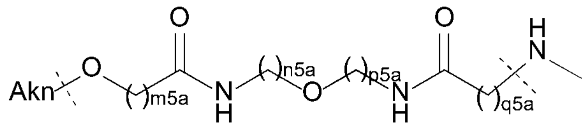 | m5a = 1-6<br>n5a = 2-6<br>p5a = 2-6<br>q5a = 1-6 |
| (L1AB-5b) | 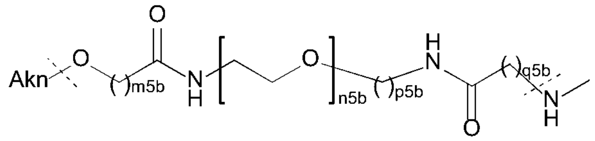 | m5b = 1-6<br>n5b = 1-6<br>p5b = 2-6<br>q5b = 1-6 |
| (L1AB-6a-p) | 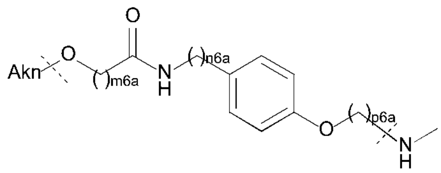 | m6a = 1-6<br>n6a = 0-6<br>p6a = 2-6 |
| (L1AB-6b-p) | 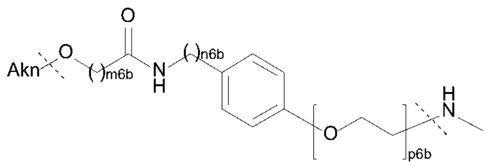 | m6b = 1-6<br>n6b = 0-6<br>p6b = 2-6 |
| (L1AB-7) | 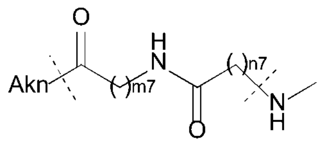 | m7 = 1-6<br>n7 = 1-6 |
| (L1AB-8a) | 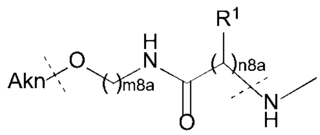 | m8a = 2-6<br>n8a = 1-6<br>R' = H,Me,Et,Bn |
| (L1AB-8b) | 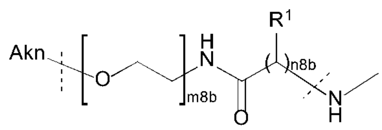 | m8b = 1-6<br>n8b = 1-6<br>$R^1$ = H,Me,Et,Bn |

(in each of the formulas, the outsides of the dashed lines at the two ends are not included); further preferably a linker selected from the following tables:

[Table 3-1]

| No. | $-L^{1A}-$ |
|---|---|
| (L1A-3-X1) | 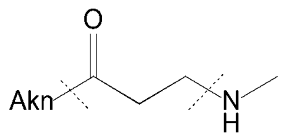 |
| (L1A-3-X2) | 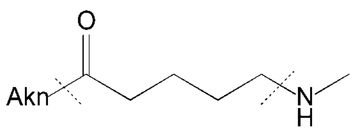 |

(continued)

| No. | $-L^{1A}-$ |
|---|---|
| (L1A-4-X) | 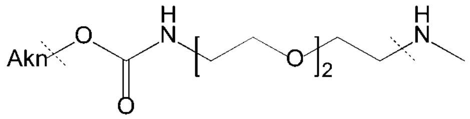 |
| (L1A-9-X) | 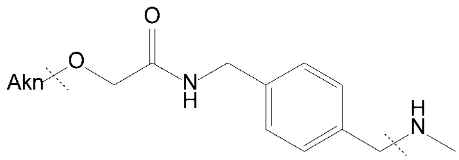 |
| (L1A-10-X) | 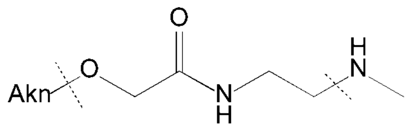 |
| (L1A-11-X) | 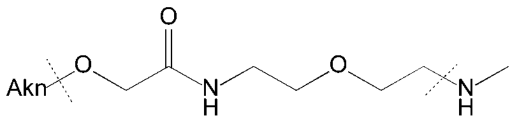 |
| (L1AB-1-X) | 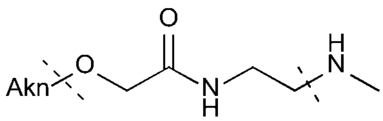 |
| (L1AB-2-X) | 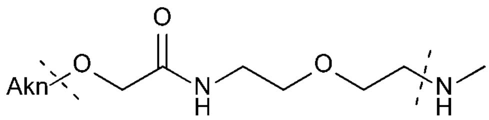 |
| (L1AB-3-X1) | 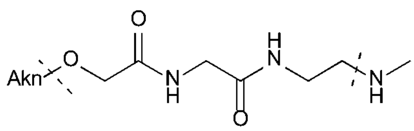 |
| (L1AB-3-X2) | 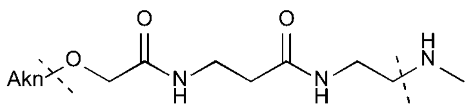 |
| (LIAB-4-XI) | 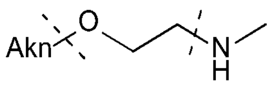 |

[Table 3-2]

| No. | $-L^{1A}-$ |
|---|---|
| (L1AB-4-X2) | 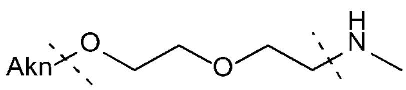 |
| (L1AB-5-X) | 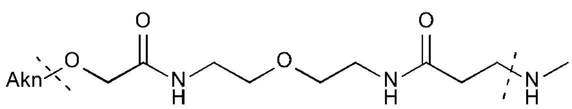 |

(continued)

| No. | $-L^{1A}-$ |
|---|---|
| (L1AB-6-X) | 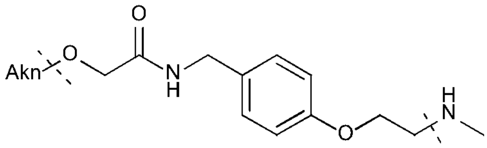 |
| (L1AB-7-X) | 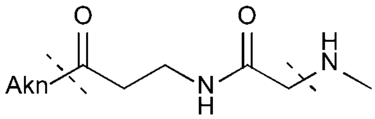 |
| (L1AB-8-X1) | 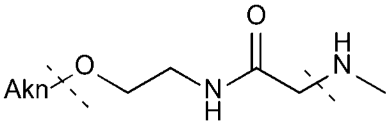 |
| (L1AB-8-X2) | 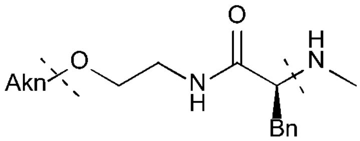 |

(in each of the formulas, the outsides of the dashed lines at the two ends are not included); and particularly preferably a linker selected from the following table:

[Table 4]

| No. | $-L^{1A}-$ |
|---|---|
| (L1A-3-X1) | 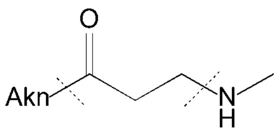 |
| (L1A-9-X) | 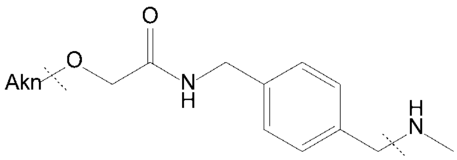 |

(in each of the formulas, the outsides of the dashed lines at the two ends are not included).

[1A-9-5] In [1A-9-2], Aky in the alginic acid derivative represented by the formula (I-A) is preferably a seven- to nine-membered cyclic alkyne group (one to five of the hydrogen atoms of the $-CH_2-$ groups in the cyclic alkyne group are each optionally replaced with such a group as a halogen atom, a hydroxy group, an amino group, a keto group, a $C_{1-3}$ alkyl group, an $-O-C_{1-3}$ alkyl group, $-NH(C_{1-3}$ alkyl group), $-N(C_{1-3}$ alkyl group$)_2$, or -COO-M (M = Na, K, 1/2 Ca, a hydrogen atom, or a $C_{1-3}$ alkyl group); and the cyclic alkyne group is optionally fused with one to three rings each being a $C_{3-8}$ cycloalkyl ring, a benzene ring, or a five- or six-membered aromatic heterocycle);
more preferably an eight-membered cyclic alkyne group (a cyclooctyne group) (one to five of the hydrogen atoms of the $-CH_2-$ groups in the cyclic alkyne group are each optionally replaced with such a group as a halogen atom, a keto group, a $C_{1-3}$ alkyl group, or an $-O-C_{1-3}$ alkyl group; and the cyclic alkyne group is optionally fused with one to three rings each being a cyclopropane ring, a benzene ring, or a fivemembered aromatic heterocycle);
further preferably a group selected from the following partial structural formulas:

[Table 5]

| No. | Akv- | No. | Aky- | No. | Aky- |
|---|---|---|---|---|---|
| (AK-1) | 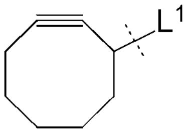 | (AK-5) | 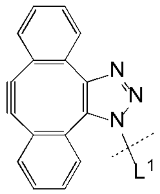 | (AK-9) | 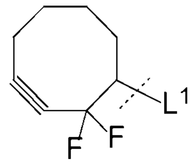 |
| (AK-2) | 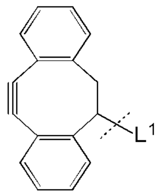 | (AK-6) | 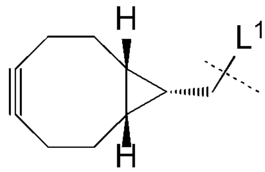 | (AK-10) | 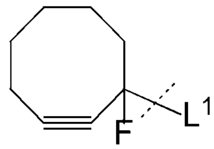 |
| (AK-3) | 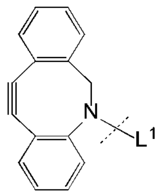 | (AK-7) | 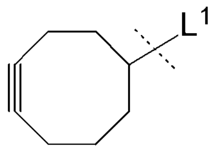 | (AK-11) | 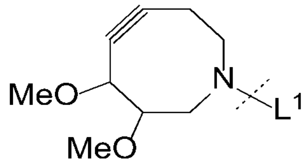 |
| (AK-4) | 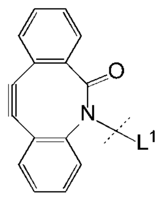 | (AK-8) | 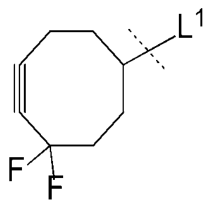 | (AK-12) | 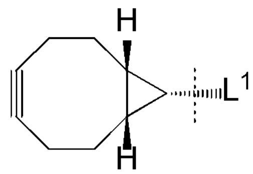 |

(in each of the formulas, the right sides of the dashed lines at the two ends are not included);
furthermore preferably a cyclic alkyne group selected from the group of the following partial structural formulas:

[Table 6]

| No. | Aky- | No. | Aky- | No. | Aky- |
|---|---|---|---|---|---|
| (AK-1) | 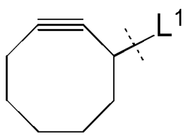 | (AK-4) | 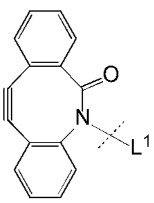 | (AK-10) | 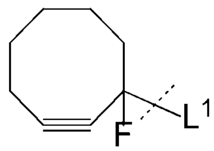 |

(continued)

| No. | Aky- | No. | Aky- | No. | Aky- |
|---|---|---|---|---|---|
| (AK-2) | 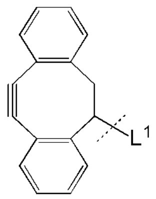 | (AK-5) | 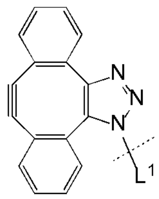 | | |
| (AK-3) | 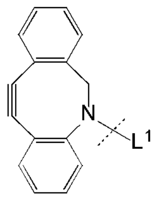 | (AK-6) | 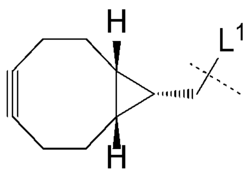 | | |

(in each of the formulas, the right sides of the dashed lines at the two ends are not included);
particularly preferably a cyclic alkyne group selected from the group of the following partial structural formulas:

[Table 7]

| No. | Aky- | No. | Aky- | No. | Aky- |
|---|---|---|---|---|---|
| (AK-1) | 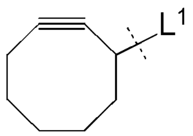 | (AK-3) | 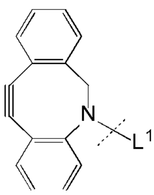 | (AK-6) | 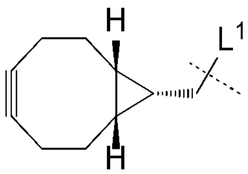 |

(in each of the formulas, the right sides of the dashed lines at the two ends are not included); and
most preferably a cyclic alkyne group selected from the group of the following partial structural formulas:

[Table 8]

| No. | Aky- |
|---|---|
| (AK-1) | 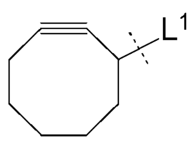 |
| (AK-3) | 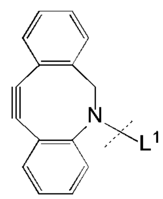 |

(in each of the formulas, the right sides of the dashed lines at the two ends are not included).

[1A-9-6] In [1A-9-2], $-L^{2A}-$ in the alginic acid derivative represented by the formula (II-A) is such a linker that:

preferably, y = 5 to 40 is satisfied, 1 to 15 of the $-CH_2-$ groups in $-L^{2A}-$ are each optionally replaced with such a group as -C(=O)-, -O-, -NH-, $-N(C_{1-3}$ alkyl group)-, a cyclohexane ring, a six-membered aromatic heterocycle, a six-membered nonaromatic heterocycle, or a benzene ring, and 1 to 10 of the hydrogen atoms of the $-CH_2-$ groups in $-L^{2A}-$ are each optionally replaced with such a group as a hydroxy group, an amino group, $C_{1-3}$ alkyl group, an $-O-C_{1-3}$ alkyl group,

-NH($C_{1-3}$ alkyl group), -N($C_{1-3}$ alkyl group)$_2$, or -COO-M (M = Na, K, 1/2 Ca, a hydrogen atom, or a $C_{1-3}$ alkyl group); more preferably, y = 5 to 40 is satisfied, and 1 to 10 of the -$CH_2$- groups in -$L^{2A}$- are each optionally replaced with such a group as -C(=O)-, -O-, -NH-, -N($C_{1-3}$ alkyl group)-, a cyclohexane ring, or a benzene ring;
further preferably, y = 5 to 40 is satisfied, and 1 to 10 of the -$CH_2$- groups in -$L^{2A}$-are each optionally replaced with such a group as -C(=O)-, -O-, -NH-, -N($C_{1-3}$ alkyl group)-, or a benzene ring;
particularly preferably, y = 5 to 20 is satisfied, and 1 to 10 of the -$CH_2$- groups in - $L^{2A}$- are each optionally replaced with such a group as -C(=O)-, -O-, -NH-, or a benzene ring; and
most preferably, y = 5 to 15 is satisfied, and 1 to 10 of the -$CH_2$- groups in -$L^{2A}$- are each optionally replaced with such a group as -C(=O)-, -O-, -NH-, or a benzene ring.

[1A-9-7] In [1A-9-2], -$L^{2A}$- in the alginic acid derivative represented by the formula (II-A) is preferably a linker selected from the following tables:

[Table 9-1]

| No. | -$L^{2A}$- | |
|---|---|---|
| (L2A-1) | 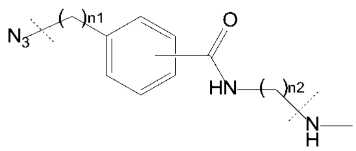 | n1 = 1-6, n2 = 2-6 |
| (L2A-2) | 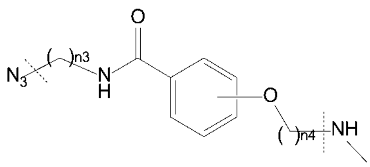 | n3 = 2-6, n4 = 2-6 |
| (L2A-3) | 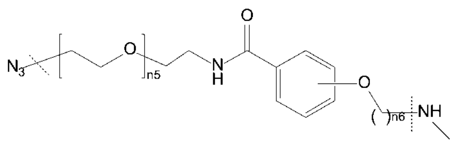 | n5 = 1-6, n6 = 2-6 |
| (L2A-4) | 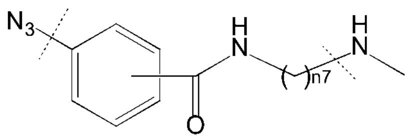 | n7 = 2-6 |
| (L2A-5) | 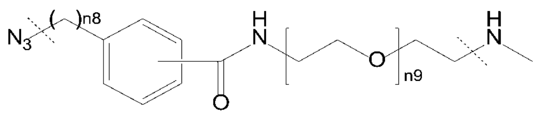 | n8 = 1-4, n9 = 1-6 |
| (L2A-6) | 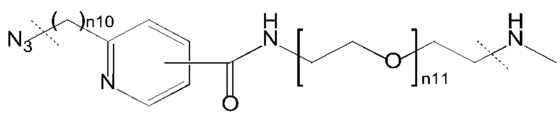 | n10 = 1-4, n11 = 1-6 |
| (L2A-7) | 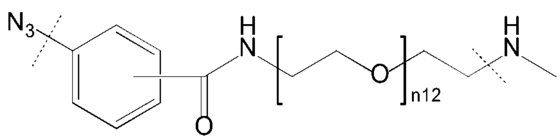 | n12 = 1-6 |
| (L2AB-1) | 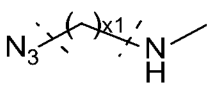 | x1 = 2-6 |
| (L2AB-2a) | 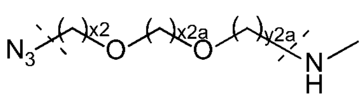 | x2 = 2-6, x2a = 2-6, v2a = 2-6 |

(continued)

| No. | -$L^{2A}$- | |
|---|---|---|
| (L2AB-2b) | 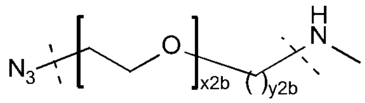 | x2b = 1-6, y2b = 2-6 |
| (L2AB-3) | 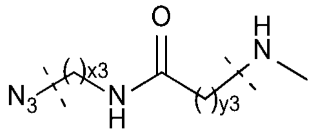 | x3 = 2-6, y3 = 1-6 |
| (L2AB-4) | 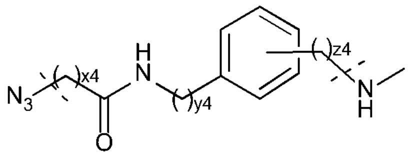 | x4 = 1-6, y4 = 1-6, z4 = 1-6 |

[Table 9-2]

| No. | -$L^{2A}$- | |
|---|---|---|
| (L2AB-5a) | 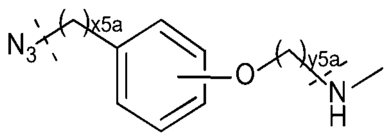 | x5a = 0-4, y5a = 2-6 |
| (L2AB-5b) | 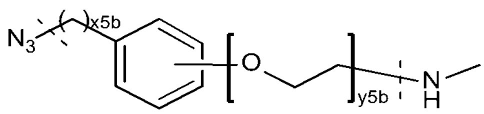 | x5b = 0-4, y5b = 1-6 |
| (L2AB-6a) | 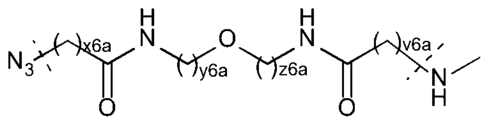 | x6a = 1-6, y6a = 2-6, z6a = 2-6, v6a = 1-6 |
| (L2AB-6b) | 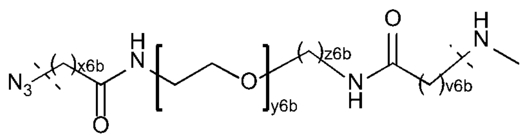 | x6b = 1-6, y6b = 1 = 6, z6b = 2-6, v6b = 1-6 |
| (L2AB-7a) | 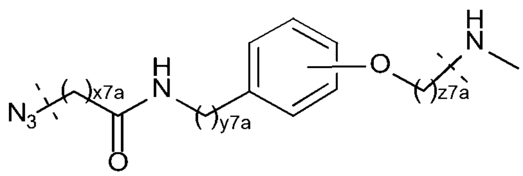 | x7a = 1-6, y7a = 1-6, z7a = 2-6 |
| (L2AB-7b) | 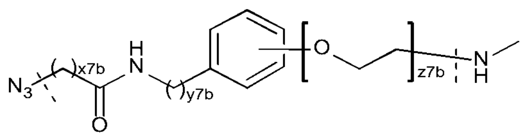 | x7b = 1-6, y7b = 1-6, z7b = 1-6 |
| (L2AB-8a) | 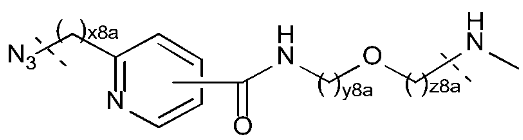 | x8a = 0-4, y8a = 2-6, z8a = 3-6 |

(continued)

| No. | -L2A- | |
|---|---|---|
| (L2AB-8b) | 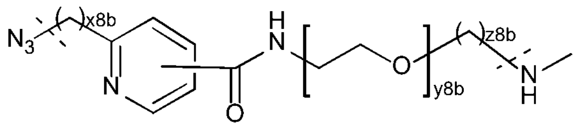 | x8b = 0-4, y8b = 1-6, z8b = 3-6 |
| (L2AB-9a) | 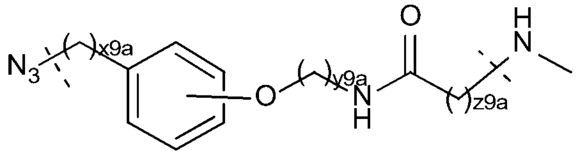 | x9a = 0-4, y9a = 2-6, z9a = 1-6 |
| (L2AB-9b) | 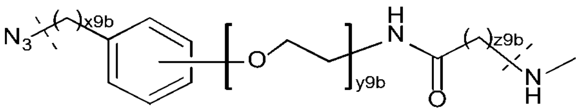 | x9b = 0-4, y9b = 1-6, z9b = 1-6 |
| (L2AB-10) | 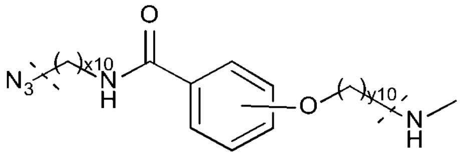 | x10 = 2-6, y10 = 2-6 |

(in each of the formulas, the outsides of the dashed lines at the two ends are not included); more preferably a linker selected from the following tables:

[Table 10-1]

| No. | -L2A- | |
|---|---|---|
| (L2A-1-1) | 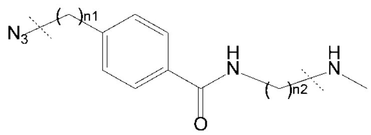 | n1 = 1-6, n2 = 2-6 |
| (L2A-2-1) | 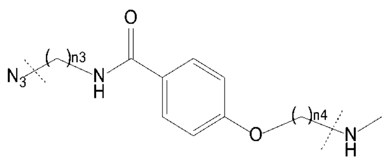 | n3 = 2-6, n4 = 2-6 |
| (L2A-3-1) | 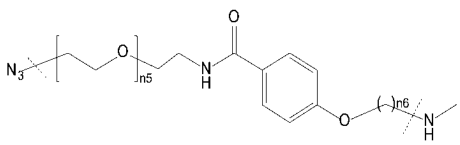 | n5 = 1-6, n6 = 2-6 |
| (L2A-4-1) | 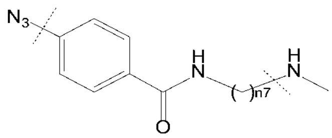 | n7 = 2-6 |
| (L2A-5-1) | 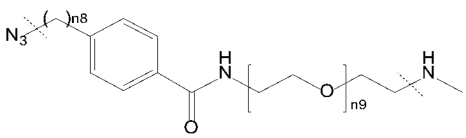 | n8 = 1-4, n9 = 1-6 |

(continued)

| No. | -L2A- | |
|---|---|---|
| (L2A-6-1) | 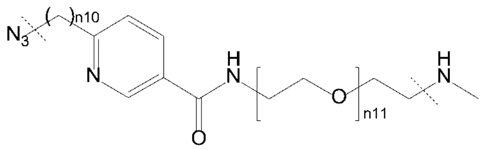 | n10 = 1-4, n11 = 1-6 |
| (L2A-7-1) | 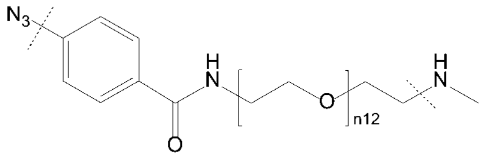 | n12 = 1-6 |
| (L2AB-1) | 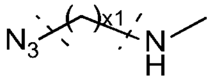 | x1 = 2-6 |
| (L2AB-2a) | 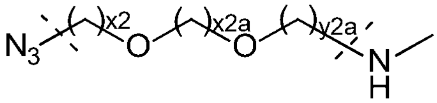 | x2 = 2-6, x2a = 2-6, y2a = 2-6 |
| (L2AB-2b) | 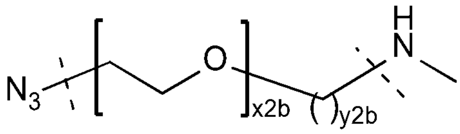 | x2b = 1-6, y2b = 2-6 |
| (L2AB-3) | 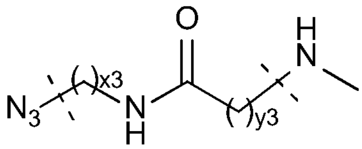 | x3 = 2-6, y3 = 1-6 |

[Table 10-2]

| No. | -L2A- | |
|---|---|---|
| (L2AB-4-p) | 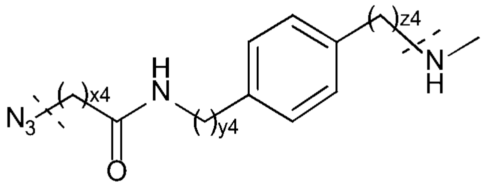 | x4 = 1-6, y4 = 1-6, z4 = 1-6 |
| (L2AB-5a-p) | 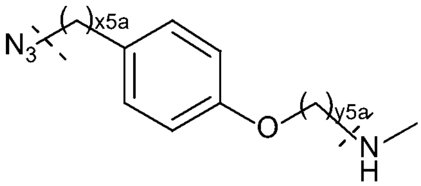 | x5a = 0-4, y5a = 2-6 |
| (L2AB-5b-p) | 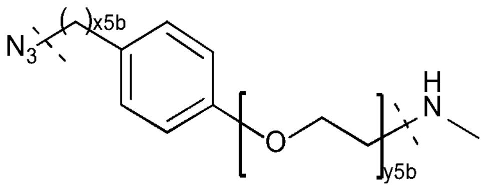 | x5b = 0-4, y5b = 1-6 |

(continued)

| No. | -$L^{2A}$- | |
|---|---|---|
| (L2AB-10-p) | 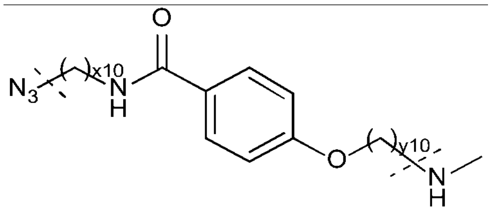 | x10 = 2-6, y10 = 2-6 |

(in each of the formulas, the outsides of the dashed lines at the two ends are not included);
further preferably a linker selected from the following tables:

[Table 11-1]

| No. | -$L^{2A}$- |
|---|---|
| (L2A-1-X1) | 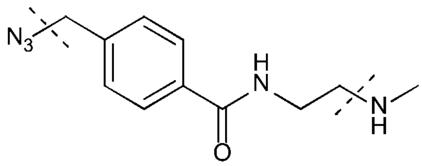 |
| (L2A-2-X1) | 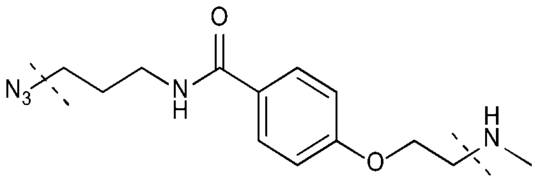 |
| (L2A-3-X1) | 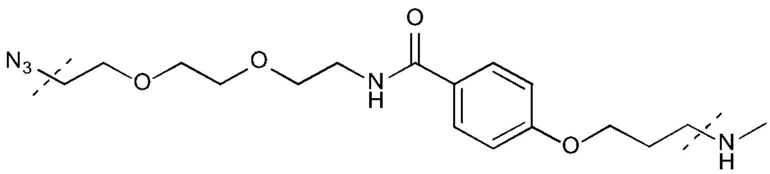 |
| (L2A-4-X1) | 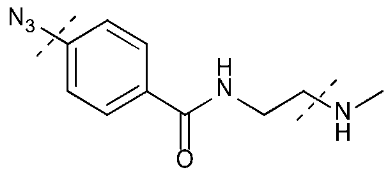 |
| (L2A-5-X1) | 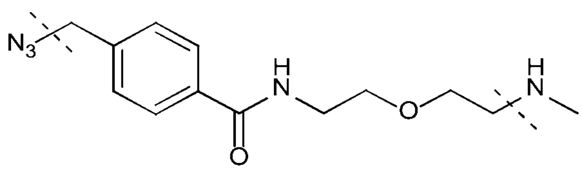 |
| (L2A-5-X2) | 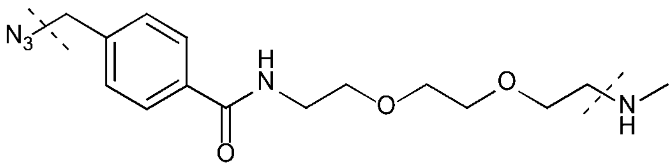 |

(continued)

| No. | -L2A- |
|---|---|
| (L2A-6-X1) | 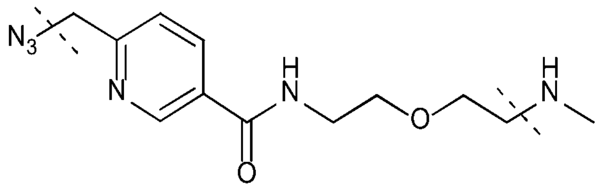 |
| (L2A-7-X1) | 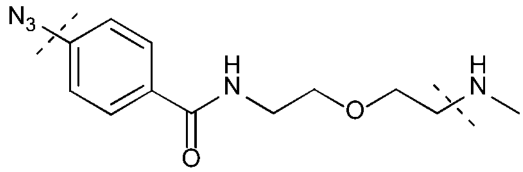 |
| (L2A-7-X2) | 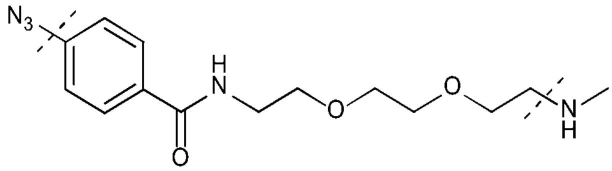 |

[Table 11-2]

| No. | -L2A- |
|---|---|
| (L2AB-1-X1) | 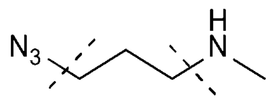 |
| (L2AB-2ab-X1) | 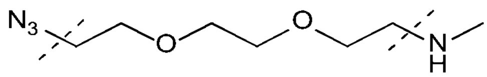 |
| (L2AB-3-X1) | 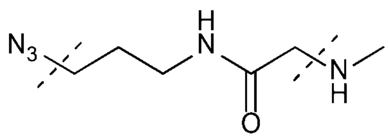 |
| (L2AB-3-X2) | 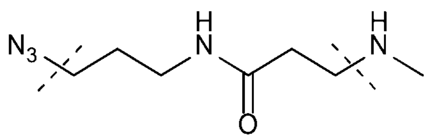 |
| (L2AB-4-X1) | 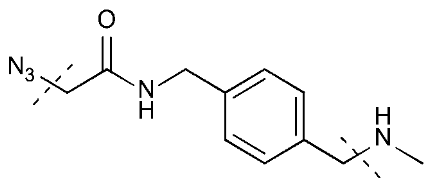 |
| (L2AB-5ab-X1) | 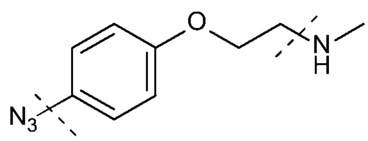 |

(in each of the formulas, the outsides of the dashed lines at the two ends are not included); and particularly preferably a linker selected from the following table:

[Table 12]

| No. | $-L^{2A}-$ |
|---|---|
| (L2A-2-X1) | 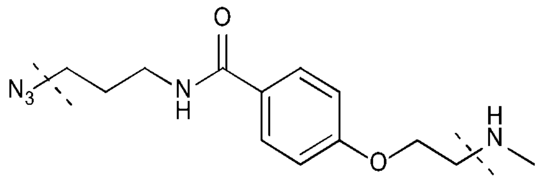 |
| (L2A-4-X1) | 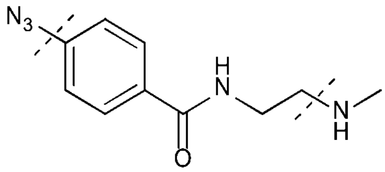 |
| (L2A-5-X1) | 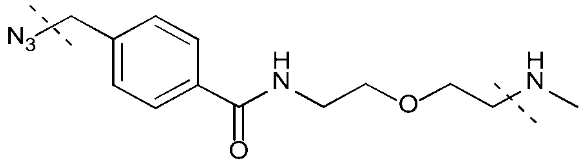 |
| (L2A-7-X1) | 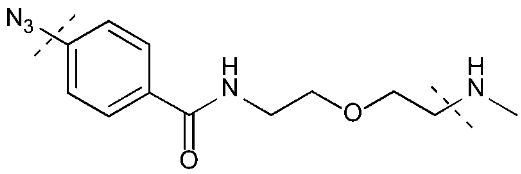 |

(in each of the formulas, the outsides of the dashed lines at the two ends are not included).

[1A-10] In [1A-9-2], the alginic acid derivative represented by the formula (I-A) is preferably selected from the following formulas (A01) to (A15):

[Chem. 5]

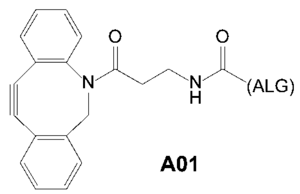

A01

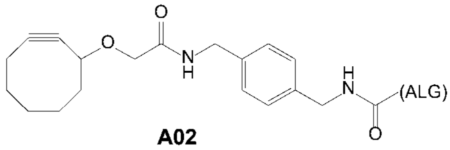

A02

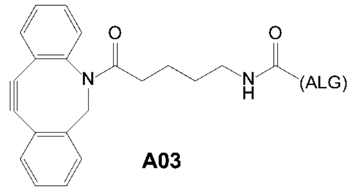

A03

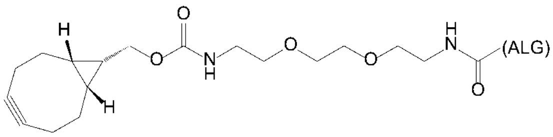

A04

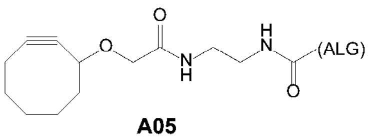

A05

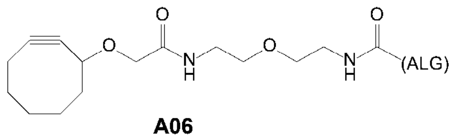

A06

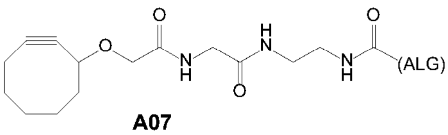

A07

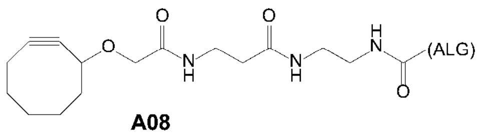

A08

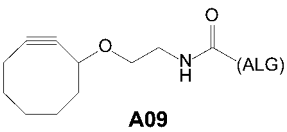

A09

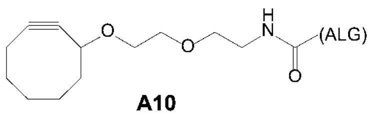

A10

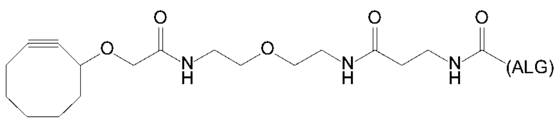

A11

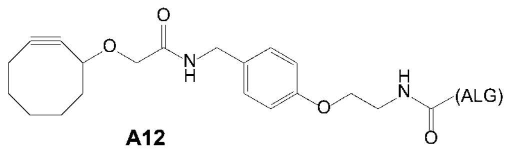

A12

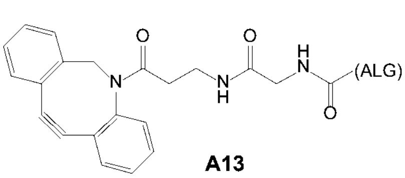

A13

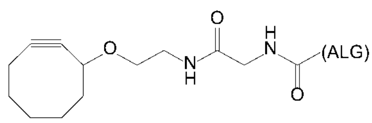

A14

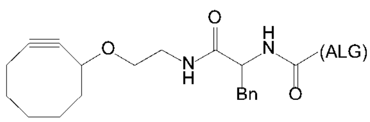

A15

wherein, in each formula, (ALG) represents alginic acid, and -NHCO- bonding to (ALG) represents an amide bond via any carboxyl group of alginic acid; and more preferably selected from the following formulas (A01) and (A02):

[Chem. 6]

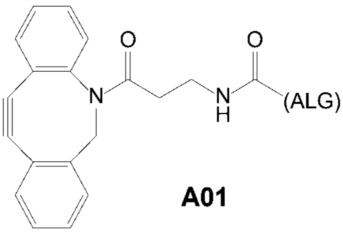
A01

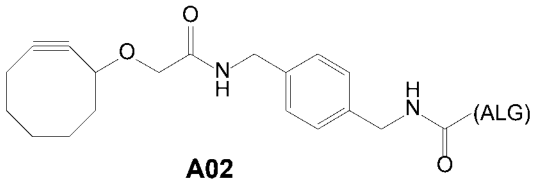
A02

wherein, in each formula, (ALG) represents alginic acid, and -NHCO- bonding to (ALG) represents an amide bond via any carboxyl group of alginic acid.

[1A-11] In [1A-9-2], the alginic acid derivative represented by the formula (II-A) is preferably selected from the following formulas (N01) to (N15):

[Chem. 7]

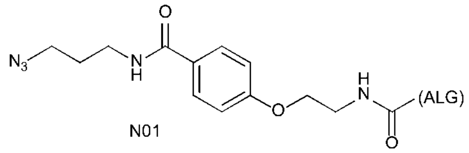
N01

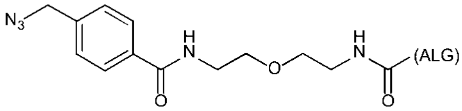
N02

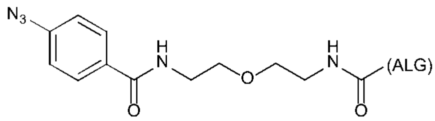
N03

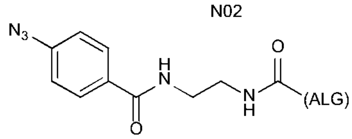
N04

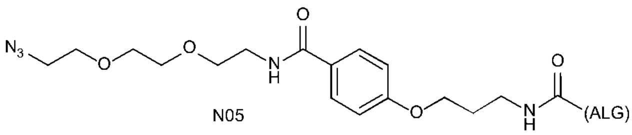
N05

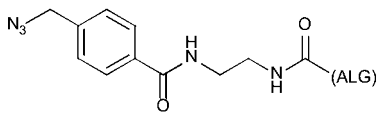
N06

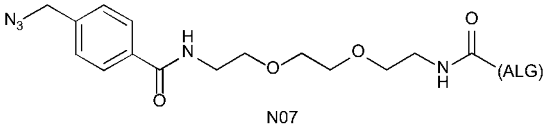
N07

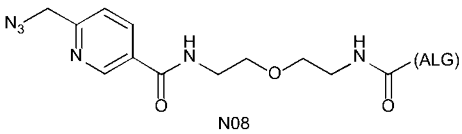
N08

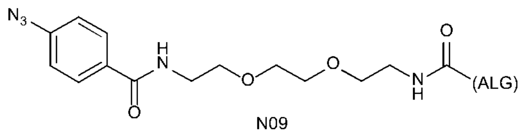
N09

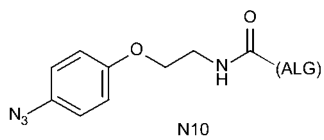
N10

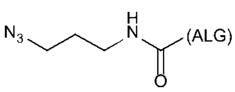
N11

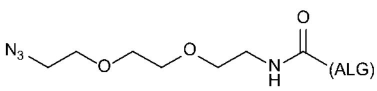
N12

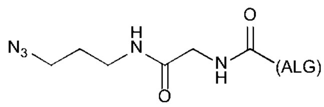
N13

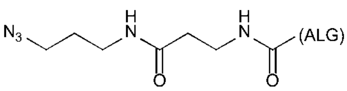
N14

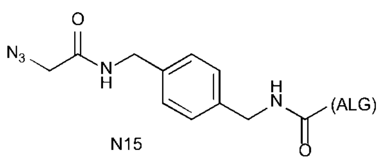
N15

wherein, in each formula, (ALG) represents alginic acid, and -NHCO- bonding to (ALG) represents an amide bond via any carboxyl group of alginic acid; and more preferably selected from the following formulas (N01) to (N04):

[Chem. 8]

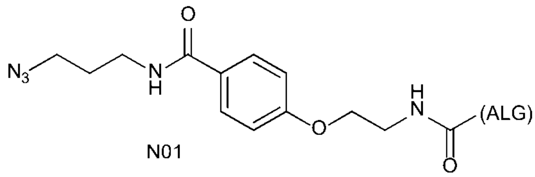

N01

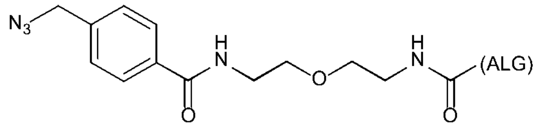

N02

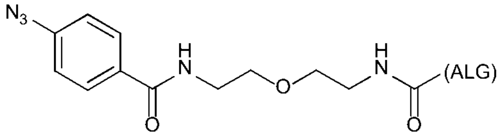

N03

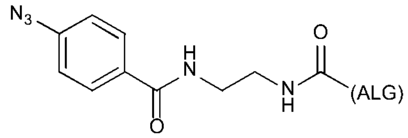

N04

wherein, in each formula, (ALG) represents alginic acid, and -NHCO- bonding to (ALG) represents an amide bond via any carboxyl group of alginic acid.

[1A-12] The biocompatible device according to any one of [1A-1-1] to [1A-11], wherein the composite membrane has a thickness of approximately 10 to approximately 1500 μm.

[1A-13] The biocompatible device according to any one of [1A-1-1] to [1A-12], wherein the composite membrane has a strength of approximately 1 MPa or more as measured in tensile test in accordance with JIS K 7127.

[1A-14] The biocompatible device according to any one of [1A-1-1] to [1A-13], for use in living-donor transplantation.

[1A-15] The biocompatible device according to any one of [1A-1-1] to [1A-14], wherein the biocompatible composite membrane is formed by laminating a cellulose acetate derivative on one surface of a non-woven fabric by a Loeb-Sourirajan method.

[1A-16] A biocompatible composite membrane for producing the biocompatible device according to any one of [1A-1-1] to [1A-15].

[1A-17] A kit for living-donor transplantation, the kit comprising: a biocompatible device with an internal space formed by a biocompatible composite membrane having a cellulose derivative laminated on one surface of a non-woven fabric; and a carrier comprising a cell or tissue that secretes a biologically active substance, wherein the kit is used in such a manner that the cell or tissue is enclosed in the internal space.

[1A-18-1] A method of producing a biocompatible device, the method comprising a step of covering a hydrogel embedding a cell or tissue that secretes a biologically active substance with a biocompatible composite membrane including a semi-permeable membrane layer and a non-woven fabric layer, wherein, in the step, the semi-permeable membrane layer is disposed on an outer side and the non-woven fabric layer is disposed on a hydrogel side.

[1A-18-2] A method of producing a biocompatible device, the method comprising a step of covering a hydrogel embedding a cell or tissue that secretes a biologically active substance with a biocompatible composite membrane having a cellulose derivative laminated on one surface of a non-woven fabric, wherein, in the step, the surface of the biocompatible composite membrane with the cellulose derivative laminated thereon is disposed on an outer side.

[1A-18-3] The method of producing a biocompatible device according to [1A-18-1] or [1A-18-2], wherein the hydrogel is a hydrogel formed by gelation of one or more of the gelable substances shown in any one of [1A-9] to [1A-11].

[1A-19-1] A method of producing a biocompatible device, the method comprising a step of injecting a solution comprising a cell or tissue that secretes a biologically active substance and is mixed with a solution capable of hydrogelation into an internal space formed by a biocompatible composite membrane including a semi-permeable membrane layer and a non-woven fabric layer and gelling the solution capable of hydrogelation, wherein, in the step, the semi-permeable membrane layer is on an outer side and the non-woven fabric layer is on an internal-space side in the composite membrane.

[1A-19-2] A method of producing a biocompatible device, the method comprising a step of injecting a mixed solution comprising a cell or tissue that secretes a biologically active substance and mixed with a solution capable of hydrogelation into an internal space formed by a biocompatible composite membrane having a cellulose derivative laminated on one surface of a non-woven fabric and gelling the mixed solution, wherein, in the step, the surface of the biocompatible composite membrane with the cellulose derivative laminated thereon is disposed on an outer side.

[1A-19-3] The method of producing a biocompatible device according to any one of [1A-19-1] or [1A-19-2], wherein the solution capable of hydrogelation is a solution comprising one or more of the gelable substances shown in any one of [1A-9] to [1A-11].

[2-1] A biocompatible composite membrane comprising a semi-permeable membrane layer and a non-woven fabric layer, wherein the semi-permeable membrane layer comprises a cellulose derivative formed on a non-woven fabric

serving as a support, the non-woven fabric layer comprises the thermoplastic non-woven fabric, and the composite membrane is used for enclosing a cell or tissue.

[2-2] The composite membrane according to [2-1], having immunoisolatability.

[2-3] The composite membrane according to [2-1] or [2-2], wherein the cellulose derivative is cellulose acetate.

[2-4] The composite membrane according to any one of [2-1] to [2-3], wherein the non-woven fabric comprises one or more resins selected from the group consisting of polyethylene, polypropylene, and polyethylene terephthalate.

[2-5] The composite membrane according to any one of [2-1] to [2-4], wherein an internal space can be formed by partial fusion of the non-woven fabric layer.

[2-6] The composite membrane according to any one of [2-1] to [2-5], wherein the composite membrane has a structure with partial intrusion of the cellulose derivative into the non-woven fabric.

[2-7] The composite membrane according to any one of [2-1] to [2-6], wherein the cell or tissue is enclosed in an internal space with a hydrogel serving as a carrier.

[2-8] The composite membrane according to any one of [2-1] to [2-7], wherein the cell or tissue is a cell or tissue that secretes a biologically active substance.

[2-9] The composite membrane according to any one of [2-1] to [2-8], wherein the cell is an insulin-secreting cell or a pancreatic islet.

[2-10] The composite membrane according to any one of [2-1] to [2-9], having a thickness of 10 to 1500 μm.

[2-11] The composite membrane according to any one of [2-1] to [2-10], having a strength of 1 MPa or more as measured in tensile test in accordance with JIS K 7127.

[2-12] The composite membrane according to any one of [2-1] to [2-11], for use in producing a living-donor transplantation device.

[2A-1-1] A biocompatible composite membrane comprising a semi-permeable membrane layer and a non-woven fabric layer, wherein the semi-permeable membrane layer comprises a cellulose derivative laminated on a non-woven fabric using the non-woven fabric as a support, the non-woven fabric layer comprises a thermoplastic non-woven fabric, and the composite membrane is used for enclosing a cell or tissue.

[2A-1-2] A biocompatible composite membrane consisting of a semi-permeable membrane layer and a non-woven fabric layer, wherein the semi-permeable membrane layer comprises a cellulose derivative laminated on a non-woven fabric using the non-woven fabric as a support, the non-woven fabric layer comprises a thermoplastic non-woven fabric, and the composite membrane is used for enclosing a cell or tissue.

[2A-1-3] A biocompatible composite membrane comprising a cellulose derivative laminated on one surface of a non-woven fabric, wherein the composite membrane is used for enclosing a cell or tissue.

[2A-2] The composite membrane according to any one of [2A-1-1] to [2A-1-3], having immunoisolatability.

[2A-3] The composite membrane according to any one of [2A-1-1] to [2A-2], wherein the cellulose derivative is cellulose acetate.

[2A-4] The composite membrane according to any one of [2A-1-1] to [2A-3], wherein the non-woven fabric comprises one or more resins selected from the group consisting of polyethylene (PE), polypropylene (PP), and polyethylene terephthalate (PET).

[2A-5] The composite membrane according to any one of [2A-1-1] to [2A-4], wherein an internal space can be formed by partial fusion of a surface of the non-woven fabric layer that is free of cellulose derivative.

[2A-6] The composite membrane according to any one of [2A-1-1] to [2A-5], wherein the composite membrane has a structure with partial intrusion of the cellulose derivative into the non-woven fabric.

[2A-7] The composite membrane according to any one of [2A-1-1] to [2A-6], wherein a cell or tissue is enclosed, with a hydrogel serving as a carrier, in an internal space formed by partial fusion of a surface of the non-woven fabric layer that is free of cellulose derivative.

[2A-7-1] The composite membrane according to [2A-7], wherein the hydrogel is a hydrogel formed by gelation of one or more of the gelable substances shown in any one of [1A-9] to [1A-11].

[2A-8] The composite membrane according to [2A-7] or [2A-7-1], wherein the cell or tissue is a cell or tissue that secretes a biologically active substance.

[2A-9] The composite membrane according to any one of [2A-1-1] to [2A-8], wherein the cell is an insulin-secreting cell or a pancreatic islet.

[2A-10] The composite membrane according to any one of [2A-1-1] to [2A-9], having a thickness of approximately 10 to approximately 1500 μm.

[2A-11] The composite membrane according to any one of [2A-1-1] to [2A-10], having a strength of approximately 1 MPa or more as measured in tensile test in accordance with JIS K 7127.

[2A-12] The composite membrane according to any one of [2A-1-1] to [2A-11], for use in producing a living-donor transplantation device.

[2A-13] The composite membrane according to any one of [2A-1-1] to [2A-12], wherein the composite membrane is formed by laminating a cellulose acetate derivative on one surface of a non-woven fabric by a Loeb-Sourirajan method.

[3-1] The device according to any one of [1-7] to [1-11] or the composite membrane according to any one of [2-7] to [2-12], wherein the hydrogel comprises a hydrogel formed by gelation of one or more selected from the group consisting of alginic acid, collagen, hyaluronan, gelatin, fibronectin, elastin, tenascin, laminin, vitronectin, polypeptide, heparan sulfate, chondroitin, chondroitin sulfate, keratan, keratan sulfate, dermatan sulfate, carrageenan, heparin, chitin, chitosan, agarose, agar, cellulose, methylcellulose, carboxylmethylcellulose, glycogen, and derivatives of these, and fibrin, fibrinogen, thrombin, polyglutamic acid, polylactic acid, polyglycolic acid, lactic acid-glycolic acid copolymer, gellan gum, xanthan gum, galactomannan, guar gum, locust bean gum, and tara gum.

[3-2] The device according to any one of [1-7] to [1-11] or the composite membrane according to any one of [2-7] to [2-12], wherein the hydrogel comprises a hydrogel formed by gelation of one or more selected from the group consisting of alginic acid, collagen, hyaluronan, gelatin, fibronectin, elastin, tenascin, laminin, vitronectin, polypeptide, heparan sulfate, chondroitin, chondroitin sulfate, keratan, keratan sulfate, dermatan sulfate, carrageenan, heparin, chitin, chitosan, agarose, agar, cellulose, methylcellulose, carboxylmethylcellulose, glycogen, and derivatives of these.

[3-3] The device according to any one of [1-7] to [1-11] or the composite membrane according to any one of [2-7] to [2-12], wherein the hydrogel comprises a hydrogel formed by gelation of alginic acid or an alginic acid derivative.
[3-4] The device according to any one of [1-7] to [1-11] or the composite membrane according to any one of [2-7] to [2-12], wherein the hydrogel comprises a hydrogel formed by gelation of an alginic acid derivative through chemical cross-linking.

[3-5] The device or composite membrane according to [3-4], wherein the alginic acid derivative comprises alginic acid derivatives represented by the following formula (H-I) and formula (H-II).

[Alginic acid derivative represented by formula (H-I)]

An alginic acid derivative represented by the following formula (H-I):

[Chem. 9]

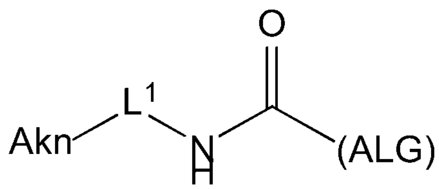

(H-I)

wherein (ALG) represents alginic acid; -NHCO- represents an amide bond via any carboxyl group of alginic acid; and $-L^1-$ is a divalent linker bonding to a cyclic alkyne group (Akn).

[Alginic acid derivative represented by formula (H-II)]

An alginic acid derivative represented by the following formula (H-II):

[Chem. 10]

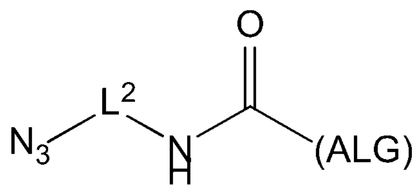

(H-II)

wherein (ALG) represents alginic acid; -NHCO- represents an amide bond via any carboxyl group of alginic acid; and $-L^2-$ is a divalent linker bonding to an azide group.

[3-6] The device or composite membrane according to [3-5], wherein,

in the formula (H-I), $-L^1-$ is $-(CH_2)_{n1}-$ (wherein n1 = 1 to 50 is satisfied, $-CH_2-$groups in the group are optionally replaced with 1 to 10 groups selected from -CO-, - CONH-, -NHCO-, -O-CONH-, -NHCO-O-, -O-, and -NH- or with a benzene ring, and the hydrogen atoms of the $-CH_2-$ groups are each optionally replaced with a $C_{1-3}$ alkyl group or a $C_{1-3}$ alkyl group substituted with a phenyl group),
Akn is a compound of an eight-membered cyclic alkyne group (wherein the cyclic alkyne group is optionally further

fused with one or two rings each being a benzene ring, a cyclopropane ring, or a 1,2,3-triazole ring and is optionally bound to $-L^1-$ in any fused ring, the eight-membered cyclic alkyne group is optionally an eight-membered ring group obtained by replacing $-CH_2-$ groups in the eight-membered cyclic alkyne group with one or two groups selected from -C(=O)-, - CONH-, and -NH-, and hydrogen atoms of the $-CH_2-$ groups in the alkyne group are optionally replaced with one or two groups selected from a $C_{1-3}$ alkyl group, a fluorine atom, a hydroxy group, and a $C_{1-3}$ alkyloxy group), and in the formula (H-II), $-L^2-$ is $-(CH_2)_{n2}-$ (wherein n2 = 1 to 50 is satisfied, - $CH_2$- groups in the group are optionally replaced with 1 to 10 groups selected from - CONH-, -NHCO-, -O-, and -NH- or with a benzene ring or a pyridine ring, and the hydrogen atoms of the $-CH_2-$ groups are each optionally replaced with a $C_{1-3}$ alkyl group).

[3-7] The device or composite membrane according to [3-5] or [3-6], wherein,

in the formula (H-I), $-L^1-$ is $-(CH_2)_{n1}-$ (wherein n1 = 2 to 15 is satisfied, $-CH_2$-groups in the group are optionally replaced with one to five groups selected from - CO-, -CONH-, -NHCO-, -O-CONH-, -NHCO-O-, -O-, and -NH- or with a benzene ring, and the hydrogen atoms of the $-CH_2-$ groups are each optionally replaced with a $C_{1-3}$ alkyl group or a $C_{1-3}$ alkyl group substituted with a phenyl group),
Akn is a compound of an eight-membered cyclic alkyne group (wherein the cyclic alkyne group is optionally further fused with one or two benzene rings and is optionally an eight-membered ring group obtained by replacing $-CH_2-$ in the eight-membered cyclic alkyne group with -NH-), and
in the formula (H-II), $-L^2-$ is $-(CH_2)_{n2}-$ (wherein n2 = 2 to 15 is satisfied, - $CH_2$- groups in the group are optionally replaced with one to five groups selected from -CONH-, -NHCO-, -O-, and -NH- or with a benzene ring).

[3-8] The device or composite membrane according to any one of [3-4] to [3-7], wherein the chemical cross-linking is chemical cross-linking by a combination of alginic acid derivatives shown in the following formulas (H-I) and (H-II).

[Alginic acid derivative represented by formula (H-I)]

An alginic acid derivative represented by the following formula (H-I):

[Chem. 11]

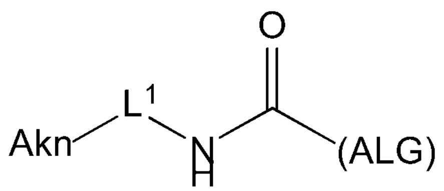

(H-I)

wherein (ALG) represents alginic acid; -NHCO- represents an amide bond via any carboxyl group of alginic acid; $-L^1-$ represents a divalent linker selected from the group of the following partial structural formulas [in each of the formulas, the outsides of the wavy lines at the two ends are not included]:

[Chem. 12]

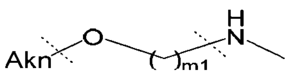

(LN-1) m1=2-6

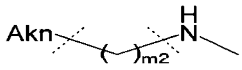

(LN-2) m2=1-6

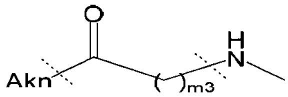

(LN-3) m3=1-6

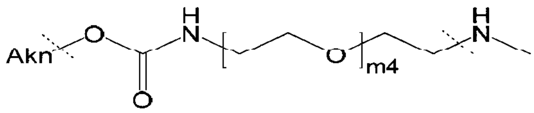

(LN-4) m4=1-6

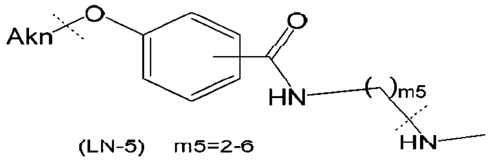

(LN-5) m5=2-6

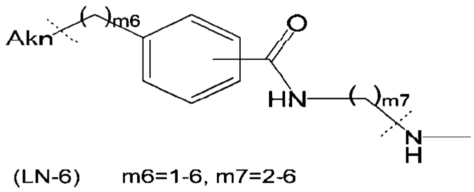

(LN-6) m6=1-6, m7=2-6

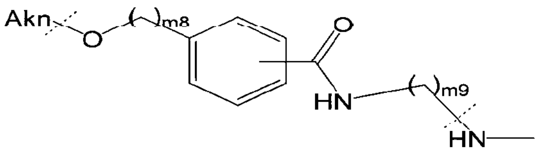

(LN-7) m8=1-6, m9=2-6

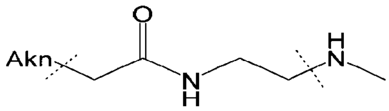

(LN-8)

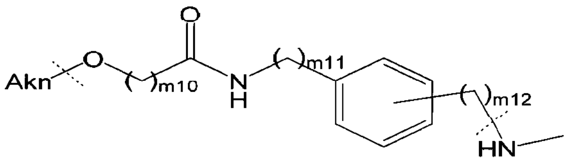

(LN-9) m10=1-4, m11=1-6,m12=1-6

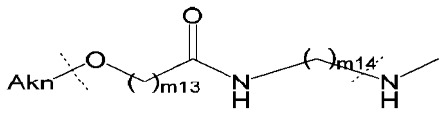

m13=1-4, m14=2~6

(LN-10)

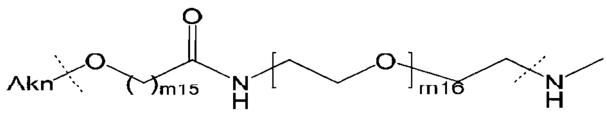

m15=1-4, m16=1-6

(LN-11)

Akn represents a cyclic alkyne group selected from the group of the following partial structural formulas [in each of the formulas, the right side of the wavy line is not included]:

[Chem. 13]

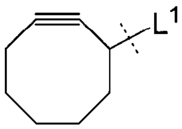

(AK-1)

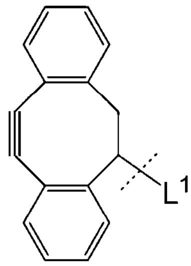

(AK-2)

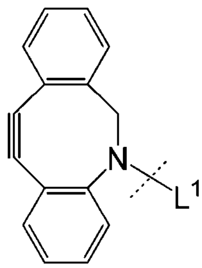

(AK-3)

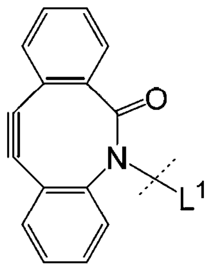

(AK-4)

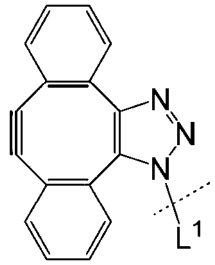

(AK-5)

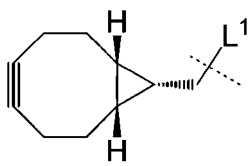

(AK-6)

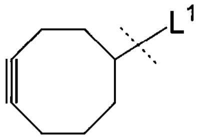

(AK-7)

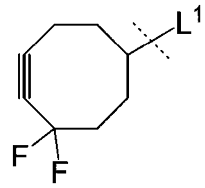

(AK-8)

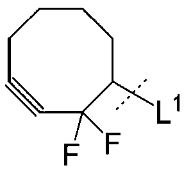

(AK-9)

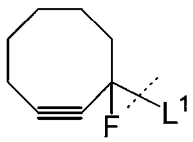

(AK-10)

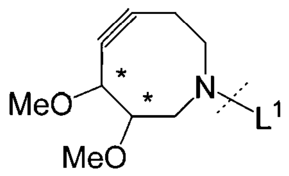

(AK-11)

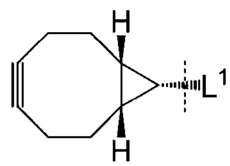

(AK-12)

wherein each asterisk symbol indicates a chiral center.

[Alginic acid derivative represented by formula (H-II)]

An alginic acid derivative represented by the following formula (H-II):

[Chem. 14]

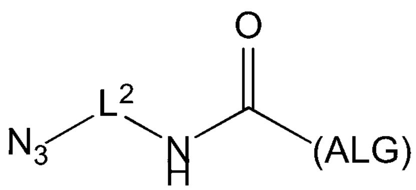

(H-II)

wherein (ALG) represents alginic acid; -NHCO- represents an amide bond via any carboxyl group of alginic acid; $-L^2-$ represents a divalent linker selected from the group of the following partial structural formulas [in each of the formulas, the outsides of the wavy lines at the two ends are not included]:

[Chem. 15]

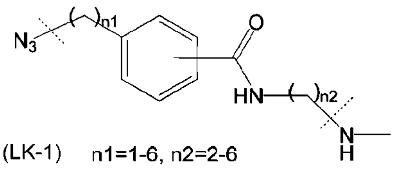

(LK-1) n1=1-6, n2=2-6

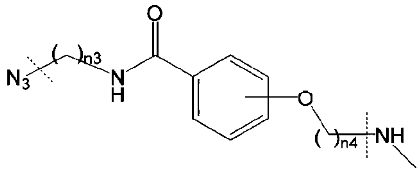

(LK-2) n3=2-6, n4=2-6

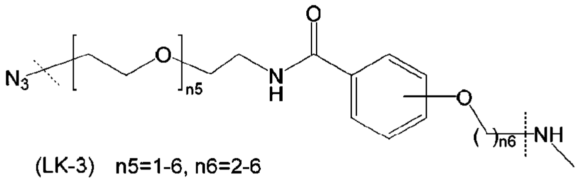

(LK-3) n5=1-6, n6=2-6

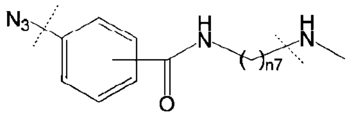

(LK-4) n7=2-6

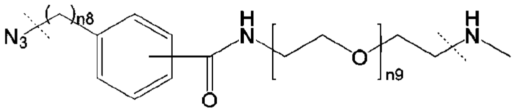

(LK-5) n8=1-4, n9=1-6

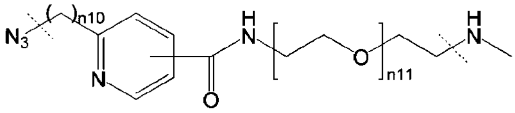

(LK-6) n10=1-4, n11=1-6

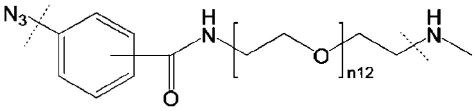

(LK-7) n12=1-6

[3-9] The device or composite membrane according to any one of [3-5] to [3-8], wherein the alginic acid derivative represented by the formula (H-I) is the following formula (A01) or (A02), and the alginic acid derivative represented by the formula (H-II) is the following formula (N01), (N02), (N03), or (N04):

[Chem. 16]

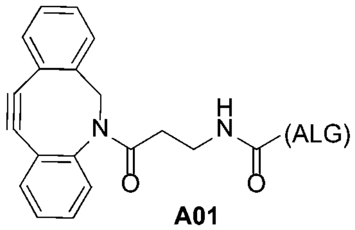

A01

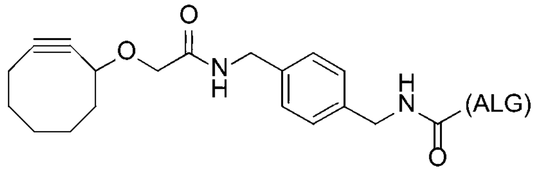

A02

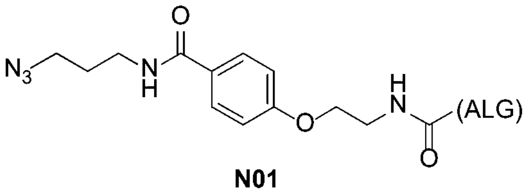

N01

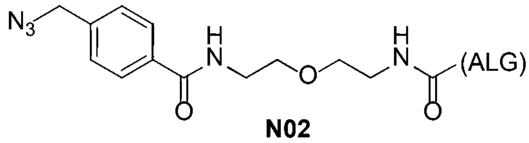

N02

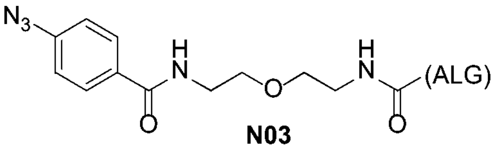

N03

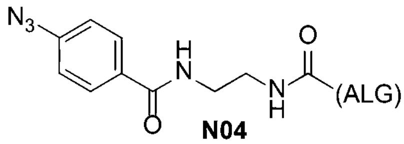

N04

wherein (ALG) represents alginic acid; and -NHCO- bonding to (ALG) represents an amide bond via any carboxyl group of alginic acid.

[3-10] The device or composite membrane according to any one of [3-4] to [3-9], wherein the chemically cross-linked alginic acid derivative is a cross-linked alginic acid in which any carboxyl group of a first alginic acid and any carboxyl group of a second alginic acid are bound via the following formula (H-III-L):

[Chem. 17]

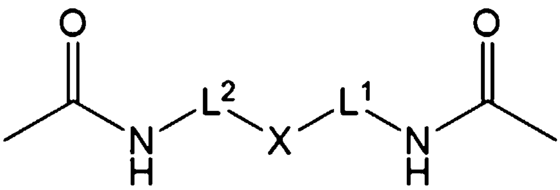

**(H-III-L)**

wherein -CONH- and -NHCO- at the two ends each represent an amide bond via any carboxyl group of alginic acid;

- $-L^1-$ is as defined in any one of [3-5] to [3-8];
- $-L^2-$ is as defined in any one of [3-5] to [3-8]; and
- X is a cyclic group selected from the group of the following partial structural formulas:

[Chem. 18]

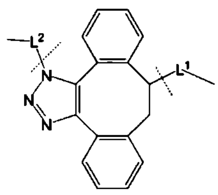
(TZ-1)

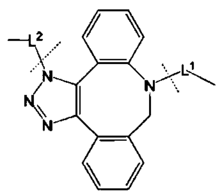
(TZ-2)

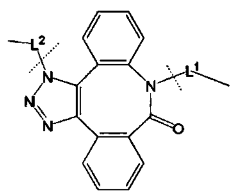
(TZ-3)

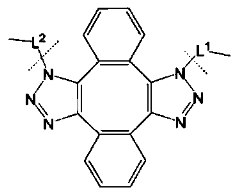
(TZ-4)

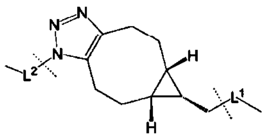
(TZ-5)

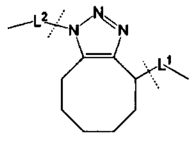
(TZ-6)

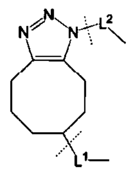
(TZ-7)

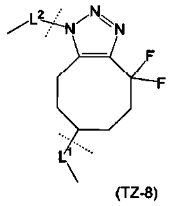
(TZ-8)

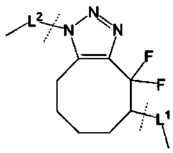
(TZ-9)

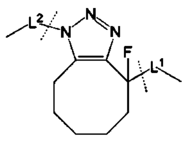
(TZ-10)

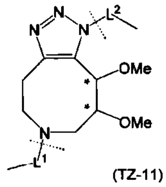
(TZ-11)

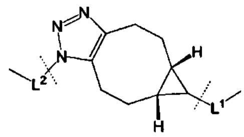
(TZ-12)

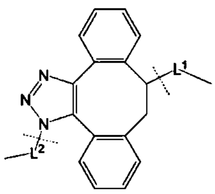
(TZ-1-r)

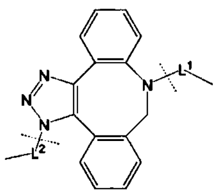
(TZ-2-r)

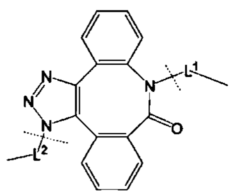
(TZ-3-r)

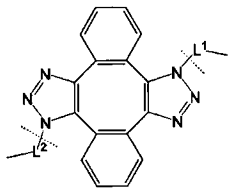
(TZ-4-r)

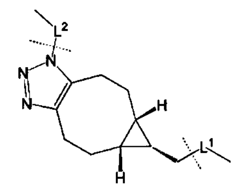
(TZ-5-r)

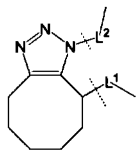
(TZ-6-r)

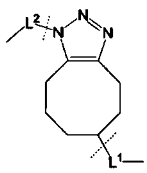
(TZ-7-r)

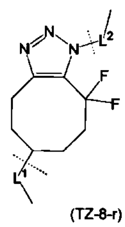
(TZ-8-r)

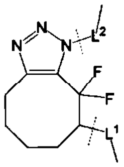
(TZ-9-r)

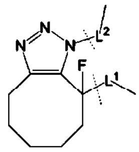
(TZ-10-r)

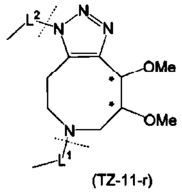
(TZ-11-r)

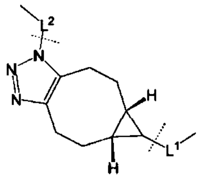
(TZ-12-r)

(in each of the formulas, the outsides of the dashed lines at the two ends are not included), wherein each asterisk symbol indicates a chiral center.

[3-11] The device or composite membrane according to [3-10], wherein, in the formula (H-III-L),

-$L^1$- is a divalent linker selected from the group of the following partial structural formulas (in each of the formulas, the outsides of the dashed lines at the two ends are not included):

[Chem. 19]

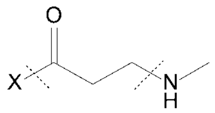

(LN-3-a)

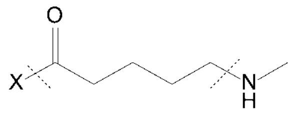

(LN-3-b)

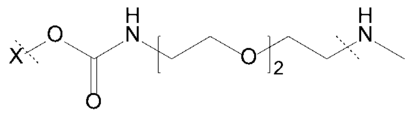

(LN-4-a)

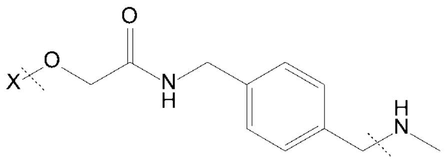

(LN-9-p-a)

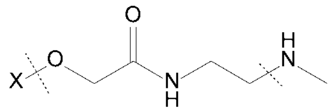

(LN-10-a)

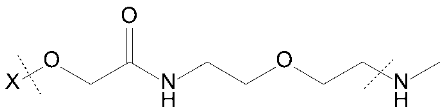

(LN-11-a)

-$L^2$- is a divalent linker selected from the group of the following partial structural formulas (in each of the formulas, the outsides of the dashed lines at the two ends are not included):

[Chem. 20]

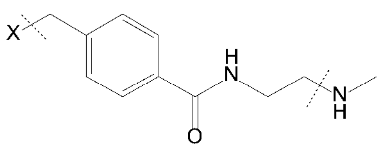

(LK-1-1-a)

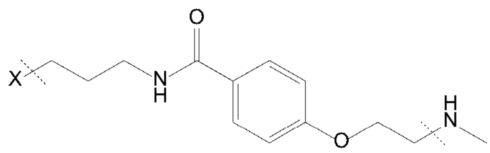

(LK-2-1-a)

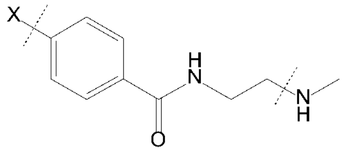

(LK-4-1-a)

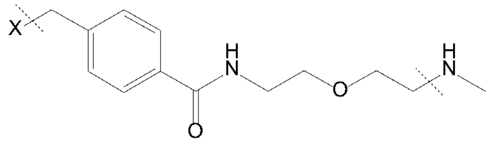

(LK-5-1-a)

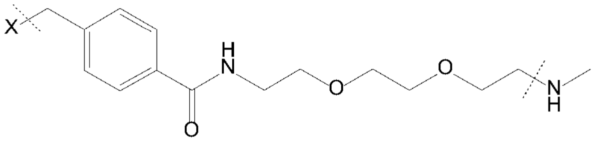

(LK-5-1-b)

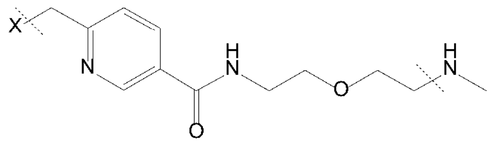

(LK-6-1-a)

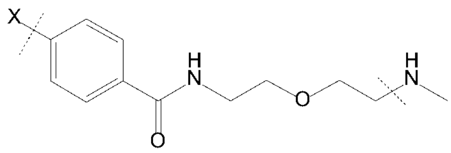

(LK-7-1-a)

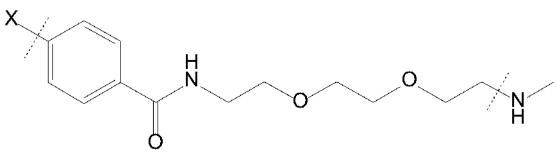

(LK-7-1-b)

and X is a cyclic group selected from the group of the following partial structural formulas (in each of the formulas, the outsides of the dashed lines at the two ends are not included):

[Chem. 21]

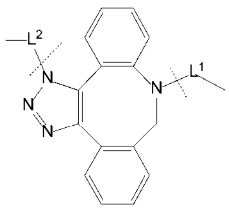

(TZ-2)

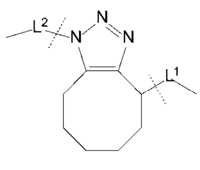

(TZ-6)

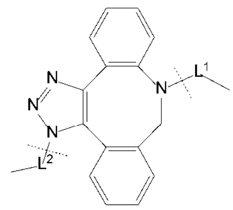

(TZ-2-r)

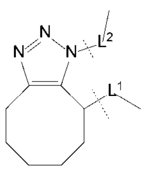

(TZ-6-r)

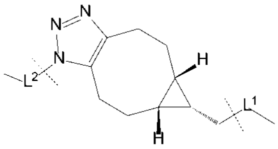

(TZ-5)

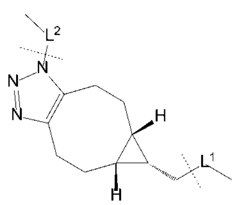

(TZ-5-r)

[3-12] The device or composite membrane according to [3-10] or [3-11], wherein a combination of $-L^2-X-L^1-$ in the formula (H-III-L) is selected from the group of the following partial structural formulas (in each of the formulas, the outsides of the dashed lines at the two ends are not included):

[Chem. 22]

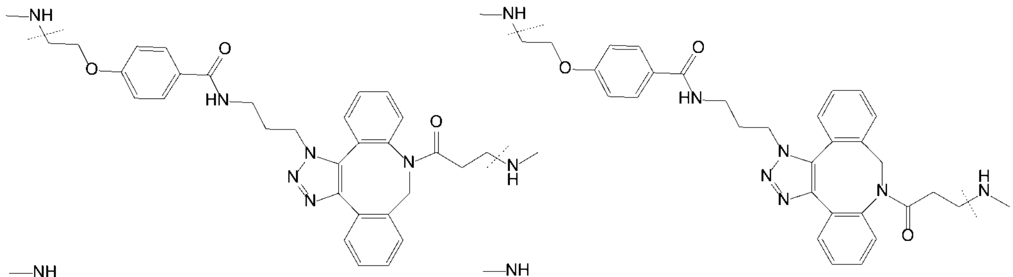

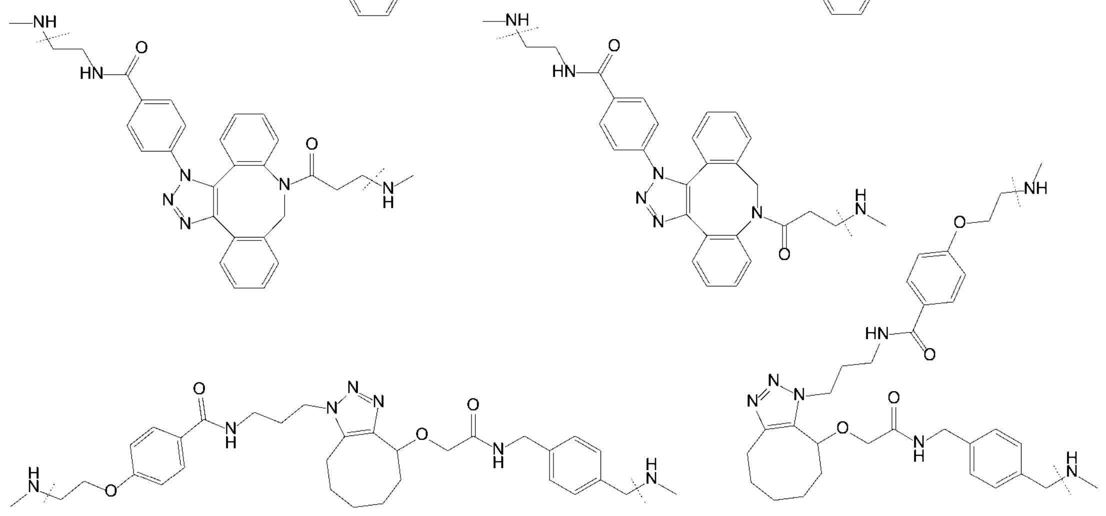

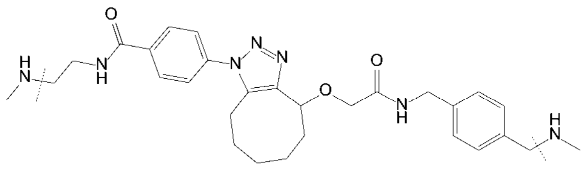

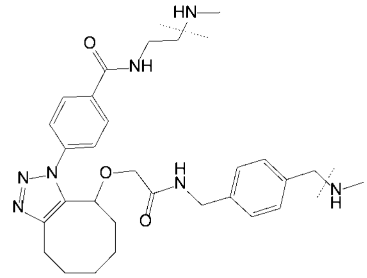

[Chem. 23]

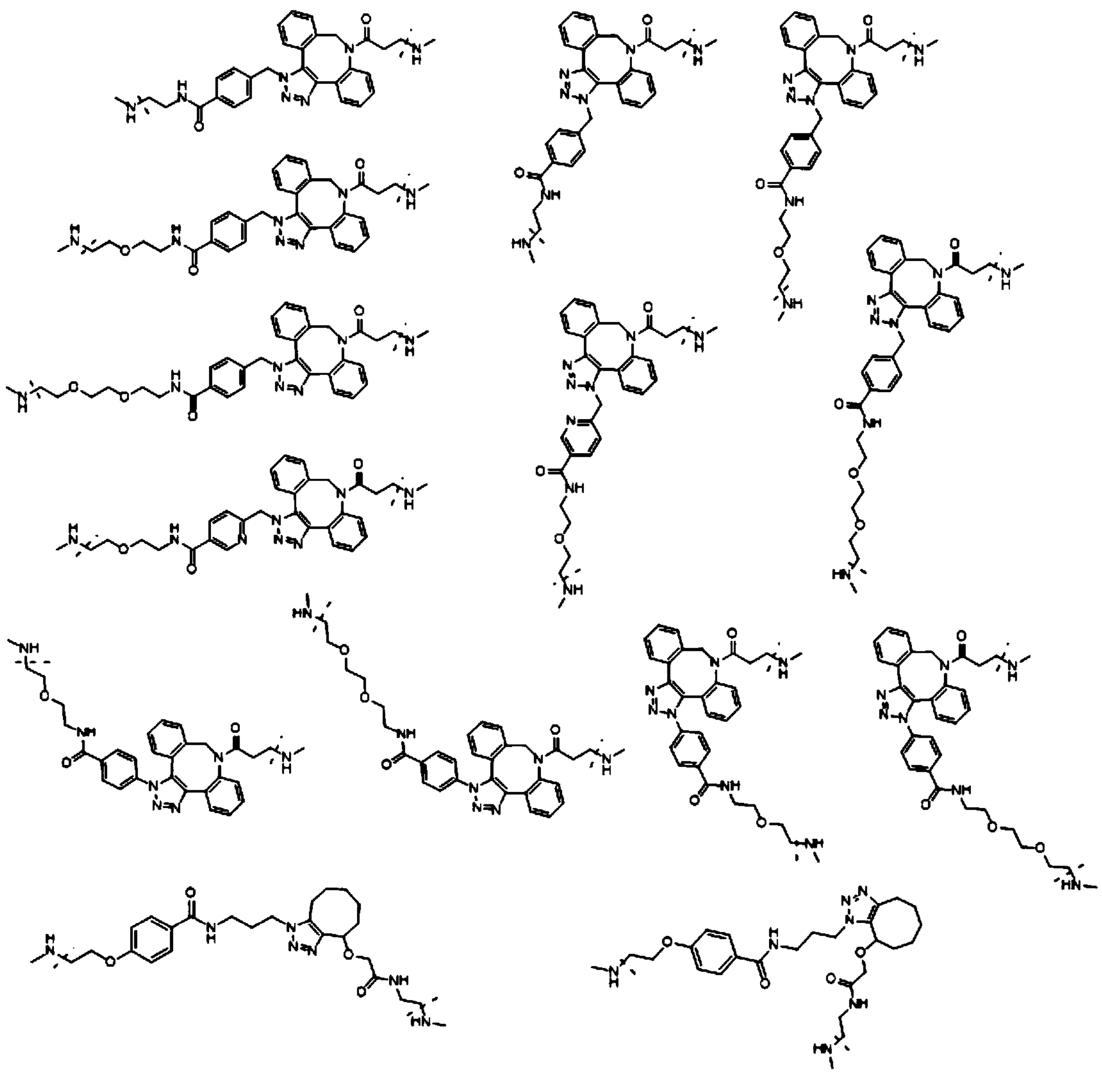

[3-13] The device or composite membrane according to any one of [3-10] to [3-12], wherein a combination of -$L^2$-X-$L^1$- in the formula (H-III-L) is selected from the group of the following partial structural formulas (in each of the formulas, the outsides of the dashed lines at the two ends are not included):

[Chem. 24]

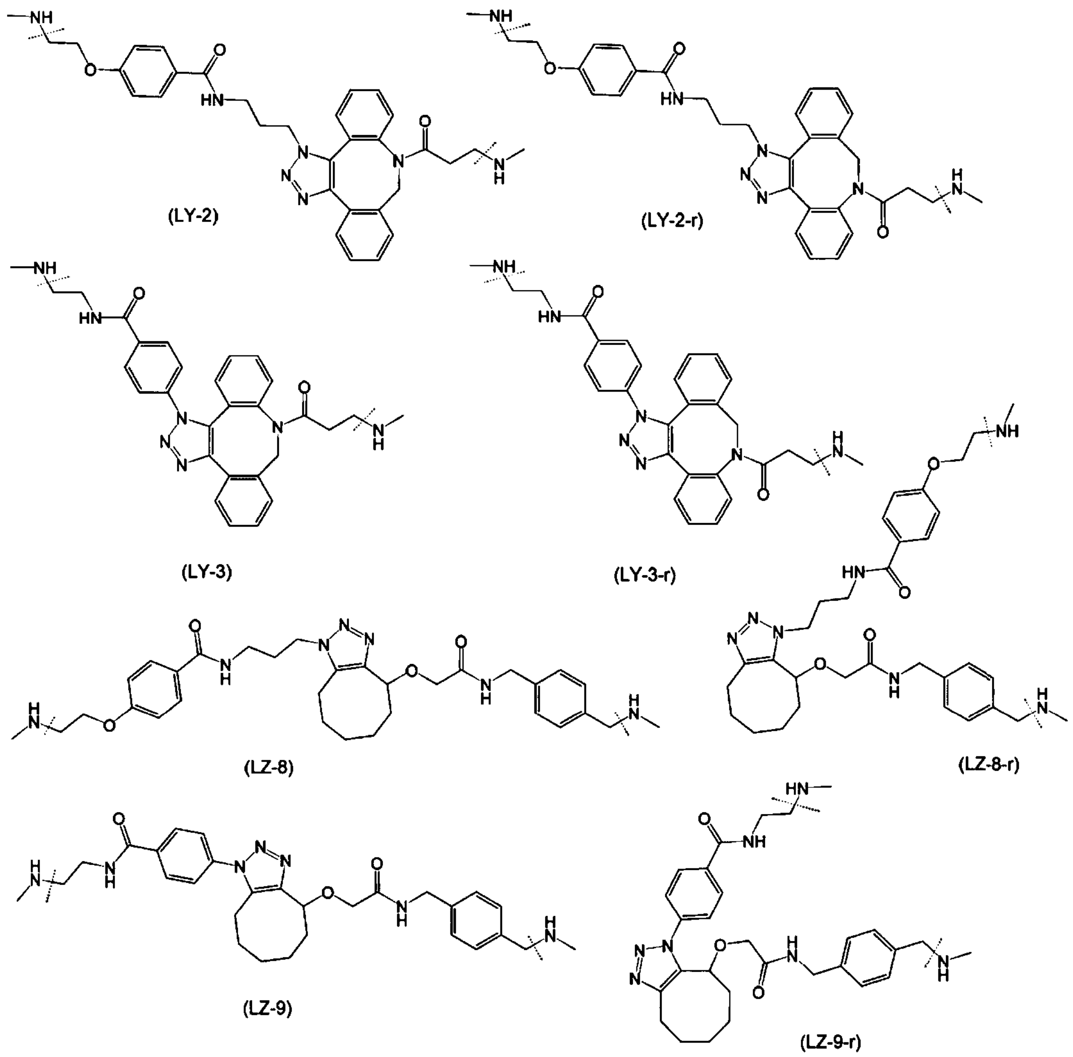

(LY-2) (LY-2-r)

(LY-3) (LY-3-r)

(LZ-8) (LZ-8-r)

(LZ-9) (LZ-9-r)

[3A] The biocompatible device according to [1A-9-2], wherein the hydrogel formed by gelation through cross-linking with the alginic acid derivatives represented by the formula (I-A) and formula (II-A) (cross-linked alginic acid gel) is a cross-linked alginic acid gel obtained by bonding via a cyclic group represented by the following formula (III-Lx):

[Chem. 25]

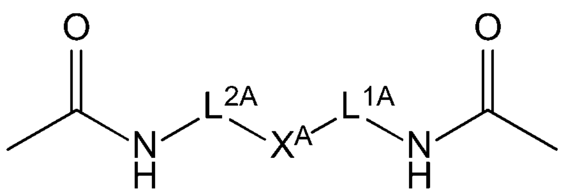

(III-Lx)

wherein -NHCO- at the right end is an amide bond derived from the alginic acid derivative of the formula (I-A), and -CONH- at the left end represents an amide bond derived from the alginic acid derivative of the formula (II-A);

-$L^{1A}$- and -$L^{2A}$- are as defined in the embodiment of [1A-9-2]; and
-$X^A$- represents the following partial structural formula:

[Chem. 26]

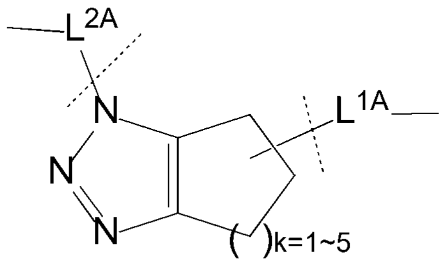

wherein one to four of the $-CH_2-$ groups in the $C_{5-9}$ cycloalkene ring are each optionally replaced with a group selected from the group consisting of -NH-, -S-, - O-, and =C(=O); one to five of the hydrogen atoms of the $-CH_2-$ groups in the $C_{5-9}$ cycloalkene ring are each optionally replaced with such a group as a halogen atom, a hydroxy group, an amino group, a keto group, a $C_{1-3}$ alkyl group, an $-O-C_{1-3}$ alkyl group, an-$NHC_{1-3}$ alkyl group, $-N(C_{1-3}$ alkyl group$)_2$, or -COO-M (M = Na, K, 1/2 Ca, a hydrogen atom, or a $C_{1-3}$ alkyl group); the $C_{5-9}$ cycloalkene ring is optionally fused with one to three rings each being a $C_{3-8}$ cycloalkyl ring, a benzene ring, or a five- or six-membered aromatic heterocycle; and if the $C_{5-9}$ cycloalkene ring is fused with a $C_{3-8}$ cycloalkyl ring, a benzene ring, or a five- or six-membered aromatic heterocycle, $-L^{1A}-$ optionally substitutes the $C_{3-8}$ cycloalkyl ring, benzene ring, or five- or six-membered aromatic heterocycle (in each of the formulas, the outsides of the dashed lines at the two ends are not included).

[3A-1] For preferable, more preferable, further preferable, particularly preferable, and most preferable $-L^{1A}-$ groups in [3A], the same definition as described for $-L^{1A}-$in [1A-9-3] is applied.

[3A-1-2] For preferable, more preferable, further preferable, and particularly preferable $-L^{1A}-$ groups in [3A], the same definition as described for $-L^{1A}-$in [1A-9-4] is applied.

[3A-2] For preferable, more preferable, further preferable, particularly preferable, and most preferable $-L^{2A}-$ groups in [3A], the same definitions as described for $-L^{2A}-$in [1A-9-6] are applied.

[3A-2-2] For preferable, more preferable, further preferable, and particularly preferable $-L^{2A}-$ groups in [3A], the same definition as described for $-L^{2A}-$ in [1A-9-7] is applied.

[3A-3] In the embodiment of [3A], $-X^A-$ is preferably a cyclic group selected from the group of the following partial structural formulas:

[Chem. 27]

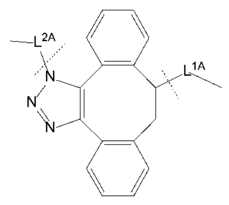
(TZ-1)

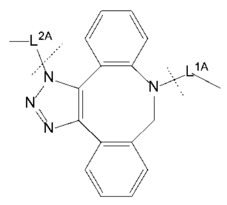
(TZ-2)

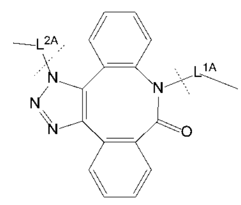
(TZ-3)

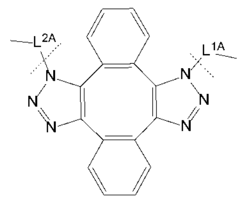
(TZ-4)

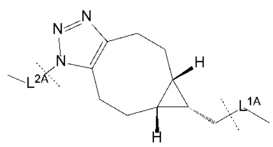
(TZ-5)

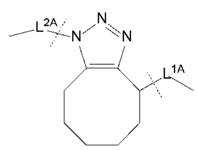
(TZ-6)

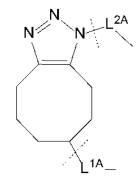
(TZ-7)

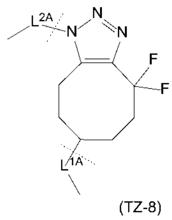
(TZ-8)

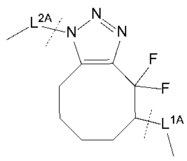
(TZ-9)

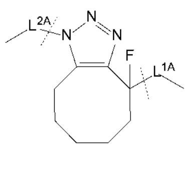
(TZ-10)

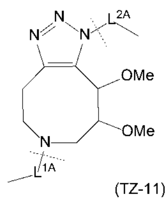
(TZ-11)

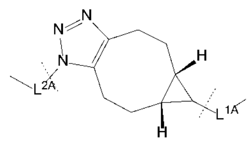
(TZ-12)

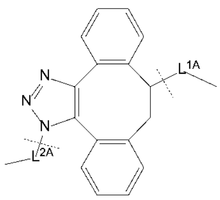
(TZ-1-r)

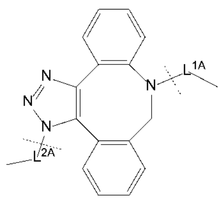
(TZ-2-r)

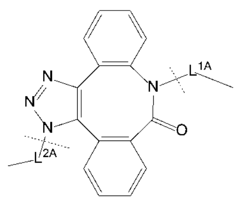
(TZ-3-r)

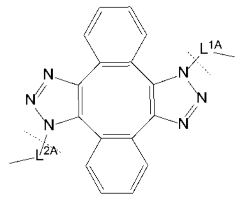
(TZ-4-r)

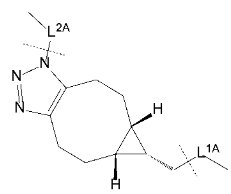
(TZ-5-r)

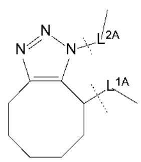
(TZ-6-r)

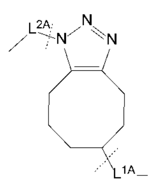
(TZ-7-r)

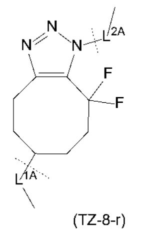
(TZ-8-r)

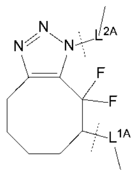
(TZ-9-r)

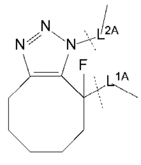
(TZ-10-r)

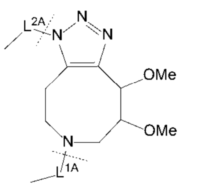
(TZ-11-r)

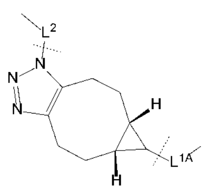
(TZ-12-r)

(in each of the formulas, the outsides of the dashed lines at the two ends are not included); more preferably a cyclic group selected from the group of the following partial structural formulas:

[Chem. 28]

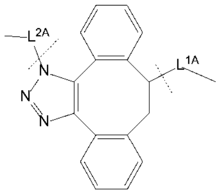
(TZ-1)

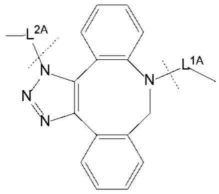
(TZ-2)

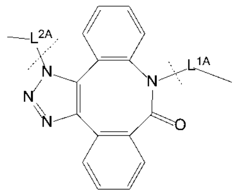
(TZ-3)

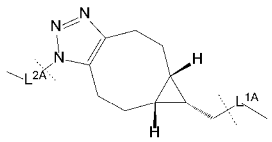
(TZ-5)

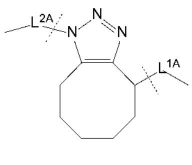
(TZ-6)

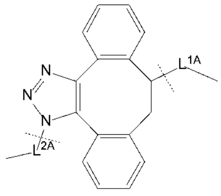
(TZ-1-r)

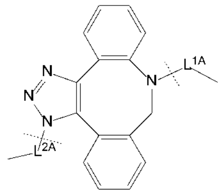
(TZ-2-r)

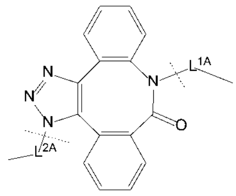
(TZ-3-r)

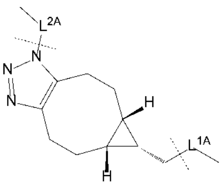
(TZ-5-r)

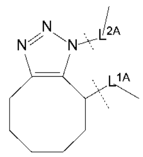
(TZ-6-r)

(in each of the formulas, the outsides of the dashed lines at the two ends are not included); and further preferably a cyclic group selected from the group of the following partial structural formulas:

[Chem. 29]

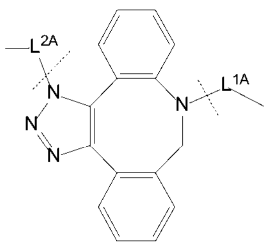
(TZ-2)

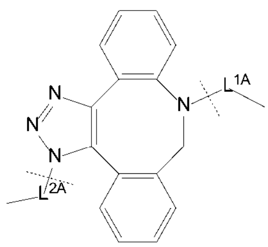
(TZ-2-r)

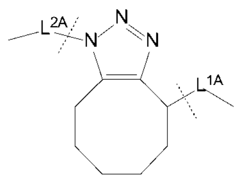
(TZ-6)

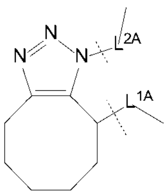
(TZ-6-r)

(in each of the formulas, the outsides of the dashed lines at the two ends are not included).

[3A-4] Any preferable embodiment of the formula (III-Lx) for the cross-linked alginic acid gel can be formed by appropriately combining the definitions of $-L^{1A}-$, $-L^{2A}-$, and $-X^{A}-$ described in [3A] to [3A-3].

[3A-5] In [3A], specific preferable combinations of $-L^{1A}-X^{A}-L^{2A}-$ in the formula (III-Lx) are those listed in the following table:

[Table 13]

| -$L^{2A}$- | -$X^{A}$- | -$L^{1A}$- |
|---|---|---|
| Linker selected from (L2A-1-X1), (L2A-2-XI), (L2A-3-X1), (L2A-4-X1), (L2A-5-XI), (L2A-5-X2), (L2A-6-XI), (L2A-7-X1), (L2A-7-X2), (L2AB-I-XI), (L2AB-2ab-X1), (L2AB-3-XI), (L2AB-3-X2), (L2AB-4-X1), and (L2AB-5ab-X1) | (TZ-2)<br>(TZ-2-r)<br>(TZ-5)<br>(TZ-5-r)<br>(TZ-6)<br>(TZ-6-r) | Linker selected from (L1A-3-X1), (L1A-3-X2), (L1A-4-X), (L1A-9-X), (L1A-10-X), (L1A-11-X), (L1AB-1-X), (LTAB-2-X), (L1IAB-3-X1), (L1AB-3-X2), (L1AB-4-X1), (L1AB-4-X2), (L1AB-5-X), (L1AB-6-X), (L1AB-7-X), (L1AB-8-X1), (L1AB-8-X2), and (L1AB-8-X2) |

and more preferable combinations are those listed in the following table:

[Table 14]

| -$L^{2A}$- | -$X^{A}$- | -$L^{1A}$- |
|---|---|---|
| (L2A-2-X1) | (TZ-2) | (L1A-3-X1) |
| (L2A-4-X1) | (TZ-2) | (L1A-3-X1) |
| (L2A-5-X1) | (TZ-2) | (L1A-3-X1) |
| (L2A-7-X1) | (TZ-2) | (L1A-3-X1) |
| (L2A-2-X1) | (TZ-2-r) | (L1A-3-X1) |
| (L2A-4-X1) | (TZ-2-r) | (L1A-3-X1) |
| (L2A-5-X1) | (TZ-2-r) | (L1A-3-X1) |
| (L2A-7-X1) | (TZ-2-r) | (L1A-3-X1) |
| (L2A-2-X1) | (TZ-6) | (L1A-9-X) |
| (L2A-4-X1) | (TZ-6) | (L1A-9-X) |
| (L2A-5-X1) | (TZ-6) | (L1A-9-X) |
| (L2A-7-X1) | (TZ-6) | (L1A-9-X) |
| (L2A-2-X1) | (TZ-6-r) | (L1A-9-X) |
| (L2A-4-X1) | (TZ-6-r) | (L1A-9-X) |
| (L2A-5-X1) | (TZ-6-r) | (L1A-9-X) |
| (L2A-7-X1) | (TZ-6-r) | (L1A-9-X) |

[4-1] The kit for living-donor transplantation according to [1-13], wherein the hydrogel comprises the alginic acid derivative or cross-linked alginic acid described in any one of [3-5] to [3-13].
[4-2] The production method according to [1-14], wherein the hydrogel comprises the alginic acid derivative or cross-linked alginic acid described in any one of [3-5] to [3-13].
[4-3] The production method according to [1-15], wherein the solution comprises an alginic acid solution containing the alginic acid derivative or cross-linked alginic acid described in any one of [3-5] to [3-13].
[4A-1] The kit for living-donor transplantation according to [1-13] or [1A-17], wherein the hydrogel comprises the alginic acid derivative or cross-linked alginic acid described in any one of [1A-9] to [1A-11] and [3A] to [3A-5].
[4A-2] The production method according to [1-14], [1A-18-1], or [1A-18-2], wherein the hydrogel comprises the alginic acid derivative or cross-linked alginic acid described in any one of [1A-9] to [1A-11] and [3A] to [3A-5].
[4A-3] The production method according to [1-15], [1A-19-1], or [1A-19-2], wherein the solution comprises an alginic acid solution containing the alginic acid derivative or cross-linked alginic acid described in any one of [1A-9] to [1A-11] and [3A] to [3A-5].

Advantageous Effects of Invention

The present invention provides, as an embodiment, a novel, practical biocompatible device. The present

invention provides, as an embodiment, a biocompatible composite membrane that can be used as a constituent member of the device.

Further, the present invention provides a method of producing the device, and a kit for living-donor transplantation, each as an embodiment.

## Brief Description of Drawings

[Figure 1] Figure 1 is a schematic diagram of the biocompatible device of the present invention in an embodiment.
[Figure 2] Figure 2 is a schematic diagram of a cross-section of the biocompatible composite membrane of the present invention in an embodiment.
[Figure 3] Figure 3 is a schematic diagram of a cross-section of the biocompatible device of the present invention in an embodiment.
[Figure 4] Figure 4 is a photograph of the biocompatible device of the present invention in an embodiment.

## Description of Embodiments

For numerical ranges shown herein, any upper limit value and lower limit value can be combined. If "3.0 to 10, 3.2 to 8" is shown as a numerical range, for example, a range of "3.0 to 8" and a range of "3.2 to 10" are also included in the numerical range shown herein. If "3.0 or more, 3.2 or more, and 10 or less, 8 or less" is shown as a numerical range, for example, a range of "3.0 to 8" and a range of "3.2 to 10" are also included in the numerical range shown herein.

For example, a statement of "5 to 10" as a numerical range shown herein means the range "5 or more and 10 or less".

Moreover, any upper limit value and lower limit value, selected as appropriate from options in the specification shown herein for upper limit values and lower limit values, can be combined to specify a numerical range from the lower limit to the upper limit value.

In addition, a plurality of requirements shown for preferable embodiments described herein can be combined.

### 1. Biocompatible device

Provided here is a biocompatible device having an internal space formed by a biocompatible composite membrane including a semi-permeable membrane layer and a non-woven fabric layer, wherein the semi-permeable membrane layer comprises a cellulose derivative formed on a non-woven fabric serving as a support and the non-woven fabric layer comprises the thermoplastic non-woven fabric, the semi-permeable membrane layer is disposed on an outer side and the non-woven fabric layer is disposed on an internal-space side in the device, and the internal space includes a cell or tissue.

Also provided is a biocompatible device including an internal space configured to accommodate a cell or tissue that secretes a biologically active substance, wherein the internal space is formed by disposing a biocompatible composite membrane including/consisting of a semi-permeable membrane layer and a non-woven fabric layer in the device such that the semi-permeable membrane layer is on an outer side and the non-woven fabric layer is on an internal-space side, the semi-permeable membrane layer comprises a cellulose derivative laminated on a non-woven fabric using the non-woven fabric as a support, and the non-woven fabric layer comprises a thermoplastic non-woven fabric.

Further provided is a biocompatible device including an internal space configured to accommodate a cell or tissue that secretes a biologically active substance, wherein the internal space is formed by disposing a biocompatible composite membrane having a cellulose derivative laminated on one surface of a non-woven fabric in the device such that the surface of the composite membrane with the cellulose derivative laminated thereon is on an outer side.

Herein, the "biocompatible device" is a device in which an internal space is formed by a biocompatible composite membrane including a semi-permeable membrane layer and a non-woven fabric layer, irrespective of the presence or absence of a cell or tissue enclosed therein. In other words, the "biocompatible device" is a device in which an internal space is formed by a biocompatible composite membrane having a cellulose derivative laminated on one surface of a non-woven fabric. Herein, the terms "biocompatible device" and "biocompatible composite membrane" are occasionally expressed in a simple manner as "device" and "composite membrane", respectively.

Herein, the term "biocompatibility" means characteristics that do not cause any problem with biological safety in retaining in a living body, examples of the characteristics include such a property that interaction between any material for living bodies that constitutes a biocompatible device and a living body, local reaction of tissue adjacent to the material for living bodies, or systemic reaction is not caused, or such reaction is reduced, and having those characteristics is expressed as having biocompatibility.

Herein, the expressions "a biocompatible device" and "a biocompatible composite membrane" mean "a device having biocompatibility" and "a composite membrane having biocompatibility", respectively.

Including not only a semi-permeable membrane layer but also a non-woven fabric layer, the biocompatible device of the present invention in preferable embodiments has proper material permeability in combination with satisfactory flexibility and strength, and is a device having physical characteristics suitable for long-term *in vivo* uses including transplantation into a living body.

When a cell or tissue is enclosed, with a hydrogel serving as a carrier, in the internal space of the biocompatible device of the present invention in preferable embodiments, the internal space exhibits superior preservation of the shape of the hydrogel. Accordingly, the internal space of the biocompatible device in preferable embodiments can provide an environment suitable for the survival and functional maintenance of a cell or tissue, and allow a cell or tissue enclosed in the internal space to exert its functions (e.g., hypoglycemic action due to glucose-responsive insulin secretion when an insulin-producing cell is used) for a long period of time.

Furthermore, with the configuration in which a semi-permeable membrane layer and a non-woven fabric layer (a biocompatible composite membrane having a cellulose derivative laminated on one surface of a non-woven fabric) are disposed as described above, the biocompatible device of the present invention in preferable embodiments is capable of serving as a highly safe device that causes less adhesion, inflammation, and the like.

The device in preferable embodiments has immunoisolatability.

Herein, the term "immunoisolatability" means properties to isolate a cell or tissue from the immune system of a recipient. Having the properties, the device can prevent the immunological rejection (including cellular immune response and humoral immune response) of a recipient in transplantation. The immunoisolatability can be adjusted through selection of permeability for the semi-permeable membrane as described later so as to block the intrusion of antibodies, immunocytes, and the like.

Herein, the term "cross-linking (reaction)" means "ionic cross-linking (reaction)" or "chemical cross-linking (reaction)". Herein, the term "ionic cross-linking (reaction)" means that ionic cross-linkages (ionic bonds) are formed between one alginic acid or alginic acid derivative and another in a certain embodiment via divalent or higher-valent metal ions. Herein, the term "chemical cross-linking (reaction)" means "cross-linking (reaction) by chemical bonding", and means that, through chemical reaction (e.g., Huisgen reaction) with one alginic acid derivative and another in a certain embodiment, chemical cross-linkages (chemical bonds) are formed between the alginic acid derivatives.

The following describes the components of the biocompatible device in detail.

1-1. Biocompatible composite membrane

Herein, the "biocompatible composite membrane" includes a semi-permeable membrane layer and a non-woven fabric layer. The semi-permeable membrane layer comprises a cellulose derivative formed on a non-woven fabric serving as a support, and the non-woven fabric layer comprises the thermoplastic non-woven fabric. Alternatively, the semi-permeable membrane layer comprises a cellulose derivative laminated on a non-woven fabric using the non-woven fabric as a support, and the non-woven fabric layer comprises a thermoplastic non-woven fabric.

Alternatively, the biocompatible composite membrane is a membrane having a cellulose derivative laminated on one surface of a non-woven fabric, and the non-woven fabric is thermoplastic.

(Semi-permeable membrane layer)

The semi-permeable membrane layer comprises a cellulose derivative formed on a non-woven fabric serving as a support. The semi-permeable membrane layer comprises a cellulose derivative laminated on a non-woven fabric using the non-woven fabric as a support. In some embodiments, the semi-permeable membrane layer includes a semi-permeable membrane comprising the cellulose derivative. In some other embodiments, the semi-permeable membrane layer consists of a semi-permeable membrane comprising the cellulose derivative.

Herein, the "semi-permeable membrane" is a membrane that is permeable to only molecules or ions smaller than a specific size. The semi-permeable membrane has such a property that when solutions of two concentrations, each being a system of a solute that does not permeate the semi-permeable membrane and a solvent that does, are brought into contact via the semi-permeable membrane, osmotic pressure is generated between one side and the other of the membrane, and only the solvent permeates the membrane.

Herein, the term "cellulose derivatives" is a collective term for compounds obtained by introducing other substituents into hydroxy groups contained in a cellulose molecule, and cellulose derivatives are classified into cellulose ethers with ether bonds (e.g., methylcellulose, ethylcellulose, ethylmethylcellulose, carboxymethylcellulose, carboxyethylcellulose, hydroxyethylcellulose, hydroxypropylcellulose, benzylcellulose, tritylcellulose, cyanoethylcellulose, aminoethylcellulose) and cellulose esters with ester bonds (e.g., cellulose acetate, diacetylcellulose, triacetylcellulose, cellulose propionate, cellulose butyrate).

In some embodiments, the cellulose derivative comprises a cellulose ester. In some embodiments, the cellulose ester comprises cellulose acetate. In some other embodiments, the semi-permeable membrane layer may comprise a non-cellulose-derivative resin as a constituent component of the semi-permeable membrane. Examples of the resin include, but are not limited to, such resins as polysulfone polymer, polyacrylonitrile polymer, polyamide polymer, and polycarbonate polymer.

In an embodiment of the present invention, the cellulose derivative content as a constituent component of the semi-permeable membrane is approximately 50 to approximately 100% by mass, approximately 60 to approximately 100% by mass in another embodiment, and approximately 70 to approximately 100% by mass in a still another embodiment, on the basis of the total amount of the constituent components of the semi-permeable membrane.

In an embodiment of the present invention, the non-cellulose-derivative resin content as a constituent component of the semi-permeable membrane is approximately 1 to approximately 50% by mass, approximately 1 to approximately 40% by mass in another embodiment, and approximately 1 to approximately 30% by mass in still another embodiment, on the basis of the total amount of the constituent components of the semi-permeable membrane.

Herein, the word "approximately", when no preferable range is shown, means that values within $\pm$10% of the numerical value, or within $\pm$20% of the numerical value in certain embodiments, may be included.

In another embodiment of the present invention, the semi-permeable membrane consists of the cellulose derivative. That is, in this embodiment, the semi-permeable membrane is substantially free of a non-cellulose-derivative resin.

Herein, the phrase "substantially free of' never excludes the case that a certain component is inevitably mixed in the production process, whereas it is preferable that the amount of such a component to be inevitably mixed be as small as possible.

In the embodiment in which the semi-permeable membrane is substantially free of a non- cellulose-derivative resin, as an embodiment of the present invention, the non-cellulose-derivative resin content is, specifically, less than approximately 0.1% by mass, less than approximately 0.05% by mass in another embodiment, and less than approximately 0.01% by mass in still another embodiment, on the basis of the total amount of the constituent components of the semi-permeable membrane.

Further, in an embodiment of the present invention, the semi-permeable membrane is substantially free of an ethylene-vinyl alcohol copolymer. The ethylene-vinyl alcohol copolymer content as a constituent component of the semi-permeable membrane is, specifically, less than approximately 0.1% by mass, less than approximately 0.05% by mass in another embodiment, and less than approximately 0.01% by mass in still another embodiment, on the basis of the total amount of the constituent components of the semi-permeable membrane.

Examples of the semi-permeable membrane to form the semi-permeable membrane layer include semi-permeable membranes having properties and functions equivalent to those of membranes or tubes for dialysis, specifically, semi-permeable membranes equivalent to Cellu-Sep T Tubular Membrane (Membrane Filtration Products, Inc.), Spectra Biotech Membrane (Repligen Corporation), and Spectra/Por CE Dialysis Tube (Repligen Corporation), each being a product name. The semi-permeable membrane can be produced, for example, by dissolving a resin containing the cellulose derivative in a solvent and solidifying the dissolved resin.

The semi-permeable membrane to be used here has "molecular weight cutoff". The term "molecular weight cutoff" means the size of maximum molecular weight to be substantially non-blocked. Molecules having molecular weights larger than the molecular weight cutoff are substantially inhibited from going in and out of the semi-permeable membrane. In some embodiments, the "molecular weight cutoff" of the semi-permeable membrane is 100 kDa, and 300 kDa in another embodiment. With use of a semi-permeable membrane having a molecular weight cutoff value of that numerical value, the semi-permeable membrane layer can keep constant rates of permeation of glucose, insulin, nutrients for cells, and small-molecular-weight components such as oxygen and at the same time have a function to inhibit the permeation of polymers such as antibodies and complements or immunocytes or the like.

For example, an Spectra/Por CE Dialysis Tube (Repligen Corporation), which is a cellulose ester dialysis tube, is sold under specifications of the cutoff value "MWCO" of 100 to 500 Da, 0.5 to 1 kDa, 3.5 to 5 kDa, 8 to 10 kDa, 20 kDa, 50 kDa, 100 kDa, 300 kDa, 1000 kDa, and so on. The unit symbol for Dalton is Da, and 1,000 Da is equivalent to 1 kDa.

Herein, the thickness of a layer obtained by subtracting the thickness of the non-woven fabric layer (non-woven fabric) (NLT: reference numeral 21 in Figure 2) described later from the thickness of the biocompatible composite membrane (MT) can be regarded as the thickness of the semi-permeable membrane layer (semi-permeable membrane) (SLT: reference numeral 22 in Figure 2), and can be determined from SLT = MT - NLT. For example, the thickness of the semi-permeable membrane layer (semi-permeable membrane) can be calculated in such a manner that the composite membrane is cut out of the biocompatible device into any size, a cross-section of the composite membrane is observed with a microscope or the like, and the thickness of the non-woven fabric is subtracted from the thickness of the whole composite membrane.

The thickness of the semi-permeable membrane layer (semi-permeable membrane) (SLT) is not limited, and examples thereof include such a thickness as 0 $\mu$m or more, approximately 0.1 $\mu$m or more, approximately 1.0 $\mu$m or more,

approximately 5.0 μm or more, or approximately 10.0 μm or more. The upper limit value of the thickness of the semi-permeable membrane layer (semi-permeable membrane) is not limited, and examples thereof include such a thickness as approximately 500 μm or less, approximately 400 μm or less, approximately 300 μm or less, approximately 200 μm or less, or approximately 100 μm. It should be noted that SLT = 0 μm is not permitted.

The composite membrane of the present invention in an embodiment has a structure with partial intrusion of a constituent component of the semi-permeable membrane (e.g., the cellulose derivative) into the non-woven fabric, and, as described later, the thickness of the part with partial intrusion of a constituent component of the semi-permeable membrane into the non-woven fabric (reference numeral 212 in Figure 2) is not included in the thickness of the semi-permeable membrane layer (semi-permeable membrane).

The semi-permeable membrane layer can be formed, for example, as described later in Examples, by applying a solution containing a cellulose acetate derivative onto one surface of a non-woven fabric by using a Loeb-Sourirajan method to laminate thereon.

Herein, the expressions "intrusion" and "intruding" mean that the cellulose derivative (semi-permeable membrane) has partially soaked from a surface of the non-woven fabric into the inside. For the intrusion of the cellulose derivative, reference can be made to description in "Non-woven fabric layer" shown in the following.

(Non-woven fabric layer)

The non-woven fabric forming the non-woven fabric layer may be any one that can serve as a support of the semi-permeable membrane without limitation. As a tendency, conventional semi-permeable membranes have difficulty in achieving sufficient strength against external force such as tearing. In addition, in the case that a cellulose derivative such as cellulose acetate is used as a semi-permeable membrane, operations of thermocompression bonding for the membrane are complicated because of the high melting point of the cellulose derivative, and the degree of sealing and strength of fused parts may be insufficient. In view of such circumstances, the present invention successfully achieves enhanced strength for the composite membrane by forming the composite membrane in such a manner that the semi-permeable membrane is laminated on a non-woven fabric by using the non-woven fabric as a support of the semi-permeable membrane. In addition, use of a thermoplastic non-woven fabric successfully gives enhanced thermal fusion properties for the composite membrane, reducing or preventing the leakage of a cell or tissue from the device, and furthermore the immunoisolatability of the device is successfully maintained.

Moreover, the unevenness of the non-woven fabric surface functions as an anchor for a hydrogel described later to be enclosed in the internal space of the biocompatible device. Because of this, for example, the shape of the hydrogel is kept in the internal space of the biocompatible device, and the maldistribution, folding, and collapse or the like of the gel in the internal space of the device are successfully prevented. Without wishing to be bound by any theory, that is expected to result in an enhanced availability of a cell or tissue embedded in the hydrogel, leading to a much higher therapeutic effect.

In an embodiment, the non-woven fabric layer includes a part with partial intrusion of a constituent component of the semi-permeable membrane (e.g., the cellulose derivative) into the non-woven fabric. That is, the non-woven fabric layer in an embodiment of the present invention includes a part without any intrusion of a constituent component of the semi-permeable membrane into the non-woven fabric and a part with partial intrusion of a constituent component of the semi-permeable membrane into the non-woven fabric. The part with partial intrusion of a constituent component of the semi-permeable membrane into the non-woven fabric is a part formed through such a process that a non-woven fabric is soaked in a solution of the cellulose derivative or the like to form the semi-permeable membrane layer and as a result a constituent component of the semi-permeable membrane (e.g., the cellulose derivative) intrudes into the inside of the network structure of the non-woven fabric.

Thus, the composite membrane of the present invention in an embodiment does not have a structure in which a semi-permeable membrane and a non-woven fabric have been simply laminated (e.g., a laminate structure), but has a structure in which a semi-permeable membrane and a non-woven fabric have been integrated through a process that a constituent component of the semi-permeable membrane partially enters the network structure of the non-woven fabric and the constituent component of the semi-permeable membrane solidifies. Accordingly, the device of the present invention in an embodiment does not include, as a composite membrane, any laminate membrane formed by a lamination process. This configuration successfully prevents the peeling-off, gaps, defects, and the like of the semi-permeable membrane, successfully making the composite membrane stable over time.

In the non-woven fabric layer in another embodiment of the present invention, a constituent component of the semi-permeable membrane (e.g., the cellulose derivative) may be totally intruding into the non-woven fabric. In this embodiment, the non-woven fabric layer does not have a part without any intrusion of a constituent component of the semi-permeable membrane (reference numeral 211 in Figure 2).

In a preferable embodiment of the present invention, the non-woven fabric layer includes a part without any intrusion of a constituent component of the semi-permeable membrane (reference numeral 211 in Figure 2) and a part with partial intrusion of a constituent component of the semi-permeable membrane into the non-woven fabric (reference

numeral 212 in Figure 2), from the viewpoint of maintaining the thermal fusion properties of the non-woven fabric layer.

Herein, the term "fusion" means a state attained through a process that fibers melt and bond to each other.

Herein, the term "sealing" means enclosing in such a manner that, for example, two sheets of the biocompatible composite membrane are overlapped in such a manner that each semi-permeable membrane layer is disposed as an outer surface, and a part in the range of several millimeters to several centimeters from the periphery of the membrane is then heated to allow the thermoplastic non-woven fabric layers to fuse together.

For the non-woven fabric, a thermoplastic resin can be used as a raw material. Specific examples of such resins include polyolefin resins such as polyethylene (PE) and polypropylene (PP), polyester resins such as polyethylene terephthalate (PET), fluororesins such as polytetrafluoroethylene (PTFE), polyvinyl resins such as polystyrene (PS), and polyamide resins such as nylon. One of these resins may be used; alternatively, two or more thereof may be used. In a preferable embodiment of the present invention, the non-woven fabric comprises a resin having a melting point in the range of approximately 90 to approximately 180°C, from the viewpoint of enhancing the thermal fusion properties. Especially, the non-woven fabric preferably at least comprises a polyolefin resin or polyester resin having a melting point in the range of approximately 90 to approximately 180°C, for example, preferably comprises at least polyethylene, from the viewpoint of enhancing the strength of the device and the thermal fusion properties of the composite membrane.

The non-woven fabric in an embodiment of the present invention may be formed of a fiber comprising two components having different melting points. Specifically, the non-woven fabric in an embodiment of the present invention may be formed of, for example, a core-in-sheath composite fiber. In a preferable embodiment of the present invention, the non-woven fabric may be a non-woven fabric formed of a core-in-sheath fiber having thermal fusion properties, from the viewpoint of enhancing the thermal fusion properties. More specific examples of such a non-woven fabric include a non-woven fabric formed of a core-in-sheath fiber having polypropylene or polyethylene terephthalate as a core material and polyethylene as a sheath material.

From that viewpoint, it is preferable not to use a biodegradable or bioabsorbable resin for the non-woven fabric. Examples of the biodegradable or bioabsorbable resin include polylactic acid, polyglycolic acid, and poly-p-dioxane.

The non-woven fabric in an embodiment of the present invention is substantially free of an ethylene-vinyl alcohol copolymer. In an embodiment of the present invention, the ethylene-vinyl alcohol copolymer content as a constituent component of the non-woven fabric is less than approximately 0.1% by mass, less than approximately 0.05% by mass in another embodiment, and less than approximately 0.01% by mass in still another embodiment, on the basis of the total amount of the constituent components of the non-woven fabric.

In an embodiment of the present invention, the thickness of the non-woven fabric layer (non-woven fabric) corresponds to the thickness of the non-woven fabric itself, irrespective of the degree of intrusion of a constituent component of the semi-permeable membrane. The thickness of the non-woven fabric layer (non-woven fabric) is specifically approximately 10 to approximately 1000 μm, approximately 50 to approximately 750 μm in another embodiment, and approximately 100 to approximately 500 μm in still another embodiment. In an embodiment of the present invention, the basis weight (weight per unit area) of the non-woven fabric is approximately 5 to approximately 100 $g/m^2$, approximately 10 to approximately 75 $g/m^2$ in another embodiment, and approximately 15 to approximately 50 $g/m^2$ in still another embodiment.

(Biocompatible composite membrane)

In an embodiment of the present invention, the thickness of the biocompatible composite membrane can be the sum total of the thickness of the non-woven fabric itself and the thickness of the semi-permeable membrane layer, irrespective of the degree of intrusion of a constituent component of the semi-permeable membrane into the non-woven fabric. Specifically, the thickness of the biocompatible composite membrane is approximately 10 to approximately 1500 μm in an embodiment, approximately 25 to approximately 800 μm in another embodiment, approximately 25 to approximately 400 μm in still another embodiment, and approximately 100 to approximately 300 μm in still another embodiment.

As described later in Examples, for example, the biocompatible composite membrane of the present invention is formed by laminating a cellulose derivative on one surface of a non-woven fabric by using a Loeb-Sourirajan method, and occasionally referred to as an asymmetric porous membrane.

1-2. Shape, structure, etc., of biocompatible device

The biocompatible device of the present invention has an internal space formed by a biocompatible composite membrane including a semi-permeable membrane layer and a non-woven fabric layer. In the device, the semi-permeable membrane layer is disposed on an outer side and the non-woven fabric layer is disposed on an internal-space side, and the internal space includes a cell or tissue that secretes a biologically active substance.

Alternatively, the biocompatible device of the present invention has an internal space formed by a biocompatible

composite membrane having a cellulose derivative laminated on one surface of a non-woven fabric. In the device, the surface of the biocompatible composite membrane with the cellulose derivative laminated thereon is disposed on an outer side, and the internal space includes a cell or tissue that secretes a biologically active substance.

For example, Figure 1 is a schematic diagram of the biocompatible device of the present invention in an embodiment. In Figure 1, a biocompatible device 1 has an internal space 3 formed by a biocompatible composite membrane 2. The internal space 3 is hollow in order to enclose a cell or tissue 4. The shape, capacity, and the like of the internal space 3 are not limited, and can be appropriately designed to fit with the application of the device 1. In an embodiment, the internal space 3 may be formed by partial fusion of a non-woven fabric layer (non-woven fabric) 21. In an embodiment, the cell or tissue 4 may be enclosed in the internal space 3 with a hydrogel 5 serving as a carrier. In another embodiment, the cell or tissue 4 may lack the hydrogel 5 serving as a carrier. In this case, the cell or tissue 4 mixed with a culture solution or the like can be enclosed in the internal space 3.

Figure 2 is a schematic diagram illustrating a cross-section of the biocompatible composite membrane 2 of the present invention in an embodiment. In Figure 2, a semi-permeable membrane layer (semi-permeable membrane) 22 constituting the biocompatible composite membrane 2 is disposed on the outer side of the biocompatible device 1 and the non-woven fabric layer (non-woven fabric) 21 is disposed on the internal-space 3 side of the biocompatible device 1. As described above, the non-woven fabric layer (non-woven fabric) 21 may have a part 211 without any intrusion of a constituent component of the semi-permeable membrane and a part 212 with partial intrusion of a constituent component of the semi-permeable membrane.

The shape of the biocompatible device is not limited to the shape shown in Figure 1, and any shape is acceptable as long as the internal space is formed by the biocompatible composite membrane.

Examples of methods for forming the internal space by the biocompatible composite membrane include, but are not limited to, a method in which two sheets of the biocompatible composite membrane (e.g., squares, rectangles) are prepared and overlapped to face each other in such a manner that each semi-permeable membrane layer is on the outer side and each non-woven fabric layer is on the internal-space side, and the periphery is fused to seal (reference numeral 6 in Figure 3 corresponds to the fusion site), and, as another embodiment, a method in which one sheet of the biocompatible composite membrane (e.g., a square, a rectangle) is prepared and folded to overlap in such a manner that the semi-permeable membrane layer is on the outer side and the non-woven fabric layer is on the internal-space side, and the periphery is fused to seal.

Although not illustrated, the device in an embodiment may have one internal space formed therein, or a plurality of internal spaces formed therein. For forming a plurality of internal spaces, for example, one internal space may be divided into a plurality of internal spaces, and another example is a method of linking a plurality of devices each having one internal space into one device having a plurality of internal spaces.

The device can be designed, for example, to have a shape and size fitting with the site to be subjected to transplantation. Specific examples of the shape of the device include a plate-like shape. The term plate means a flat sheet, and refers to a sheet-like shape having an almost constant thickness and a broad area. Examples of the shape of the sheet include a flat sheet-like shape such as a polygon such as a triangle, a quadrilateral, and a pentagon and a circle. The thickness of the plate-shaped device is approximately 0.1 to approximately 100 mm in a certain embodiment, approximately 0.2 to approximately 50 mm in another embodiment, approximately 0.5 to approximately 20 mm in still another embodiment, and approximately 1 to approximately 20 mm in still another embodiment. In a certain embodiment, the plate-shaped device has a thickness that corresponds to the aforementioned thickness and is almost constant throughout the plate-like shape. The variation of thickness in the sheet-shaped device is preferably within $\pm 10\%$, and more preferably within $\pm 5\%$.

The thickness of the plate-shaped device corresponds to the thickness of a thickest part of the device. For example, the device may have a shape looking like a rugby ball, which is slightly thinner in its two end parts and thicker at the center than in the end parts. In the case of such a shape, the thickness of the device is the thickness near the center, which is a thickest part of the device.

In some embodiments, the plate-shaped device is approximately 1 to approximately 200 mm in length, approximately 1 to approximately 200 mm in width, and approximately 0.2 to approximately 50 mm in thickness, as the shape is represented as length $\times$ width $\times$ thickness. In some other embodiments, the plate-shaped device has an area of approximately 1 to approximately 40000 $mm^2$ as the area is represented as length $\times$ width, and a thickness of approximately 0.2 to approximately 50 mm, approximately 0.5 to approximately 50 mm, or approximately 1 to approximately 50 mm. In those embodiments, the device may have such a shape as a circle, a quadrilateral, a hexagon, or an octagon.

In the case that the device is designed to have a tubular shape, the shape of the device may be any tubular or fiber-like slender shape without limitation. The tubular shape is not limited to such a shape that the cross-sectional shape in the direction perpendicular to the central axis is circular, and may be any of asymmetric structures and distorted shapes, including a shape whose cross-section is a polygon such as a triangle, a quadrilateral, and a pentagon, and a shape whose cross-section is an oval. In a certain embodiment, the cross-sectional shape is circular. Also permitted are a ring shaped by

linking the two ends of a tube, a chain formed by linking a plurality of such rings, a product by bundling tubes, and a product by linking tubes into a sheet.

In the case that the device is designed to have a tubular shape, the diameter of the cross-section (hereinafter, the major axis or maximum diameter in the case of a non-circular shape) is, for example, approximately 0.1 to approximately 100 mm in a certain embodiment, approximately 0.2 to approximately 50 mm in another embodiment, approximately 0.5 to approximately 20 mm in still another embodiment, and approximately 1 to approximately 10 mm in still another embodiment.

In the case that the device is designed to have a tubular shape, the length is not limited, and approximately 10 mm to approximately 5 m in a certain embodiment, approximately 10 mm to less than approximately 30 cm in another embodiment, and approximately 30 cm to approximately 5 m in still another embodiment.

1-3. Cell or tissue to be enclosed in biocompatible device

Examples of a cell or tissue that can be enclosed in the biocompatible device include, but are not limited to, a cell or tissue that secretes a biologically active substance (a substance having pharmacological activity or bioactivity).

The biologically active substance is preferably a substance that is used as a pharmaceutical, but is not limited thereto, and examples thereof include low-molecular-weight pharmaceuticals, middle-molecular-weight pharmaceuticals such as peptides and oligonucleic acids, and biopharmaceuticals and bioactive substances such as proteins. Herein, bioactive substances include not only bioactive substances originally possessed by living bodies (bioactive natural substances), but also substances obtained by modifying or altering a bioactive substance, and substances that activate or inhibit bioactivity, including naturally occurring substances and substances produced by genetic engineering or chemically synthesized, as well as prodrugs of them. Specific examples can include vitamins, coenzymes, hormones, antibiotics, neurotransmitters, cytokines, enzymes, growth factors, antibodies, and other biologic factors; more specifically, insulin, dopamine, factor VIII, and factor IX.

Acceptable as the cell that secretes a biologically active substance is not only a natural cell but also a cell subjected to any artificial alteration operation, as well as a cell cluster consisting of a plurality of cells. The cell that secretes a biologically active substance may be a cell for transplantation. Examples of the cell for transplantation can include an insulin-secreting cell, a pancreatic islet, and a pancreatic islet cell.

Other examples of the cell that can secrete a biologically active substance can include a dopamine-secreting cell, a pituitary cell, a growth hormone-secreting cell, a parathyroid gland cell, a nerve growth factor-secreting cell, a blood coagulation factor-secreting cell, a hepatocyte, a parathyroid cell, an erythropoietin-secreting cell, and a norepinephrine-secreting cell.

The term "an insulin-secreting cell" means a cell having a function of secreting insulin, and, in the case of a cell constituting the pancreatic islet, the term means a β cell, which secretes insulin. The "insulin-secreting cell" may be a cell that has come to have an insulin secretion function through differentiation, mature, or alteration or the like, and examples thereof can include a cell having an insulin secretion function obtained by differentiating a stem cell such as an iPS cell, ES cell, or somatic stem cell (e.g., a mesenchymal stem cell), a cell having an insulin secretion function obtained by maturing a juvenile cell or progenitor cell, and a cell to which an insulin secretion function has been imparted by gene recombination. Here, differentiating or maturing such cells includes culturing such cells; that is, cells obtained by differentiating or maturing can include cells obtained by culturing.

A "pancreatic islet", which is also called an islet of Langerhans, is a cell cluster composed of approximately 2000 pancreatic islet cells on average. A pancreatic islet is composed of five types of cells: α cells, which secrete glucagon, β cells, which secrete insulin, δ cells, which secrete somatostatin, ε cells, which secrete ghrelin, and PP (pancreatic polypeptide) cells, which secrete pancreatic polypeptide.

Acceptable herein as the "pancreatic islet cell" is one including at least one type of a cell among the five types of cells constituting a pancreatic islet, but the "pancreatic islet cell" preferably includes at least a β cell. In some embodiments, the pancreatic islet cells may be a mixture comprising all types of cells of α cells, β cells, δ cells, ε cells, and PP cells, or be in such a state that the pancreatic islet cells are contained in a pancreatic islet.

The "pancreatic islet cell" may be one formed through differentiation, maturation, or alteration or the like. In this case, a pancreatic islet cell obtained by differentiating a stem cell such as an iPS cell, ES cell, and somatic stem cell (e.g., a mesenchymal stem cell) and a pancreatic islet cell obtained by maturing a juvenile cell or progenitor cell can also be acceptable as the "pancreatic islet cell".

In the case of use for transplantation application, the "insulin-secreting cell" or "pancreatic islet (including a pancreatic islet cell)" preferably has survivability and functions to a degree that allows a patient who has received transplantation to recover from the pathologic condition. Examples of the functions of the insulin-secreting cell, pancreatic islet, or pancreatic islet cell include secreting insulin, and the glucose responsiveness is preferably maintained even after transplantation.

The donor of the "insulin-secreting cell", "pancreatic islet", or "pancreatic islet cell" is an animal in a certain

embodiment, a vertebrate in another embodiment, and a mammal in still another embodiment, and specific examples can include a human, a pig, a monkey, a rat, or a mouse, in particular, a human or a pig. In some embodiments, the donor of the "insulin-secreting cell", "pancreatic islet", or "pancreatic islet cell" is a pig from the viewpoint of overcoming donor shortage. The "insulin-secreting cell", "pancreatic islet", or "pancreatic islet cell" may be any of an pancreatic islet or a pancreatic islet cell obtained from a donor animal, and an insulin-secreting cell or pancreatic islet cell obtained from a donor-derived cell, for example, an insulin-secreting cell or pancreatic islet cell differentiated from a human-derived ES cell or iPS cell.

Examples of the "insulin-secreting cell", "pancreatic islet", or "pancreatic islet cell", if derived from a pig, include a pancreatic islet of an adult pig, a pancreatic islet of a pig in the embryonic stage, in the neonatal period, or in the perinatal period, or an insulin-secreting cell or pancreatic islet cell obtained from any of the pancreatic islets. The pancreatic islet may be appropriately cultured before use, and a pancreatic islet obtained by maturing a pancreatic islet of a pig in the embryonic stage, in the neonatal period, or in the perinatal period may be used therefor.

Examples of blood coagulation factor-secreting cells can include factor VIII-secreting cells and factor IX-secreting cells.

Other examples of the cells to be enclosed can include animal cells in a certain embodiment, vertebrate-derived cells in another embodiment, and mammal-derived cells in still another embodiment, in particular, human-derived cells or pig-derived cells. For the type of vertebrate-derived cells (in particular, human-derived cells), any of stem cells (e.g., pluripotent stem cells (versatile cells), or somatic stem cells), progenitor cells, and mature cells are acceptable. As pluripotent stem cells, for example, embryonic stem (ES) cells, germline stem (GS) cells, or induced pluripotent stem (iPS) cells can be used. As somatic stem cells, for example, mesenchymal stem cells (MSCs), hematopoietic stem cells, amniocytes, cord blood cells, bone marrow-derived cells, cardiac stem cells, adipose-derived stem cells, or neural stem cells can be used. Cells obtained by inducing differentiation of any of those stem cells can also be used.

As progenitor cells and mature cells, for example, cells derived from the skin, dermis, epidermis, muscle, myocardium, nerve, bone, cartilage, endothelium, brain, epithelium, heart, kidney, liver, spleen, oral cavity, cornea, bone marrow, cord blood, amnion, or hair can be used. As human-derived cells, for example, ES cells, iPS cells, MSCs, chondrocytes, osteoblasts, osteoprogenitor cells, mesenchymal cells, myoblasts, cardiomyocytes, cardiac myoblasts, nerve cells, hepatocytes, fibroblasts, corneal endothelial cells, vascular endothelial cells, corneal epithelial cells, amniocytes, cord blood cells, bone marrow-derived cells, or hematopoietic stem cells can be used. For the origin of cells, autologous cells and allogeneic cells are both acceptable.

In some embodiments, the biocompatible device may comprise, for example, a substance for maintaining the survivability and functions of the cell or tissue, or a substance that homogenously disperses the cell or tissue that secretes a biologically active substance (such as a hydrogel). The definition of the device of the present invention includes both of embodiments with the substance and embodiments without the substance.

In some embodiments, the cell or tissue is enclosed in the internal space with a hydrogel serving as a carrier. Herein, the term "hydrogel" refers to a polymer having a three-dimensional network structure insoluble in water, and a swollen form thereof with water. Herein, the hydrogel is occasionally written as "the gel", simply.

Examples of the material to form the hydrogel in an embodiment of the present invention include alginic acid (including alginic acid esters and alginic acid salts, as described later), collagen, hyaluronan, gelatin, fibronectin, elastin, tenascin, laminin, vitronectin, polypeptide, heparan sulfate, chondroitin, chondroitin sulfate, keratan, keratan sulfate, dermatan sulfate, carrageenan, heparin, chitin, chitosan, agarose, agar, cellulose, methylcellulose, carboxylmethylcellulose, glycogen, and derivatives of these, and fibrin, fibrinogen, thrombin, polyglutamic acid, polylactic acid, polyglycolic acid, lactic acid-glycolic acid copolymer, gellan gum, xanthan gum, galactomannan, guar gum, locust bean gum, and tara gum. One of these materials may be used singly; alternatively, two or more thereof may be used in combination. Those materials can be hydrogelled thermally, or by heat or light, or chemically (e.g., ionic cross-linking, chemical cross-linking).

In another embodiment of the present invention, the material to be used for forming the hydrogel may be alginic acid or a derivative thereof. In this case, the alginic acid or derivative thereof has been preferably modified to be able to gel through chemical cross-linking before use. Specific examples of the chemically modified alginic acid derivative include those shown in the embodiments of [3-5] to [3-9] and [1A-9-2] to [1A-11]. Examples of the alginic acid having chemical cross-linkages formed therein (chemically cross-linked alginic acid) include those shown in the embodiments of [3-10] to [3-13] and [3A] to [3A-5]. With inclusion of alginic acid, or chemically cross-linked alginic acid in a certain embodiment, as the hydrogel, the network structure of the alginic acid or chemically cross-linked alginic acid firmly associates with the above-described unevenness of the non-woven fabric surface, successfully enhancing the anchoring effect of the non-woven fabric.

The hydrogel in an embodiment of the present invention is substantially free of a vinyl alcohol polymer. In an embodiment of the present invention, the vinyl alcohol polymer content of the hydrogel is less than approximately 0.1% by mass, less than approximately 0.05% by mass in another embodiment, and less than approximately 0.01% by mass in still another embodiment, on the basis of the total amount of the constituent components of the hydrogel.

The following describes alginic acid and alginic acid derivatives and the like that serve as raw materials of chemically cross-linked alginic acid to constitute the hydrogel in the device in an embodiment.

2. Alginic acid

Herein, the statement of alginic acid means at least one alginic acid selected from the group consisting of alginic acid, alginic acid esters, and salts thereof (e.g., sodium alginate) (occasionally referred to as an "alginic acid compound"). The alginic acid for use may be a naturally derived or synthesized alginic acid, but is preferably a naturally derived alginic acid.

Herein, the alginic acid is occasionally represented as (ALG)-COOH, wherein (ALG) represents the main part of the alginic acid, and -COOH represents any one of the carboxyl groups of the alginic acid.

In some embodiments, the alginic acid is a sodium alginate. For the sodium alginate, commercially available sodium alginates can be used. Sodium alginates used in Examples shown later are those of A-1, A-2, A-3, B-1, B-2, and B-3 (provider: Mochida Pharmaceutical Co., Ltd.) listed in a table shown below. The following table shows the viscosity of 1 w/w% aqueous solution, weight-average molecular weight, and M/G ratio for the sodium alginates.

[Table 15]

| Sodium alginate | Viscosity at 1w/w% (mPa·s) | Weight-average molecular weight | | M/G ratio |
|---|---|---|---|---|
| | | GPC | GPC-MALS | |
| A-1 | 10 to 40 | 300,000 to 700,000 | 60,000 to 130,000 | 0.5 to 1.8 |
| A-2 | 50 to 150 | 700,000 to 1,400,000 | 130,000 to 200,000 | |
| A-3 | 300 to 600 | 1,400,000 to 2,000,000 | 200,000 to 400,000 | |
| B-1 | 10 to 40 | 150,000 to 800,000 | 60,000 to 130,000 | 0.1 to 0.5 |
| B-2 | 70 to 150 | 800,000 to 1,500,000 | 130,000 to 200,000 | |
| B-3 | 400 to 600 | 1,500,000 to 2,500,000 | 200,000 to 350,000 | |

Herein, the "alginic acid esters" and "alginic acid salts" are not limited, but need to be free of a functional group that inhibits cross-linking reaction. Preferable examples of the alginic acid esters include propylene glycol alginate.

Examples of the alginic acid salts herein include monovalent salts of alginic acid and divalent salts of alginic acid. Preferable examples of the monovalent salts of alginic acid include sodium alginate, potassium alginate, and ammonium alginate; more preferable is sodium alginate or potassium alginate; particularly preferable is sodium alginate. Preferable examples of the divalent salts of alginic acid include calcium alginate, magnesium alginate, barium alginate, and strontium alginate.

For other explanation on alginic acid, reference can be made to descriptions in PCT/JP2019/023478 (filed on June 13, 2019), PCT/JP2020/047100 (filed on December 17, 2020), and PCT/JP2019/007655 (filed on February 27, 2019).

3. Alginic acid derivatives

In some embodiments, alginic acid derivatives in the present specification are each a product obtained by introducing a reactive group or a complementary reactive group therewith in Huisgen reaction to each of any one or more carboxyl groups of alginic acid via an amide bond and a divalent linker.

More specifically, the alginic acid derivatives are the alginic acid derivatives described in [1A-9-2], which are an alginic acid derivative represented by the following formula (I-A):

[Chem. 30]

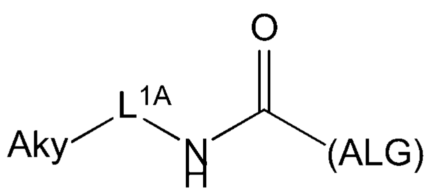

(I-A)

wherein (ALG), -NHCO-, -$L^{1A}$-, and Aky are as defined in [1A-9-2], and an alginic acid derivative represented by the following formula (II-A):

[Chem. 31]

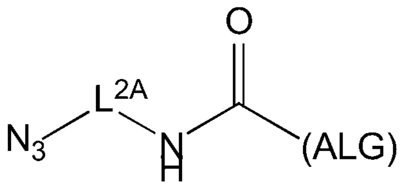

(II-A)

wherein (ALG), -NHCO-, and $-L^{2A}-$ are as defined in [1A-9-2], or otherwise, an alginic acid derivative represented by the following formula (H-I):

[Chem. 32]

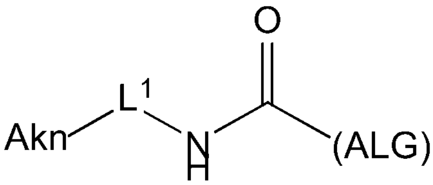

(H-I)

wherein (ALG) represents alginic acid; -NHCO- represents an amide bond via any carboxyl group of alginic acid; and $-L^1-$ is a divalent linker bonding to a cyclic alkyne group (Akn), and an alginic acid derivative represented by the following formula (H-II):

[Chem. 33]

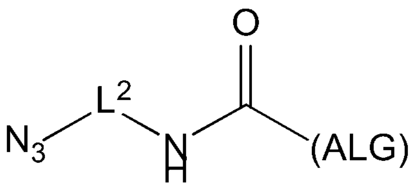

(H-II)

wherein (ALG) represents alginic acid; -NHCO- represents an amide bond via any carboxyl group of alginic acid; and $-L^2-$ is a divalent linker bonding to an azide group.

For the divalent linker ($-L^1-$ or $-L^2-$), any linear group can be used as long as the linear group does not inhibit the reaction between the reactive group and the complementary reactive group therewith. Specific examples include, but are not limited to, a linear alkylene group ($-(CH_2)_n-$, n = 1 to 30) (several (e.g., 1 to 10, or 1 to 5) of the $-CH_2-$ groups in the group are each optionally replaced with such a group as -C(=O)-, -CONH-, -O-, -NH-, -S-, a benzene ring, a heterocycle (a five- or six-membered aromatic heterocycle or five- or six-membered nonaromatic heterocycle such as a pyridine ring, a piperidine ring, and a piperazine ring), and several (e.g., 1 to 10, or 1 to 5) of the hydrogen atoms of the $-CH_2-$ groups are each optionally replaced with a group selected from groups including an oxo group (=O), a $C_{1-6}$ alkyl group (e.g., such a group as a methyl group, an ethyl group, a n-propyl group, or an isopropyl group), a halogen atom (e.g., a fluorine atom, a chlorine atom, a bromine atom, an iodine atom), and a hydroxy group (-OH)).

The hydrogen atom of the imino group (-NH-) in the -NH-CO- group of the chemically modified alginic acid derivative represented by the formula (I-A), formula (II-A), formula (H-I), or formula (H-II) can be replaced with a methyl group to form an -N(Me)-CO- group.

The bonding mode of the linker ($-L^1-$, $-L^2-$) and alginic acid in the chemically modified alginic acid derivative represented by the formula (H-I) or formula (H-II), or the bonding mode of the linker ($-L^{1A}-$, $-L^{2A}-$) and alginic acid in the chemically modified alginic acid derivative represented by the formula (I-A) or formula (II-A) is an -NH-CO- bond or an -N(Me)-CO- bond, and preferably an -NH-CO- bond. The -CO- in the -NH-CO- bond or -N(Me)-CO- bond is derived from a carboxyl group of alginic acid.

Herein, the term "cyclic alkyne group" means a "five- to nine-membered cycloalkyne group", unless otherwise stated, and the definition includes a cycloalkyne group obtained by replacing one to four $-CH_2-$ groups in a "five- to nine-membered cycloalkyne group" each with a group selected from the group consisting of -NH-, -S-, -O-, and =C(=O). In the cyclic alkyne group, one to five of the hydrogen atoms of the $-CH_2-$ groups on the ring are each optionally replaced with a

group selected from groups including a halogen atom, a hydroxy group, an amino group, a keto group, a $C_{1-3}$ alkyl group, an -O-$C_{1-3}$ alkyl group, -NH($C_{1-3}$ alkyl group), -N($C_{1-3}$ alkyl group)$_2$, and -COO-M (M = Na, K, 1/2 Ca, a hydrogen atom, or a $C_{1-3}$ alkyl group); and the cyclic alkyne group is optionally fused with one to three rings selected from a $C_{3-8}$ cycloalkyl ring, a benzene ring, and a five- or six-membered aromatic heterocycle.

Herein, the term "five- to nine-membered cycloalkyne group" means a group obtained by replacing -$CH_2$-$CH_2$- in a monocyclic saturated cycloalkyl group having five to nine carbon atoms with -C=C-, unless otherwise stated, and examples thereof include groups including a cyclopentyne group, a cyclohexyne group, a cycloheptyne group, a cyclooctyne group, and a cyclononyne group. One to four of the -$CH_2$-groups of a "five- to nine-membered cycloalkyne group" can be each replaced with a group selected from the group consisting of -NH-, -S-, -O-, and =C(=O).

Herein, the cyclic alkyne group is, unless otherwise stated, preferably a seven-to nine-membered cyclic alkyne group (including a cycloalkyne group obtained by replacing one to four of the -$CH_2$- groups of a seven- to nine-membered cycloalkyne group each with a group selected from the group consisting of -NH-, -S-, -O-, and =C(=O); one to five of the hydrogen atoms of the -$CH_2$- groups on the ring of the seven- to nine-membered cyclic alkyne group are each optionally replaced with a group selected from groups including a halogen atom, a hydroxy group, an amino group, a keto group, a $C_{1-3}$ alkyl group, an -O-$C_{1-3}$ alkyl group, -NH($C_{1-3}$ alkyl group), -N($C_{1-3}$ alkyl group)$_2$, and -COO-M (M = Na, K, 1/2 Ca, a hydrogen atom, or a $C_{1-3}$ alkyl group); and the seven- to nine-membered cyclic alkyne group is optionally fused with one to three rings selected from a $C_{3-8}$ cycloalkyl ring, a benzene ring, and a five- or six-membered aromatic heterocycle;

more preferably an eight-membered cyclic alkyne group (a cyclooctyne group) (including a cycloalkyne group obtained by replacing one to four of the -$CH_2$- groups of a cyclooctyne group each with a group selected from the group consisting of - NH-, -S-, -O-, and =C(=O); one to five of the hydrogen atoms of the -$CH_2$- groups on the ring of the cyclooctyne group are each optionally replaced with a group selected from groups including a halogen atom, a hydroxy group, an amino group, a keto group, a $C_{1-3}$ alkyl group, an -O-$C_{1-3}$ alkyl group, -NH($C_{1-3}$ alkyl group), -N($C_{1-3}$ alkyl group)$_2$, and -COO-M (M = Na, K, 1/2 Ca, a hydrogen atom, or a $C_{1-3}$ alkyl group); and the cyclooctyne group is optionally fused with one to three rings selected from a $C_{3-8}$ cycloalkyl ring, a benzene ring, and a five- or six-membered aromatic heterocycle);

further preferably a group selected from the following partial structural formulas:

[Chem. 34]

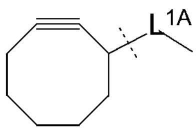
(Aky-1)

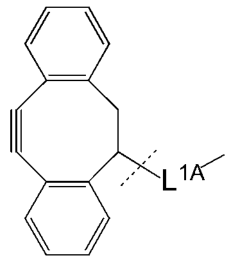
(Aky-2)

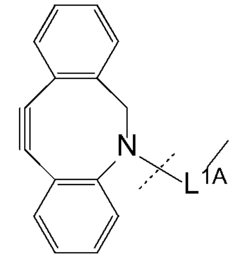
(Aky-3)

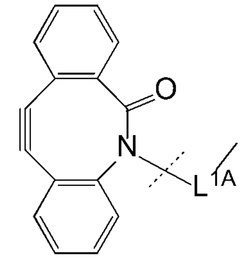
(Aky-4)

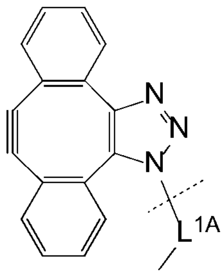
(Aky-5)

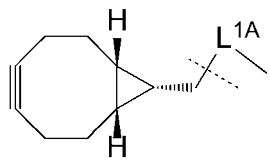
(Aky-6)

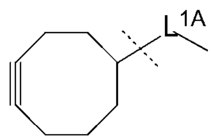
(Aky-7)

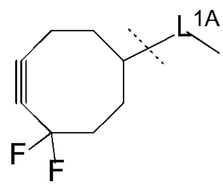
(Aky-8)

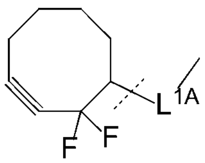
(Aky-9)

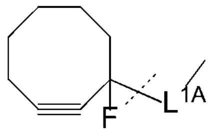
(Aky-10)

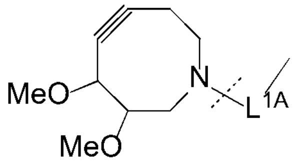
(Aky-11)

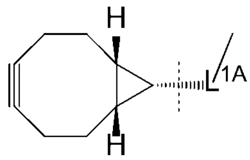
(Aky-12)

[in each of the formulas, the right sides of the dashed lines at the two ends are not included]; particularly preferably a group selected from the following partial structural formulas:

[Chem. 35]

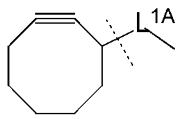
(Aky-1)

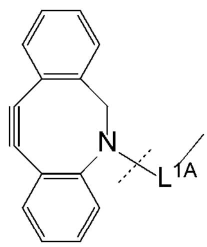
(Aky-3)

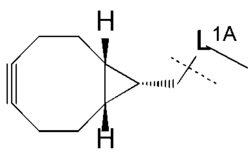
(Aky-6)

[in each of the formulas, the right sides of the dashed lines at the two ends are not included]; and most preferably a group selected from the following partial structural formulas:

[Chem. 36]

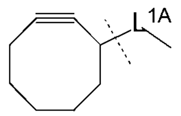

(Aky-1)

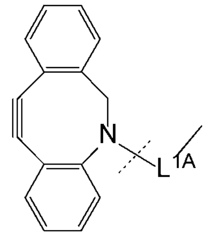

(Aky-3)

[in each of the formulas, the right sides of the dashed lines at the two ends are not included].

The alginic acid derivatives in a certain embodiment are such a compound that, in the formula (I-A) or formula (H-I), $-L^{1A}$- or $-L^{1}$- is $-(CH_2)_{n1}-$ (wherein n1 = 1 to 50 is satisfied, $-CH_2-$ groups in the group are optionally replaced with 1 to 10 groups selected from -CO-, -CONH-, -NHCO-, -O-CONH-, -NHCO-O-, -O-, and - NH- or with a benzene ring, and the hydrogen atoms of the $-CH_2-$ groups are each optionally replaced with a $C_{1-3}$ alkyl group or a $C_{1-3}$ alkyl group substituted with a phenyl group), Aky or Akn is an eight-membered cyclic alkyne group (wherein the cyclic alkyne group is optionally further fused with one or two rings each being a benzene ring, a cyclopropane ring, or a 1,2,3-triazole ring and is optionally bound to $-L^1-$ in the fused ring, $-CH_2-$ groups in the group are optionally replaced with one or two groups selected from -C(=O)-, -CONH-, and -NH-, and hydrogen atoms of the - $CH_2$- groups are optionally replaced with one or two groups selected from groups including a $C_{1-3}$ alkyl group, a fluorine atom, a hydroxy group, and a $C_{1-3}$ alkyloxy group), and such a compound that, in the formula (II-A) or formula (H-II), $-L^{2A}$- or - $L^2$- is $-(CH_2)_{n2}-$ (wherein n2 = 1 to 50 is satisfied, $-CH_2-$ groups in the group are optionally replaced with 1 to 10 groups selected from -CONH-, -NHCO-, -O-, and - NH- or with a benzene ring or a pyridine ring, and the hydrogen atoms of the $-CH_2-$groups are each optionally replaced with a $C_{1-3}$ alkyl group).

Here, n1 in the compound is 1 to 20 in a preferable embodiment, and 1 to 15 (e.g., 2 to 15) in a more preferable embodiment. In a preferable embodiment, $-L^{1A}$- or $-L^{1}$- is such a group that $-CH_2-$ groups in $-(CH_2)_{n1}-$ are optionally replaced with one to five groups, more preferably with one to three groups, selected from -CO-, - CONH-, -NHCO-, -O-CONH-, -NHCO-O-, -O-, and -NH-. Preferably, Aky or Akn is optionally fused with one or two benzene rings, and is such an eight-membered cyclic alkyne group that the $-CH_2-$ groups in the eight-membered cyclic alkyne group are each optionally replaced with -NH-. n2 is 1 to 20 in a preferable embodiment, and 1 to 15 (e.g., 2 to 15) in a preferable embodiment. In a preferable embodiment, $-L^{2A}$- or $-L^{2}$- is such a group that $-CH_2-$ groups in $-(CH_2)_{n2}-$ are optionally replaced with one to five groups, more preferably with one to three groups, selected from - CONH-, -NHCO-, -O-, and -NH-.

In a preferable embodiment, the alginic acid derivatives are such a compound that, $-L^{1A}$- in the formula (I-A) or $-L^{1}$- in the formula (H-I) is $-(CH_2)_{n1}-$ (wherein n1 = 1 to 15 (e.g., 2 to 15) is satisfied, $-CH_2-$ groups in the group are optionally replaced with one to five groups selected from -CO-, -CONH-, -NHCO-, -O-CONH-, - NHCO-O-, -O-, and -NH- or with a benzene ring, and the hydrogen atoms of the - $CH_2$- groups are each optionally replaced with a $C_{1-3}$ alkyl group or a $C_{1-3}$ alkyl group substituted with a phenyl group), Aky or Akn is an eight-membered cyclic alkyne group (wherein the cyclic alkyne group is optionally further fused with one or two benzene rings, and is optionally such an eight-membered ring group that any - $CH_2$- group in the eight-membered cyclic alkyne group is replaced with -NH-), and such a compound that $-L^{2A}$- in the formula (II-A) or $-L^{2}$- in the formula (H-II) is - $(CH_2)_{n2}$- (wherein n2 = 1 to 15 (e.g., 2 to 15) is satisfied, $-CH_2-$ groups in the group are optionally replaced with one to five groups selected from -CONH-, -NHCO-, -O-, and -NH- or with a benzene ring).

Examples of "halogen atom" herein include a fluorine atom, a chlorine atom, a bromine atom, and an iodine atom, unless otherwise stated.

Examples of "$C_{1-3}$ alkyl group" herein include groups of methyl, ethyl, propyl, and isopropyl, unless otherwise stated.

The term "$C_{2-4}$ alkanoyl group" herein means "$C_{1-3}$ alkylcarbonyl group", in which a carbonyl group is bound to the "$C_{1-3}$ alkyl group", unless otherwise stated, and examples thereof include groups of acetyl, propionyl, and butyryl.

Examples of "$C_{3-8}$ cycloalkyl ring" herein include monocyclic or polycyclic saturated or unsaturated cycloalkyl rings having three to eight carbon atoms, such as rings of cyclopropane, cyclobutane, cyclopentane, cyclohexane, cycloheptane, and cyclooctane, unless otherwise stated.

Examples of "$C_{5-9}$ cycloalkene ring" herein include monocyclic cycloalkene rings having five to nine carbon atoms, such as rings of cyclopentene, cyclohexene, cycloheptene, cyclooctene, and cyclononene, unless otherwise stated.

Herein, the term "five- or six-membered aromatic heterocycle" means a five- or six-membered unsaturated ring

having one to four heteroatoms selected from the group consisting of a nitrogen atom, a sulfur atom, and an oxygen atom, unless otherwise stated.

Examples of the "five- or six-membered aromatic heterocycle" herein include groups of pyrrole, furan, thiophene, imidazole, pyrazole, oxazole, isoxazole, thiazole, isothiazole, triazole, oxadiazole, furazan, thiadiazole, tetrazole, pyridine, pyridazine, pyrimidine, pyrazine, triazine, and thiadiazine, unless otherwise stated.

Herein, the term "five- or six-membered nonaromatic heterocycle" means a five- or six-membered saturated heterocycle containing one to four heteroatoms selected from an oxygen atom, a sulfur atom, and a nitrogen atom, unless otherwise stated.

Examples of the "five- or six-membered nonaromatic heterocycle" herein include rings of pyrrolidine, tetrahydrofuran, thiolane, piperidine, dihydropyran, tetrahydropyran, tetrahydrothiopyran, piperazine, dioxane, morpholine, thiomorpholine, and quinuclidine, unless otherwise stated.

Herein, each alginic acid derivative represented by the formula (I-A), formula (II-A), formula (H-I), or formula (H-II) can be produced, for example, by an alginic acid derivative synthesis method described later.

The alginic acid derivative represented by the formula (H-I) in a certain embodiment is such a compound that, in the formula, the definitions of $-L^1-$ and Akn are the same as those in the above-described embodiment of [3-8].

The alginic acid derivative represented by the formula (H-II) in a certain embodiment is such a compound that, in the formula, the definition of $-L^2-$ is the same as that in the above-described embodiment of [3-8].

The alginic acid derivative represented by the formula (I-A) in a certain embodiment is such a compound that, in the formula, the definitions of $-L^{1A}-$ and Aky are the same as those in the above-described embodiment of [1A-9-2]. The alginic acid derivative represented by the formula (II-A) in a certain embodiment is such a compound that, in the formula, the definition of $-L^{2A}-$ is the same as that in the above-described embodiment of [1A-9-2].

Specific examples of the alginic acid derivative represented by the formula (I-A) or formula (H-I) herein include, but are not limited to, alginic acid derivatives represented by the following formulas:

[Chem. 37]

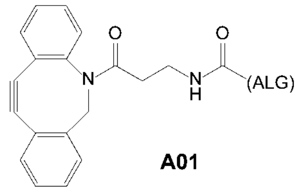
A01

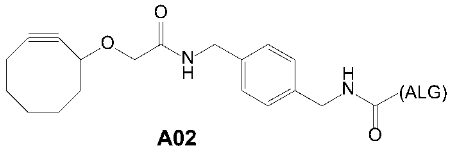
A02

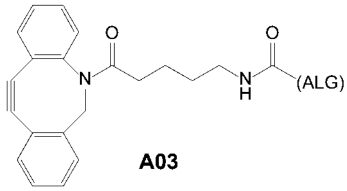
A03

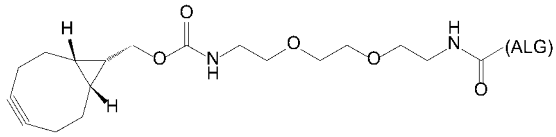
A04

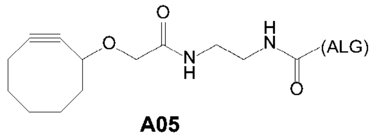
A05

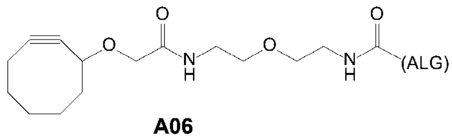
A06

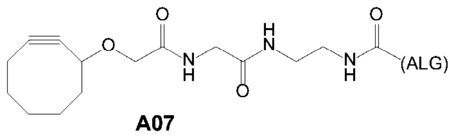
A07

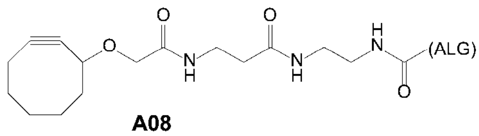
A08

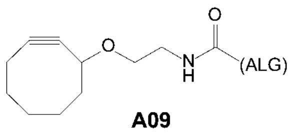
A09

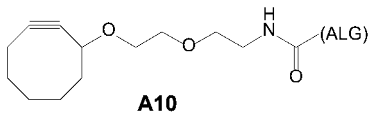
A10

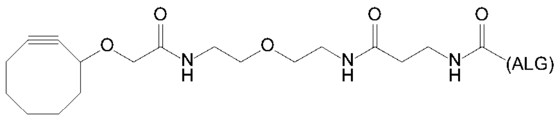
A11

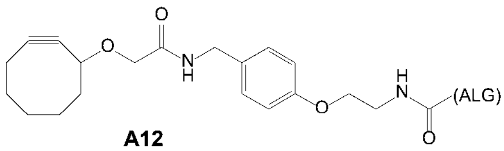
A12

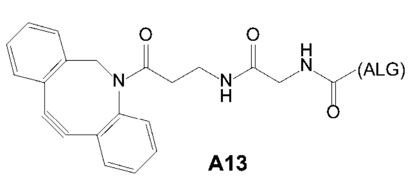
A13

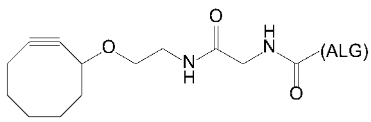
A14

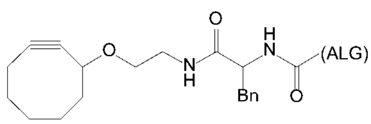
A15

Specific examples of the alginic acid derivative represented by the formula (II-A) or formula (H-II) herein include, but are not limited to, alginic acid derivatives represented by the following formulas:

[Chem. 38]

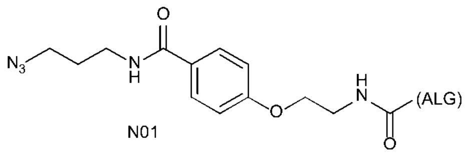

N01

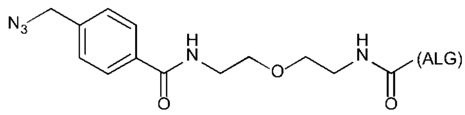

N02

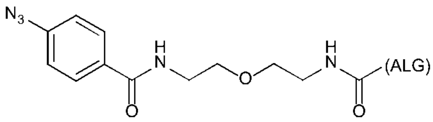

N03

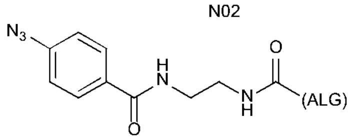

N04

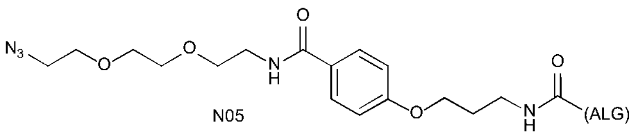

N05

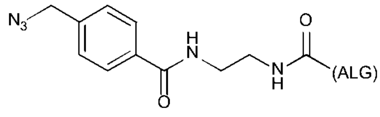

N06

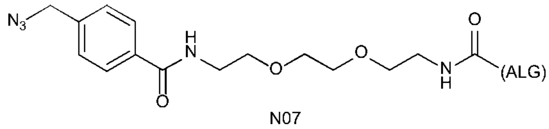

N07

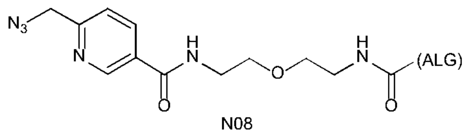

N08

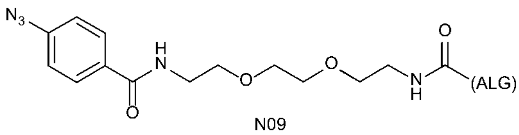

N09

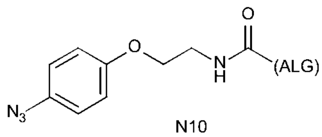

N10

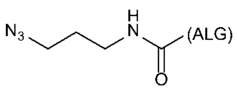

N11

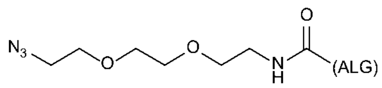

N12

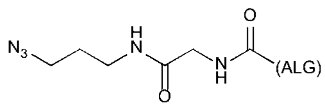

N13

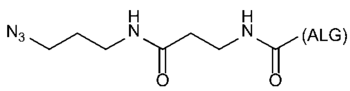

N14

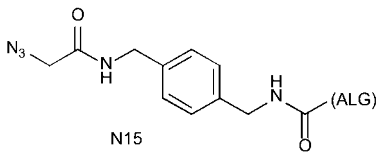

N15

Herein, if a cyclic group is substituted with a variable substituent, it means that the variable substituent is not bound to a particular carbon atom of the cyclic group, unless otherwise stated. For example, this means that, in the following formula A, substitution with the variable substituent Rs is acceptable at any of carbon atoms i, ii, iii, iv, and v in the formula A.

[Chem. 39]

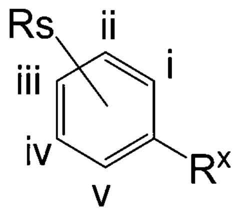

Formula A

The weight-average molecular weights of the alginic acid derivative represented by the formula (I-A) or formula (II-A) and the alginic acid derivative represented by the formula (H-I) or formula (H-II) in the present specification are each

approximately 100000 Da to approximately 3000000 Da, preferably each approximately 300000 Da to approximately 2500000 Da, and more preferably approximately 500000 Da to approximately 2000000 Da. The molecular weights of the alginic acid derivatives can be determined by methods described in PCT/JP2019/023478 (filed on June 13, 2019) and PCT/JP2020/047100 (filed on December 17, 2020).

Herein, the Aky-$L^{1A}$ -NH- group or Akn-$L^{1}$-NH- group in the formula (I-A) or formula (H-I) does not need to be bound to every carboxyl group in the constituent units of alginic acid, and the $N_3$-$L^{2A}$-NH- group or $N_3$-$L^{2}$-NH- group in the formula (II-A) or formula (H-II) does not need to be bound to every carboxyl group in the constituent units of alginic acid.

Herein, when the Aky-$L^{1A}$-NH- group or Akn-$L^{1}$-NH- group in the formula (I-A) or formula (H-I) is referred to as a reactive group, the $N_3$-$L^{2A}$-NH- group or $N_3$-$L^{2}$-NH- group in the formula (II-A) or formula (H-II) corresponds to the complementary reactive group. Conversely, when the $N_3$-$L^{2A}$-NH- group or $N_3$-$L^{2}$-NH- group in the formula (II-A) or formula (H-II) is referred to as a reactive group, the Aky-$L^{1A}$-NH- group or Akn-$L^{1}$-NH- group in the formula (I-A) or formula (H-I) corresponds to the complementary reactive group.

Herein, the introduction rate of the reactive group or complementary reactive group is approximately 0.1% to approximately 30% or approximately 1% to approximately 30%, preferably approximately 2% to approximately 20%, and more preferably approximately 3% to approximately 10% for each case.

An introduction rate of the reactive group or complementary reactive group is a value in percentage of the number of uronic acid monosaccharide units having the corresponding reactive group introduced therein out of the uronic acid monosaccharide units as the repeating units of an alginic acid compound. Herein, % used for introduction rates of the reactive group or complementary reactive group in any alginic acid derivative (formula (I-A), formula (II-A), formula (H-I), or formula (H-II)) means mol%, unless otherwise stated. The introduction rate of the reactive group or complementary reactive group can be determined by methods described in PCT/JP2019/023478 (filed on June 13, 2019) and PCT/JP2020/047100 (filed on December 17, 2020) for each case.

Herein, the cyclic alkyne group (Akn) in the formula (H-I) and the azide group in the formula (H-II) form a triazole ring through Huisgen reaction, thereby forming a cross-linkage. The cyclic alkyne group (Aky) in the formula (I-A) and the azide group in the formula (II-A) form a triazole ring through Huisgen reaction, thereby forming a cross-linkage. For the Huisgen reaction, reference can be made to descriptions in PCT/JP2019/023478 (filed on June 13, 2019) and PCT/JP2020/047100 (filed on December 17, 2020).

4. Alginic acid derivative synthesis method

The alginic acid derivatives represented by the formula (I-A), formula (II-A), formula (H-I), and formula (H-II) can be produced by a production method shown in the following (see the above description for the definitions of Aky, -$L^{1A}$-, -$L^{2A}$-, (ALG), and so on; Aky, -$L^{1A}$-, and -$L^{2A}$- can be replaced with Akn, -$L^{1}$-, and -$L^{2}$-, respectively).

[Chem. 40]

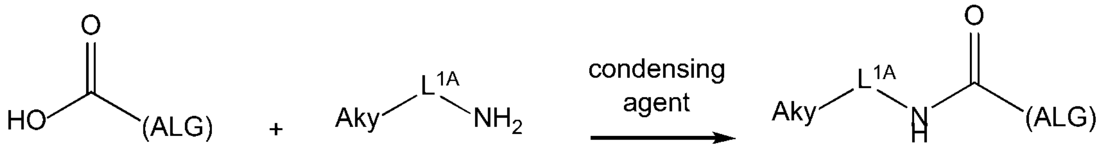


Alginic acid (AM-1A) (I-A)

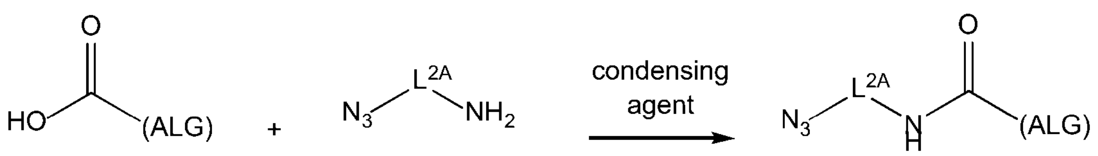


Alginic acid (AM-2A) (II-A)

For more detailed reaction conditions, reference can be made to General Production Methods and Examples in PCT/JP2019/023478 (filed on June 13, 2019) and PCT/JP2020/047100 (filed on December 17, 2020), and the alginic acid derivatives described herein can be produced with reference to them.

5. Cross-linked alginic acid

Cross-linked alginic acids include (i) those with ionic bonds of divalent metal ions, (ii) those with chemical bonds,

and (iii) those with both ionic bonds of divalent metal ions and chemical bonds. All of the cross-linked alginic acids have properties to become a gel-like to semisolid form, and even become a sponge-like form for some cases. Examples of the divalent metal ions include, but are not limited to, calcium ions, magnesium ions, barium ions, strontium ions, and zinc ions, and the divalent metal ions are calcium ions or barium ions in a certain embodiment.

In a cross-linked alginic acid with ionic bonds of divalent metal ions, reaction proceeds at an ultra-high speed and the reaction is reversible; in a cross-linked alginic acid with chemical bonds, on the other hand, reaction slowly proceeds under relatively mild conditions and the reaction is irreversible. The physical properties of a cross-linked alginic acid can be adjusted, for example, by such a method as changing the concentration of an aqueous solution containing divalent metal ions to be used (e.g., aqueous solution of calcium chloride, aqueous solution of barium chloride) or the introduction rate of the reactive group to be introduced into the alginic acid.

The divalent metal ion concentration (e.g., calcium ion or barium ion concentration) of the aqueous solution containing divalent metal ions is not limited, and is, for example, in the range of approximately 1 mmol/L to approximately 1 mol/L in a certain embodiment, in the range of approximately 2 mmol/L to approximately 500 mmol/L in another embodiment, and in the range of approximately 5 mmol/L to approximately 100 mmol/L in still another embodiment. Herein, an aqueous solution containing divalent metal ions is occasionally referred to as a divalent metal ion solution, simply, and the divalent metal ion solution is an aqueous solution, unless otherwise stated.

Use of the cross-linking reaction enables production of various alginic acid structures (e.g., a hydrogel). For example, a specific structure can be instantaneously made from an alginic acid solution through ionic cross-linking reaction, and cross-linking reaction by chemical bonding can be used for structural reinforcement of the structure (e.g., obtaining long-term stability). From a cross-linked alginic acid structure with both ionic bonds of divalent metal ions and chemical bonds, for example, even a structure in which only cross-linkages formed through chemical bonding are remained can be made by reversible release of divalent metal ions incorporated through ionic cross-linking. In a preferable embodiment, the cross-linked alginic acid comprises a chemically cross-linked alginic acid obtained by using cross-linking reaction by chemical bonding. In another preferable embodiment, the cross-linked alginic acid comprises the chemically cross-linked alginic acid and ionic cross-linked alginic acid with ionic bonds of divalent metal ions. In still another preferable embodiment, the cross-linked alginic acid comprises the chemically cross-linked alginic acid and optionally the ionic cross-linked alginic acid.

In a certain embodiment, the cross-linked alginic acid can be obtained by mixing the alginic acid derivatives of the formula (H-I) and formula (H-II) and subjecting the mixture to the Huisgen reaction. Alternatively, the cross-linked alginic acid can be obtained by mixing the alginic acid derivatives of the formula (I-A) and formula (II-A) and subjecting the mixture to the Huisgen reaction. In a certain embodiment, the cross-linked alginic acid forms a three-dimensional network structure via chemical cross-linking (cross-linking by a triazole ring formed from an alkyne group and an azide group).

In some embodiments, the cross-linked alginic acid is a cross-linked alginic acid formed through amide bonding between any carboxyl group of a first alginic acid and any carboxyl group of a second alginic acid via the following formula (H-III-L):

[Chem. 41]

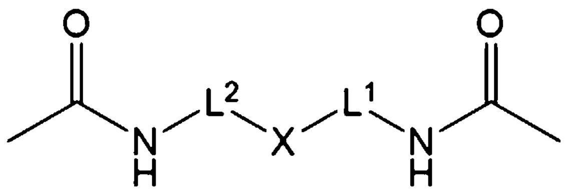

(H-III-L)

wherein -CONH- and -NHCO- at the two ends each represent an amide bond via any carboxyl group of alginic acid; and $-L^1-$, $-L^2-$, and X are as defined in any one of the embodiments of [3-10] to [3-13].

In some embodiments, the mixing ratio between the alginic acid derivative of the formula (H-I) and the alginic acid derivative of the formula (H-II) in preparing the cross-linked alginic acid is, for example, 1 to 1.5:1, preferably 1.2 to 1.5:1 or 1 to 1.2:1, and more preferably 1:1, in the weight ratio between the derivative of the formula (H-I) and the derivative of the formula (H-II).

In some embodiments, the mixing ratio between the alginic acid derivative of the formula (H-II) and the alginic acid derivative of the formula (H-I) in preparing the cross-linked alginic acid is, for example, 1 to 4.0:1, preferably 1.5 to 4.0:1 or 1.2 to 1.5:1 or 1 to 1.2:1, and more preferably 1:1, in the weight ratio between the derivative of the formula (H-II) and the derivative of the formula (H-I).

In some embodiments, more preferably, the mixing ratio between the alginic acid derivative of the formula (H-I) and the alginic acid derivative of the formula (H-II) in preparing the cross-linked alginic acid is, for example, 1 to 1.5:1,

preferably 1.2 to 1.5:1 or 1 to 1.2:1, and more preferably 1:1, in the ratio between the introduction rate (mol%) of the reactive group in the alginic acid derivative of the formula (H-I) and that in the alginic acid derivative of the formula (H-II).

In some embodiments, more preferably, the mixing ratio between the alginic acid derivative of the formula (H-II) and the alginic acid derivative of the formula (H-I) in preparing the cross-linked alginic acid is, for example, 1 to 4.0:1, preferably 1.5 to 4.0:1 or 1.2 to 1.5:1 or 1 to 1.2:1, and more preferably 1:1, in the ratio between the introduction rate (mol%) of the reactive group in the alginic acid derivative of the formula (H-II) and that in the alginic acid derivative of the formula (H-I).

For the mixing ratios, the alginic acid derivative of the formula (H-I) can be exchanged with the alginic acid derivative of the formula (H-II), and the alginic acid derivative of the formula (H-II) can be exchanged with the alginic acid derivative of the formula (H-I).

The cross-linked alginic acid does not need to have the cross-linkage of the formula (H-III-L) at every carboxyl group of the constituent units of the alginic acid. The introduction rate of the cross-linkage represented by the formula (H-III-L) (also referred to as the cross-linking rate) in the cross-linked alginic acid is, for example, in the range of approximately 0.1 to approximately 80%, approximately 0.3 to approximately 60%, approximately 0.5 to approximately 30%, or approximately 1.0 to approximately 10%.

The concentration of each alginic acid derivative of the formula (H-I) or formula (H-II) in the Huisgen reaction to obtain the cross-linked alginic acid is normally approximately 1 to approximately 500 mg/mL, and preferably in the range of approximately 5 to approximately 100 mg/mL.

In some embodiments, the cross-linked alginic acid is a cross-linked alginic acid formed through amide bonding between any carboxyl group of a first alginic acid and any carboxyl group of a second alginic acid via the following formula (III-Lx):

[Chem. 42]

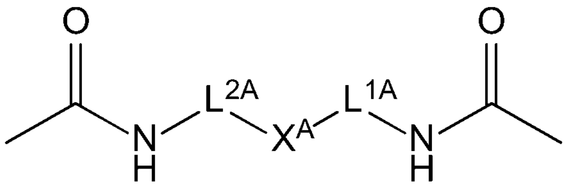

(III-Lx)

wherein -NHCO- at the right end is an amide bond derived from the alginic acid derivative of the formula (I-A), and -CONH- at the left end represents an amide bond derived from the alginic acid derivative of the formula (II-A);

- $L^{1A}$- and -$L^{2A}$- are as defined in [1A-9-2]; and
- $X^{A}$- are as defined in [3A].

In some embodiments, the mixing ratio between the alginic acid derivative of the formula (I-A) and the alginic acid derivative of the formula (II-A) in preparing the cross-linked alginic acid is, for example, 1 to 1.5:1, preferably 1.2 to 1.5:1 or 1 to 1.2:1, and more preferably 1:1, in the weight ratio between the derivative of the formula (I-A) and the derivative of the formula (II-A).

In some embodiments, the mixing ratio between the alginic acid derivative of the formula (II-A) and the alginic acid derivative of the formula (I-A) in preparing the cross-linked alginic acid is, for example, 1 to 4.0:1, preferably 1.5 to 4.0:1 or 1.2 to 1.5:1 or 1 to 1.2:1, and more preferably 1:1, in the weight ratio between the derivative of the formula (II-A) and the derivative of the formula (I-A).

In some embodiments, more preferably, the mixing ratio between the alginic acid derivative of the formula (I-A) and the alginic acid derivative of the formula (II-A) in preparing the cross-linked alginic acid is, for example, 1 to 1.5:1, preferably 1.2 to 1.5:1 or 1 to 1.2:1, and more preferably 1:1, in the ratio between the introduction rate (mol%) of the reactive group in the alginic acid derivative of the formula (I-A) and that of the alginic acid derivative of the formula (II-A).

In some embodiments, more preferably, the mixing ratio between the alginic acid derivative of the formula (II-A) and the alginic acid derivative of the formula (I-A) in preparing the cross-linked alginic acid is, for example, 1 to 4.0:1, preferably 1.5 to 4.0:1 or 1.2 to 1.5:1 or 1 to 1.2:1, and more preferably 1:1, in the ratio between the introduction rate (mol%) of the reactive group in the alginic acid derivative of the formula (II-A) and that in the alginic acid derivative of the formula (I-A).

For the mixing ratios, the alginic acid derivative of the formula (I-A) can be exchanged with the alginic acid derivative of the formula (II-A), and the alginic acid derivative of the formula (II-A) can be exchanged with the alginic acid derivative of the formula (I-A).

The cross-linked alginic acid does not need to have the cross-linkage of the formula (III-Lx) at every carboxyl groups of the constituent units of the alginic acid. The introduction rate of the cross-linkage represented by the formula (III-Lx) (also referred to as the cross-linking rate) in the cross-linked alginic acid is, for example, in the range of approximately 0.1 to approximately 80%, approximately 0.3 to approximately 60%, approximately 0.5 to approximately 30%, or approximately 1.0 to approximately 10%.
The concentration of each alginic acid derivative of the formula (I-A) or formula (II-A) in the Huisgen reaction to obtain the cross-linked alginic acid is normally approximately 1 to approximately 500 mg/mL, and preferably in the range of approximately 5 to approximately 100 mg/mL.
The reaction temperature in the Huisgen reaction is normally an external temperature of approximately 4 to approximately 60°C, and preferably an external temperature in the range of approximately 15 to approximately 40°C.
The stirring time for forming the cross-linked alginic acid is, for example, several seconds to approximately 24 hours, several seconds to approximately 12 hours, several seconds to approximately 30 minutes, or several seconds to approximately 10 minutes.
Examples of the reaction solvent or reaction solution to be used for the Huisgen reaction include, but are not limited to, tap water, pure water (e.g., distilled water, ion-exchanged water, RO water, RO-EDI water), ultrapure water, cell culture medium, phosphate-buffered saline (PBS), and physiological saline, and ultrapure water is preferable.
In some embodiments, the cross-linked alginic acid is a cross-linked alginic acid containing, as cross-linkages, chemical cross-linkages each comprising a triazole ring formed through the Huisgen reaction and ionic cross-linkages partially formed by calcium ions or barium ions.

6. Method of producing biocompatible device

The method of producing a biocompatible device will be described in more detail. The production method of the present invention for a biocompatible device in an embodiment may be, for example, a production method shown in "6-1" or "6-2" in the following.

6-1. Method of producing biocompatible device (1)

The production method in an embodiment includes the following steps (a) to (d).

Step (a): a step of producing a semi-permeable membrane layer formed by a cellulose derivative with a non-woven fabric serving as a support to form a biocompatible composite membrane having a structure with partial intrusion of the cellulose derivative into the non-woven fabric.
Step (b): a step of mixing a cell or tissue (e.g., a cell or tissue that secretes a biologically active substance) with a solution capable of hydrogelation (e.g., an alginic acid solution, the gelable solution described in any of [3-1], [3-2], and [1A-9] to [1A-11]).
Step (c): a step of gelling the solution obtained in step (b).
Step (d): a step of covering the hydrogel that has been obtained in step (c) and embedding the cell or tissue with the biocompatible composite membrane that has been obtained in step (a) and including the semi-permeable membrane layer and the non-woven fabric layer. In step (d), the semi-permeable membrane layer is disposed on the outer side and the non-woven fabric layer is disposed on the hydrogel side.

The order of the steps is not limited. For example, step (a) can be performed before step (b), between step (b) and step (c), or after step (c). Multiple steps may be performed in parallel as appropriate.
Step (a) can be replaced with the following step (A1) or step (A2). Step (A1): a step of forming a biocompatible composite membrane including/consisting of a semi-permeable membrane layer and a non-woven fabric layer, wherein the semi-permeable membrane is formed by laminating a cellulose derivative on a non-woven fabric using the non-woven fabric as a support, and the non-woven fabric layer has a structure with partial intrusion of the cellulose derivative into the non-woven fabric. Step (A2): a step of forming a biocompatible composite membrane in which a cellulose derivative is laminated on one surface of a non-woven fabric using the non-woven fabric as a support and the cellulose derivative is partially intruding into a non-woven fabric.

<Step (a), step (A1), step (A2)>

In step (a), a semi-permeable membrane layer formed by a cellulose derivative with a non-woven fabric serving as a support is produced to form a biocompatible composite membrane having a structure with partial intrusion of the cellulose derivative into the non-woven fabric. In step (A1), a biocompatible composite membrane including/consisting of a semi-permeable membrane layer and a non-woven fabric layer is formed, wherein the semi-permeable membrane layer is

formed by laminating a cellulose derivative on a non-woven fabric using the non-woven fabric as a support, and the non-woven fabric layer has a structure with partial intrusion of the cellulose derivative into the non-woven fabric. In step (A2), a biocompatible composite membrane in which a cellulose derivative is laminated on one surface of a non-woven fabric using the non-woven fabric as a support and the cellulose derivative is partially intruding into the non-woven fabric is formed.

In step (a), step (A1), or step (A2), first, a cellulose derivative (e.g., cellulose acetate) is mixed with a solvent to prepare a solution containing the cellulose derivative (also referred to as a cellulose derivative-containing solution). Examples of the solvent to be mixed with the cellulose derivative include, but are not limited to, dimethyl sulfoxide, N,N-dimethylformamide, N,N-dimethylacetamide, dioxane, and acetone.

In an embodiment of the present invention, the cellulose derivative content of the cellulose derivative-containing solution is approximately 1 to approximately 30% by mass, approximately 5 to approximately 20% by mass in another embodiment, and approximately 7 to approximately 15% by mass in still another embodiment, on the basis of the total amount of the cellulose derivative-containing solution.

Then, the cellulose derivative-containing solution is brought into contact with a non-woven fabric to impregnate the non-woven fabric with the cellulose derivative-containing solution. Examples of methods for impregnating the non-woven fabric with the cellulose derivative-containing solution include pouring or applying the cellulose derivative-containing solution onto a surface of the non-woven fabric, spraying the cellulose derivative-containing solution onto the non-woven fabric, and soaking the non-woven fabric in the cellulose derivative-containing solution. For example, use of a Loeb-Sourirajan method described later in Examples is also acceptable. After that, the non-woven fabric impregnated with the cellulose derivative-containing solution is soaked in a water bath or the like kept at approximately 40 to approximately 80°C to solidify the cellulose derivative. As a result, a biocompatible composite membrane in an embodiment is obtained. Through the described method, a biocompatible composite membrane having a structure with partial intrusion of the cellulose derivative into the non-woven fabric layer can be formed.

The resulting composite membrane can be appropriately washed, cut, and stored. The storage is preferably under refrigeration.

<Step (b)>

In step (b), a cell or tissue is mixed with a solution capable of hydrogelation (e.g., an alginic acid solution, the gelable solution described in any of [3-1], [3-2], and [1A-9] to [1A-11]).

Examples of the alginic acid solution capable of hydrogelation can include an aqueous solution of sodium alginate, and any of the above-described alginic acid derivatives represented by the formula (H-I), formula (H-II), formula (I-A), and formula (II-A).

In step (b), for example, approximately 0.1 to approximately 5% by weight aqueous solution or physiological saline solution of any of the alginic acid derivatives is produced, and the aforementioned cell or tissue in a required amount is suspended in the solution.

Here, the "solution capable of hydrogelation" includes two solutions: a solution of the above-described alginic acid derivative represented by the formula (H-I); and a solution of the above-described alginic acid derivative represented by the formula (H-II). Alternatively, for example, the "solution capable of hydrogelation" includes two solutions: a solution of the above-described alginic acid derivative represented by the formula (I-A); and a solution of the above-described alginic acid derivative represented by the formula (II-A). In this case, those two solutions are separately produced without being mixed together in step (b). At that time, the cell or tissue may be mixed with one of the two solutions, or mixed with both of them.

A material other than alginic acid derivatives including the above-described alginic acid derivatives represented by the formula (H-I) and formula (H-II) and the above-described alginic acid derivatives represented by the formula (I-A) and formula (II-A) may be added to the alginic acid solution capable of hydrogelation. Examples of the material include alginic acid (including alginic acid esters and alginic acid salts) collagen, hyaluronan, gelatin, fibronectin, elastin, tenascin, laminin, vitronectin, polypeptide, heparan sulfate, chondroitin, chondroitin sulfate, keratan, keratan sulfate, dermatan sulfate, carrageenan, heparin, chitin, chitosan, agarose, agar, cellulose, methylcellulose, carboxylmethylcellulose, glycogen, and derivatives of these, and fibrin, fibrinogen, thrombin, polyglutamic acid, polylactic acid, polyglycolic acid, lactic acid-glycolic acid copolymer, gellan gum, xanthan gum, galactomannan, guar gum, locust bean gum, and tara gum.

<Step (c)>

In step (c), the solution obtained in step (b) is gelled. In the case that the above-described alginic acid derivatives represented by the formula (H-I) and formula (H-II) are used as raw materials of the alginic acid solution capable of hydrogelation, or in the case that the alginic acid derivatives represented by the formula (I-A) and formula (II-A) are used, for example, mixing the two solutions obtained in step (b) allows chemical cross-linking by Huisgen reaction to proceed; as

a result, the solution is successfully gelled.

Alternatively, a solution containing divalent metal ions (e.g., aqueous solution of calcium chloride, aqueous solution of barium chloride) or a cross-linking agent such as a solution containing a chemical cross-linking agent can be added to or brought into contact with the solution obtained in step (b) to produce a hydrogel with bonds formed through cross-linking reaction (e.g., ionic bonds of divalent metal ions, chemical bonds).

In step (c), those multiple gelling methods may be combined. In the case that the above-described alginic acid derivatives represented by the formula (H-I) and formula (H-II) are used as raw materials of the alginic acid solution capable of hydrogelation, or in the case that the alginic acid derivatives represented by the formula (I-A) and formula (II-A) are used, for example, mixing the two solutions obtained in step (b) and then bringing the resultant into contact with a solution containing divalent metal ions allows chemical crosslinking to proceed and simultaneously allows ionic cross-linking to proceed; as a result, the solution is successfully gelled. This method also enables quick structural formation of a hydrogel embedding a cell or tissue, and enables instantaneous formation of a specific structure.

In step (c), a bioprinter may be used in mixing the solutions obtained in step (b) to form a structure.

<Step (d)>

In step (d), the hydrogel that has been obtained in step (c) and embedding the cell or tissue is covered with the biocompatible composite membrane that has been obtained in step (a), step (A1), or step (A2) and including the semi-permeable membrane layer and the non-woven fabric layer.

At that time, the hydrogel is covered in such a manner that the semi-permeable membrane layer of the biocompatible composite membrane is disposed on the outer side and the non-woven fabric layer is disposed on the hydrogel side (the hydrogel is covered in such a manner that the surface of the biocompatible composite membrane with the cellulose derivative laminated on the non-woven fabric is disposed on the outer side). Examples of methods for covering the hydrogel with the biocompatible composite membrane include such a method that the biocompatible composite membrane is cut into two sheets of appropriate size, the hydrogel is placed between the two sheets, and the peripheries of the two sheets of the composite membrane are thermally fused (heat-sealed), as described above. The production method in a preferable embodiment allows the hydrogel to be covered with the composite membrane through four or less cycles, three or less cycles, two or less cycles, or a single cycle of thermal fusion.

Through those steps, the biocompatible device in an embodiment of the present invention is successfully produced.

6-2. Method of producing biocompatible device (2)

The production method in an embodiment includes the following steps (i) to (v).

Step (i): a step of producing a semi-permeable membrane layer formed by a cellulose derivative with a non-woven fabric serving as a support to form a biocompatible composite membrane having a structure with partial intrusion of the cellulose derivative into the non-woven fabric.

Step (ii): a step of forming an internal space by the biocompatible composite membrane obtained in step (i), wherein the semi-permeable membrane layer of the biocompatible composite membrane is disposed on the outer side and the non-woven fabric layer is disposed on the internal-space side.

Step (iii): a step of mixing a cell or tissue (e.g., a cell or tissue that secretes a biologically active substance) with a solution capable of hydrogelation (e.g., an alginic acid solution, the gelable solution described in any of [3-1], [3-2], and [1A-9] to [1A-11]).

Step (iv): a step of injecting the solution obtained in step (iii) into the internal space formed by the biocompatible composite membrane in step (ii).

Step (v): a step of gelling the solution injected in step (iv).

The order of the steps is not limited. For example, steps (i) and (ii) may be performed before step (iii), or between step (iii) and step (iv). Multiple steps may be performed in parallel as appropriate.

Step (i) can be replaced with the following step (i-1) or step (i-2). Step (i-1): a step of forming a biocompatible composite membrane including/consisting of a semi-permeable membrane layer and a non-woven fabric layer, wherein the semi-permeable membrane layer is formed by laminating a cellulose derivative on a non-woven fabric using the non-woven fabric as a support, and the non-woven fabric layer has a structure with partial intrusion of the cellulose derivative into the non-woven fabric. Step (i-2): a step of forming a biocompatible composite membrane in which a cellulose derivative is laminated on one surface of a non-woven fabric using the non-woven fabric as a support and the cellulose derivative is partially intruding into the non-woven fabric.

<Step (i), step (i-1), step (i-2)>

In step (i), a semi-permeable membrane layer formed by a cellulose derivative with a non-woven fabric serving as a support is produced to form a biocompatible composite membrane having a structure with partial intrusion of the cellulose derivative into the non-woven fabric. As a specific method for step (i), the same method as in step (a) described above in Method of producing biocompatible device (1) is applied.

In step (i-1), a biocompatible composite membrane including/consisting of a semi-permeable membrane layer and a non-woven fabric layer is formed, wherein the semi-permeable membrane layer is formed by laminating a cellulose derivative on a non-woven fabric using the non-woven fabric as a support, and the non-woven fabric layer has a structure with partial intrusion of the cellulose derivative into the non-woven fabric. As a specific method for step (i-1), the same method as in step (A1) described above in Method of producing biocompatible device (1) is applied.

In step (i-2), a biocompatible composite membrane in which a cellulose derivative is laminated on one surface of a non-woven fabric using the non-woven fabric as a support and the cellulose derivative is partially intruding into the non-woven fabric is formed. As a specific method for step (i-2), the same method as in step (A2) described above in Method of producing biocompatible device (1) is applied.

<Step (ii)>

In step (ii), an internal space is formed by the biocompatible composite membrane obtained in step (i), step (i-1), or step (i-2). In this step, the semi-permeable membrane layer of the biocompatible composite membrane is disposed on the outer side and the non-woven fabric layer is disposed on the internal-space side. Alternatively, the surface of the biocompatible composite membrane with the cellulose derivative laminated on the non-woven fabric is disposed on the outer side. Examples of methods for forming the internal space by the biocompatible composite membrane include such a method that the biocompatible composite membrane is cut into two sheets of appropriate size, the two sheets are placed, and the peripheries of the two sheets of the composite membrane are thermally fused (heat-sealed), as described above. The production method in a preferable embodiment allows the internal space to be formed by the composite membrane through four or less cycles, three or less cycles, two or less cycles, or a single cycle of thermal fusion.

<Step (iii)>

In step (iii), a cell or tissue is mixed with a solution capable of hydrogelation. As a specific method for step (iii), the same method as in step (b) described above in Method of producing biocompatible device (1) is applied.

<Step (iv)>

In step (iv), the solution obtained in step (iii) is injected into the internal space formed by the biocompatible composite membrane in step (ii). Any method may be used for injecting the solution without limitation, and the solution in an amount that does not cause the leakage from the composite membrane may be injected into the internal space by using a pipette or the like.

<Step (v)>

In step (v), the solution injected in step (iv) is gelled. As a specific method for step (v), the same method as in step (c) described above in Method of producing biocompatible device (1) is applied.

Through those steps, the biocompatible device in an embodiment of the present invention is successfully produced.

7. Physical properties of biocompatible device

The physical properties of the biocompatible device can be evaluated on the basis of the physical properties of the biocompatible composite membrane.

<Material permeability>

The material permeability of the biocompatible composite membrane of the present invention in an embodiment can be evaluated by using a chamber for an oral absorbability evaluation system for drugs (D/P system).

For example, the membrane is set in a chamber for the D/P system, the permeation of each solution added (e.g., insulin solution, glucose solution, albumin solution, dextran solution) is measured over time until the lapse of 24 hours, and

the material permeation rate can be calculated from the following calculation formula.

Permeation rate (%) = 100 $\times$ Test substance concentration of permeating solution / Test substance concentration at permeation equilibrium

More specifically, evaluation of material permeability can be conducted by a method described later in Examples.
The glucose permeation rate of the biocompatible composite membrane of the present invention in an embodiment as measured by a method described later in Examples at hour 24 is approximately 80% or more, approximately 85% or more in another embodiment, and approximately 90% or more in still another embodiment.
The insulin permeation rate of the biocompatible composite membrane of the present invention in an embodiment as measured by a method described later at hour 24 is approximately 30% or more, approximately 40% or more in another embodiment, and approximately 50% or more in still another embodiment.
The dextran (molecular weight: approximately 150 kD) permeation rate of the biocompatible composite membrane of the present invention in an embodiment as measured by a method described later at hour 24 is approximately 30% or less, approximately 20% or less in another embodiment, and approximately 10% or less in still another embodiment.

<Strength>

The strength of the biocompatible composite membrane of the present invention in an embodiment can be measured in tensile test, tear test, burst test, or the like. For example, the strength can be measured in tensile test in accordance with JIS K 7127 or ASTM D882. The strength of the composite membrane in the biocompatible device of the present invention in an embodiment as measured in the tensile test is, for example, 1 MPa or more, preferably approximately 1 to approximately 100 MPa, and more preferably approximately 5 to approximately 100 MPa. Tear test and burst test can be each conducted by using a common testing method for measurement.

<Thermal fusion integrity>

The thermal fusion integrity of the biocompatible composite membrane of the present invention in an embodiment can be evaluated by a method described later in Examples. Alternatively, the thermal fusion integrity can be evaluated by a method based on filter integrity test; for example, an integrity test kit for small-capacity devices (manufactured by Merck KGaA, SLTEST000) is used to measure a bubble point value, which can be regarded as an index of the thermal fusion integrity.

8. Biocompatibilities of device and composite membrane

The device and composite membrane provided herein each have biocompatibility. In the present invention, the alginic acid derivatives and cross-linked alginic acid constituting the hydrogel have been each confirmed to have satisfactory biocompatibility like alginic acid (e.g., see aforementioned PCT/JP2019/023478, PCT/JP2020/047100, and PCT/JP2019/007655).
Herein, the biocompatibilities of the device and composite membrane are confirmed later in an example on biocompatibility.

9. Applications and uses of biocompatible device

The biocompatible device provided herein can be used as a device for living-donor transplantation. The biocompatible device in a certain embodiment can be preferably used for therapeutic applications for diabetes mellitus. The biocompatible device can be used for treatment of any diabetes mellitus of type 1 diabetes mellitus, type 2 diabetes mellitus, and diabetes mellitus that develops through any other mechanism, and is preferably used for treatment of type 1 diabetes mellitus. The device in a preferable embodiment is superior not only in biocompatibility but also in stability with less cytotoxicity, almost no adhesion and inflammation at the transplantation site, and less dissolution of gel, and allows blood glucose to be regulated for a long period of time through sustained hypoglycemic action.
Examples of the transplantation site of the device can include, but are not limited to, subcutaneous, intramuscular, intraperitoneal, intrahepatic, intraomental, and subrenal sites, and subcutaneous, intramuscular, intraperitoneal, or intraomental transplantation is preferable. As a method for transplanting the device in a living body, such means as incision and placement, an endoscope, and a laparoscope, can be used.

10. Kit for living-donor transplantation

A kit for living-donor transplantation is provided herein. The kit for living-donor transplantation in some embodiments includes: a biocompatible device having an internal space formed by a biocompatible composite membrane including a semi-permeable membrane layer and a non-woven fabric layer; and a container including a cell or tissue that secretes a biologically active substance, wherein the kit is used in such a manner that the cell or tissue is enclosed in the internal space. The "biocompatible device", "a cell or tissue", and others are as described above.

The kit for living-donor transplantation in a certain embodiment can be in the form of one package including: (1) a pack enclosing a biocompatible device; (2) a container (e.g., a vial) enclosing a cell or tissue, in a frozen state, that secretes a biologically active substance. Here, the biocompatible device of (1) may be packed in such a state that a part of the biocompatible composite membrane (e.g., one of four sides for a plate-like shape) has not been fused so as to enclose a cell or tissue in the internal space before use. In this case, a cell or tissue is enclosed in the internal space of the biocompatible device before use, and the unfused part (e.g., the residual one side for a plate-like shape) is thermally fused; as a result, the internal space is successfully sealed.

The kit for living-donor transplantation in a certain embodiment can be in the form of one package including, in addition to (1) and (2): (3) a container such as a vial enclosing alginic acid derivatives, in a frozen state, that are gelled through chemical cross-linking; and (4) a container such as a vial enclosing physiological saline for dissolution. The kit for living-donor transplantation in another embodiment can be in such a state that the cell or tissue of (2) and the alginic acid derivatives of (3) have been dissolved in advance in a container such as a vial. The kit for living-donor transplantation in still another embodiment can be in such a state that the cell or tissue of (2) have been mixed with a solution of the alginic acid derivatives of (3), gelled, and enclosed in advance in a pack. The kit of the present invention can further include an accessory such as a syringe, a pipette, an injection needle, a dedicated filling device, and an instruction manual, as appropriate.

All of the documents and publications cited herein are entirely incorporated herein by reference, irrespective of their purposes.

The purpose, features, and advantages of the present invention, and the ideas therefor are obvious from the description in the present specification to those skilled in the art, and those skilled in the art could easily put the present invention into practice with reference to the description in the present specification. The best modes, specific examples, and others for implementing the present invention show preferable embodiments for implementing the present invention, and are only presented for illustration or description, not limiting the present invention thereto. It is obvious to those skilled in the art that various modifications based on the description in the present specification are permitted within the intension and scope of the present invention disclosed herein.

Examples

Chemically modified alginic acid derivatives used in examples shown below were alginic acid derivatives synthesized in accordance with methods described in Examples in International Publication No. WO 2020/262642, specifically, the alginic acid derivative having a cyclic alkyne group, (A01) or (A02), and the alginic acid derivative having an azide group, (N01).

Huisgen reaction occurs by mixing the two alginic acid derivatives, forming a chemically cross-linked alginic acid containing the cross-linked structures of the formulas (LZ-8) and (LZ-8-r) or a chemically cross-linked alginic acid containing the cross-linked structures of the formulas (LY-2) and (LY-2-r), and thus forming a hydrogel. The structural formation of the hydrogel can be made quicker by using ionic cross-linking reaction with divalent metal ions in combination, and such ionic cross-linking reaction was used in combination in some examples.

[Example A] Example of production of composite membrane

(1) Preparation of film-forming solution (dope)

Cellulose acetate (LT-75) (manufactured by Daicel Corporation), PEG200 (manufactured by Hayashi Pure Chemical Ind., Ltd.), and dimethyl sulfoxide (DMSO) (manufactured by NACALAI TESQUE, INC.) were weighed to give 11% by weight, 10% by weight, and 79% by weight, respectively, and a mixed solution of PEG200 and DMSO was first prepared. Then, cellulose acetate was put in small portions into the mixed solution while the mixed solution was stirred at 70 to 80°C; thus, a dope for film formation was obtained.

(2) Production of composite membrane

A composite membrane was produced by a Loeb-Sourirajan method, a method of producing asymmetric porous

membranes (known documents therefor include S. Loeb and S. Sourirajan, ibid No. 59-28 (1959)). A non-woven fabric (HOP-30H) (manufactured by HIROSE PAPER MFG. CO., LTD.) was cut into the same size as a glass sheet, and attached to the glass sheet with tape. A film applicator was placed on one edge of the non-woven fabric on the glass sheet, the dope having the temperature kept at 60°C was homogeneously poured from one end of the applicator to the other end in a width of about 1 cm, and the applicator was slid while being pressed onto the non-woven fabric to cast the dope on the non-woven fabric.

The non-woven fabric after casting, together with the glass sheet, was sunk in a water bath at 60°C for several minutes to solidify the dope; thus, a composite membrane having cellulose acetate laminated on the non-woven fabric was obtained. The composite membrane obtained, together with the glass sheet, was washed, then peeled off from the glass sheet, and soaked in water nearly overnight to sufficiently remove the solvent contained in the membrane.

(3) Character, physical properties, etc., of composite membrane

- Thermal fusion integrity

The composite membrane was cut into two rectangular sheets of approximately 3.5 cm × approximately 2.0 cm in size, the two sheets were overlapped in such a manner that the non-woven fabric layers faced each other on the inner side, and three sides, with one 2.0-cm side excluded, were subjected to thermocompression bonding with a POLYSEALER (manufactured by FUJIIMPULSE (R) CO., LTD., P-300) to shape into an envelope-like structure. Into the internal space generated through the thermocompression bonding, a coloring solution (5 mg/mL TRITC-dextran 150 solution prepared with physiological saline (manufactured by TdB Labs AB)) was injected, and the residual side was subjected to thermocompression bonding. After the lapse of a certain time, the absence of the leakage of the coloring solution to the outer side of the sealed parts was checked. If the leakage of liquid was not found, the composite membrane was determined to have no defect on thermal fusion properties.

The composite membrane of the present invention had no leakage of liquid to the outer side of the sealed parts, thus confirmed to have sufficient thermal fusion properties.

- Strength

Higher strength than those of commercially available membranes was confirmed from visual observation and touch feeling.

[Example B] Evaluation of material permeability of composite membrane

The material permeability of the composite membrane was evaluated by using a horizontal chamber (manufactured by Osaka Rika) for an oral absorbability evaluation system for drugs (D/P system).

A membrane to be evaluated was cut into a piece of 3 cm × 3 cm in size, and the piece was soaked in 0.1% Tween 20 solution (dialysis solution) prepared with physiological saline, and then set in the chamber. Into a bath on the addition side of the chamber and a bath positioned on the opposite side (recovery side) and separated by the membrane, 9 mL and 7.5 mL of the dialysis solution were put, respectively. After that, 1 mL of a test solution shown below was added to the addition side while the two bathes were stirred (200 rpm) at room temperature to initiate material permeability test. The time when the solution was added was defined as the start of test, and sampling was performed for the recovery side 2 hours and 24 hours after the start of test. Sampling was performed also for the addition side 24 hours after the start of test. The test substance concentration of each sampled solution was calculated on the basis of quantitative values obtained by using a measurement kit for the test substance or quantitative values obtained by fluorescence measurement for labeled products, and the material permeation rate was further calculated from a calculation formula shown below.

(Membranes used)

Example: composite membrane produced in Example A

Reference Example: dialysis tube "Spectra/Por CE (molecular weight cutoff: 100000)" manufactured by Repligen Corporation (hereinafter, referred to as a commercially available membrane)

(Test solution)

- 100 mg/dL glucose solution (manufactured by Otsuka Pharmaceutical Co., Ltd.)
- 2000 ng/mL insulin solution (manufactured by Eli Lilly and Company)
- 500 μg/mL FITC-albumin solution (manufactured by Sigma-Aldrich Co. LLC)
- 2000 μg/mL TRITC-dextran 150 solution (manufactured by TdB Labs AB) (Calculation formula for material permeation rate)

Permeation rate (%) = 100 × Test substance concentration of sampled solution / Test substance concentration at permeation equilibrium

Table 16 shows the material permeation rates of the composite membrane of Example and the commercially available membrane.

Directions for the membranes to be evaluated are as follows: for the composite membrane, the semi-permeable membrane layer side is defined as Out and the non-woven fabric layer side as In; for the commercially available membrane, the outer side of the tubular dialysis tube is defined as Out and the inner side thereof as In; and the direction of material permeation in the evaluation system is indicated with an arrow symbol. Specifically, for example, "Composite membrane (Out → In)" means that evaluation was conducted with the semi-permeable membrane layer side of the composite membrane facing the addition side and the non-woven fabric layer side thereof facing the recovery side.

[Table 16]

| Membrane to be evaluated | Glucose | | Insulin | | Albumin | | Dextran | |
|---|---|---|---|---|---|---|---|---|
| | 2 hours | 24 hours | 2 hours | 24 hours | 2 hours | 24 hours | 2 hours | 24 hours |
| Composite membrane (Out → In) | 23% | 98% | 5% | 57% | 2% | 27% | 1% | 9% |
| Composite membrane (In → Out) | 21% | 95% | 5% | 55% | 2% | 26% | 1% | 6% |
| Commercially available membrane (Out → In) | 37% | 100% | 4% | 51% | 4% | 38% | 1% | 13% |
| Commercially available membrane (In → Out) | 26% | 97% | 9% | 73% | 2% | 22% | 0% | 5% |

As demonstrated in Table 16, the composite membrane of Example was confirmed to exhibit material permeability comparable to that of the commercially available membrane, and to have functions as a semi-permeable membrane.

[Example C] Evaluation of complement sequestration capacity

A device containing a sensitized ovine erythrocyte solution was shaken in a buffer mixed with human serum, and the hemolysis rate was measured to evaluate the complement sequestration capacity of the device.

(1) Production of device for evaluating complement sequestration capacity

A solution was prepared by dissolving the alginic acid derivatives (A02) and (N01) in CH50 buffer (a buffer attached to One-point CH50 "Seiken" (Denka Company Limited)) to give alginic acid derivatives having a final concentration of 0.5% (mixing equal amounts of the alginic acid derivative (A02) having a final concentration of 0.25% and the alginic acid derivative (N01) having a final concentration of 0.25%), and sensitized ovine erythrocytes in a 1/3 volume of the solution were added (erythrocyte-containing alginic acid solution).

(Production of hydrogel)

A commercially available membrane (a dialysis tube of 1 cm in plane width) was cut into a piece of approximately 2.7 cm in length, and one side of 1 cm in width was subjected to thermocompression bonding to shape into an envelope-like structure. Into the internal space generated through the shaping, 100 μL of the erythrocyte-containing alginic acid solution was injected. The residual side was subjected to thermocompression bonding, and the resultant was then soaked in 10

mmol/L $BaCl_2$ solution for 10 minutes to promote the hydrogel formation.

(Device of Example)

The thus-produced hydrogel was taken out of the bag. The composite membrane produced in Example A was cut to prepare two rectangular sheets of approximately 1.5 cm × approximately 2.5 cm in size, the two sheets were overlapped on the top and bottom of the gel in such a manner that the non-woven fabric layers were on the gel side, and the four sides were subjected to thermocompression bonding to give a device of Example.

(Device of Reference Example)

The hydrogel produced in the above, which was enveloped by the commercially available membrane, was used as a device of Reference Example.

(2) Evaluation method

The devices for evaluation, in each of which the hydrogel had been enclosed in the composite membrane of Example or the commercially available membrane, were each immersed in 3 mL of CH50 buffer to which 10 μL of human serum (complement control "Seiken" HC(H) (Denka Company Limited)) had been added, and incubated at 30°C for 24 hours. After the incubation, the absorption intensities of the supernatants at 541 nm were measured, and the hemolysis rates were calculated. As a control, a mixture of CH50 buffer and sensitized ovine erythrocytes at a volume ratio of 3:1 (erythrocyte solution) was used in place of the devices. To 3 mL of CH50 buffer with addition of 10 μL of human serum, 100 μL of the erythrocyte solution was added, incubated in the same manner as for the devices, and evaluated as a complement hemolysis control group. The hemolysis rate of 3.01 mL of water to which 100 μL of the erythrocyte solution had been added was defined as 100%, and the hemolysis rates of the samples were calculated.

The results showed that the hemolysis rate of the complement hemolysis control group was about 70%, whereas the hemolysis rates of the device of Example with the composite membrane of Example and the device of Reference Example with the commercially available membrane were both 1% or less, and almost no hemolysis was found. Thus, the device produced with the composite membrane of Example was confirmed to have complement sequestration capacity.

[Example D] Glucose-responsive insulin secretion test

With devices containing insulin-secreting cells, evaluation of glucose-responsive insulin secretion capacity was conducted.

(1) Production of devices

To 100 μL of a solution obtained by mixing equal amounts of 0.5% aqueous solutions of alginic acid derivatives (A01) and (N01), MIN6 cells ($2.5 \times 10^6$ cells), which were derived from pancreatic β cells, were suspended. The composite membrane produced in Example A was cut to prepare two rectangular sheets of approximately 2 cm × approximately 1 cm in size, the two sheets were overlapped in such a manner that the non-woven fabric layers faced each other on the inner side, and three sides, with one of the 1-cm sides excluded, were subjected to thermocompression bonding to shape in an envelope-like structure. In the same manner as in Example C, the commercially available membrane was shaped into an envelope-like structure of the same size. Into each of the internal spaces generated through the shaping, the cell suspension was injected. The residual side in each structure was subjected to thermocompression bonding, and the resultants were then each soaked in 50 mmol/L $CaCl_2$ solution for 10 minutes to promote the hydrogel formation, giving a device of Example and device of Reference Example for evaluation.

(2) Evaluation method

Each device was cultured with culture medium (15% FBS-containing Optimized DMEM (Addexbio Technologies)) at 37°C for 1 day, and evaluated on insulin secretion depending on glucose concentration.

Specifically, each device was soaked in 2 mmol/L glucose solution for 1 hour, or in 20 mmol/L glucose solution for 2 hours, and the insulin concentrations resulting from secretion into a solution were measured under the respective conditions. Thereby, evaluation can be conducted on the insulin secretion capacity of cells depending on glucose concentration.

Table 17 shows the results of the glucose-responsive insulin secretion test for the device of Example produced with the composite membrane of Example and the device of Reference Example produced with the commercially available

membrane. In Table 17, "HG" signifies high-glucose (20 mmol/L glucose solution), and "LG" signifies low-glucose (2 mmol/L glucose solution).

[Table 17]

| Device to be evaluated | Insulin concentration (pg/mL/min) | |
|---|---|---|
| | LG | HG |
| Device of Example | 14.6 | 30.9 |
| Device of Reference Example | 14.9 | 40.0 |

The result that the device produced with the composite membrane of Example exhibited glucose-responsive insulin secretion comparable to that of the device with the commercially available membrane suggested that comparable cellar functions are exerted in the two devices.

[Example E] Biocompatibility test (rat) for composite membrane

(1) Production of membrane

The following four were produced as membranes for implantation.

- (Example): a membrane obtained in such a manner that the composite membrane produced in Example A was sterilized in advance by UV irradiation and cut into two rectangular sheets of approximately 2.8 cm × approximately 1.8 cm in size, the two sheets were overlapped in such a manner that the non-woven fabric layers faced each other on the inner side, and the four sides were subjected to thermocompression bonding
- (Reference Example): a membrane obtained in such a manner that the commercially available membrane was cut into two rectangular sheets of approximately 2 cm × approximately 1 cm in size, the two sheets were overlapped, and the four sides were subjected to thermocompression bonding
- (Adhesion-inducing control): a membrane obtained in such a manner that 0.75% Zinc diethyldithiocarbamate-containing polyurethane (Food and Drug Safety Center Hatano Research Institute) was cut into a sheet of approximately 2 cm × approximately 1 cm, and the sheet was autoclave-sterilized
- (Non-influencing control): a membrane obtained in such a manner that a high-density polyethylene sheet (Food and Drug Safety Center Hatano Research Institute) was cut into a sheet of approximately 2 cm × approximately 1 cm, and the sheet was autoclave-sterilized

(2) Implantation method and evaluation method

Eight-week-old Sprague-Dawley rats (manufactured by Japan SLC, Inc.) were subjected to intraperitoneal implantation of the four membranes and wound closure under anesthesia, and reared and observed. Two weeks after the implantation, each animal was subjected to laparotomy under anesthesia, and visually observed for intraperitoneal adhesion and the state of the implanted object. Table 18 shows the results of evaluation of membrane adhesion, membrane alteration, and organ-to-organ adhesion by visual observation.

[Table 18]

| | Example | Reference Example | Adhesin-inducing control | Non-influencing control | Without implantation (only surgery) |
|---|---|---|---|---|---|
| Membrane adhesion | none | none | inclusion in fat | none | - |
| Membrane alteration | none | none | discoloration | none | - |

(continued)

| | Example | Reference Example | Adhesin-inducing control | Non-influencing control | Without implantation (only surgery) |
|---|---|---|---|---|---|
| Organo-to-organ adhesion | none | slight adhesion | adhesion between fat and intestinal tract adhesion between fat and liver periphery | none | none |

As demonstrated in Table 18, the composite membrane of Example did not exhibit any of membrane adhesion, membrane alteration, and organ-to-organ adhesion, and thus is suitable for transplantation. By contrast, the commercially available membrane as Reference Example was observed to exhibit slight organ-to-organ adhesion.

While body weight loss was found for all of the cases, it was only transient, and probably was due to the influence of the treatments, and the rats recovered after that. For general condition, no abnormality was found, as well.

[Example F] Biocompatibility test for devices (dogs, minipigs)

(1) Production of devices

The following two were produced as devices for implantation.

(Device of Example)

The composite membrane produced in Example A was cut into two square sheets of approximately 2.4 cm × approximately 2.4 cm in size, the two sheets were overlapped in such a manner that the non-woven fabric layers faced each other on the inner side, and three sides were subjected to thermocompression bonding to shape into an envelope-like structure. Into the internal space generated through the shaping, 156 μL of a mixture of equal amounts of 1% aqueous solutions of the alginic acid derivatives (A02) and (N01) was injected. The residual side was subjected to thermocompression bonding, and the structure was then soaked in 10 mmol/L $BaCl_2$ solution for 10 minutes to promote the hydrogel formation; thus, a device of Example was obtained.

(Device of Reference Example)

The commercially available membrane was cut into two square sheets of approximately 2.4 cm × approximately 2.4 cm in size, the two sheets were overlapped, and three sides were subjected to thermocompression bonding to shape into an envelope-like structure. Into the internal space generated through the shaping, 156 μL of a mixture of equal amounts of 1% aqueous solutions of the alginic acid derivatives (A02) and (N01) was injected. The residual side was subjected to thermocompression bonding, and the structure was then soaked in 10 mmol/L $BaCl_2$ solution for 10 minutes to promote the hydrogel formation; thus, a device of Reference Example was obtained.

(2) Implantation method and evaluation method

Beagle dogs and minipigs (Goettingen minipigs) were subjected to intraperitoneal implantation of the devices and wound closure under anesthesia, and reared and observed. Four weeks after the implantation, each animal was subjected to laparotomy under anesthesia, and visually observed for intraperitoneal adhesion and the state of the implanted object.

The results found slight adhesion to surrounding tissues in the case with implantation of the device of Reference Example. By contrast, adhesion, inflammation, or the like was not observed in the case with implantation of the device of Example. No influence on peritoneal organs was found in the case with implantation of the device of Example.

[Example G] Production of device with 3D printer and biocompatibility test (dogs, minipigs) (1) Production of device

An aqueous solution containing the alginic acid derivatives (A02) and (N01) (final concentration: 0.5% for each), alginic acid (A-2: manufactured by Mochida Pharmaceutical Co., Ltd., sodium alginate listed in Table 1) (final concentration: 1%), and $BaCl_2$ (final concentration: 2.5 mmol/L) was prepared, and injected with a 3D printer (BIO X (TM), manufactured by CELLINK) via a nozzle of 410 μm in diameter to give a rectangular shape of approximately 6.5 cm × approximately 3 cm. After that, 10 mmol/L $BaCl_2$ solution was laminated on the injected product, and the resultant was

left to stand for 10 minutes to form a hydrogel.

The composite membrane produced in Example A was cut to prepare two rectangular sheets of approximately 7 cm × approximately 3.5 cm in size, the two sheets were overlapped on the top and bottom of the hydrogel in such a manner that the non-woven fabric layers were on the gel side, and the four sides were subjected to thermocompression bonding to give a device for implantation.

(2) Implantation method and evaluation method

The device produced was implanted in beagle dogs and minipigs (Goettingen minipigs) and evaluated in the same manner as in Example F (Biocompatibility test for devices (dogs, minipigs)).

As shown by the results, even for the device produced by using the hydrogel produced with the 3D printer and the composite membrane produced in Example A, any adhesion or inflammation probably due to a material of the device was not observed.

[Example H] Examination of implantation site in biocompatibility test (dogs, minipigs)

(1) Production of devices

The composite membrane produced in Example A was cut into two square sheets of approximately 7 cm × approximately 3.5 cm in size, the two sheets were overlapped in such a manner that the non-woven fabric layers faced each other on the inner side, and three sides, with one of the short sides excluded, were subjected to thermocompression bonding to shape into an envelope-like structure. Into the internal space generated through the shaping, 700 μL of a mixture of equal amounts of 1% aqueous solutions of the alginic acid derivatives (A02) and (N01) was injected. The residual side was subjected to thermocompression bonding, and the structure was then soaked in 10 mmol/L $BaCl_2$ solution for 10 minutes to promote the hydrogel formation; thus, a device for implantation was obtained.

(2) Implantation method and evaluation method

In accordance with the method in Example F (Biocompatibility test for devices (dogs, minipigs)), the device was implanted into beagle dogs and minipigs (Goettingen minipigs) at any of the following three implantation sites.

- Implantation site 1: inside of peritoneal cavity
  Intraperitoneal implantation and wound closure were performed under anesthesia.
- Implantation site 2: inside of omental pocket
  After laparotomy under anesthesia, the greater omentum was folded in half, and sewed together to produce a pocket. The device was implanted in the pocket, and wound closure was performed.
- Implantation site 3: abdominal wall

After laparotomy under anesthesia, the device was sutured on an abdominal wall away from the midline, and wound closure was performed.

Four weeks after the implantation, each animal was subjected to laparotomy under anesthesia, and visually observed for intraperitoneal adhesion and the state of the implanted object.

As shown by the results, any adhesion or inflammation probably due to a material of the device was not observed, irrespective of the implantation sites.

[Example I] Drug efficacy evaluation for device (mice)

(1) Production of device

The same MIN6 cells ($2.2 \times 10^6$ cells) as in Example D were suspended in 50 μL of a solution obtained by mixing equal amounts of 0.5% aqueous solutions of the alginic acid derivatives (A01) and (N01). The composite membrane produced in Example A was cut to prepare two rectangular sheets of approximately 2 cm × approximately 1 cm in size, the two sheets were overlapped in such a manner that the non-woven fabric layers faced each other on the inner side, and three sides, with one of the 1-cm sides excluded, were subjected to thermocompression bonding to shape into an envelope-like structure. In the same manner as in Example C, the commercially available membrane was shaped into an envelope-like structure of the same size. Into each of the internal spaces generated through the shaping, the cell suspension was injected. The residual side in each structure was subjected to thermocompression bonding, and the resultants were then each soaked in 50 mmol/L $CaCl_2$ solution for 10 minutes to promote the hydrogel formation, giving a

device of Example and device of Reference Example for evaluation.

(2) Implantation method and evaluation method

C57BL/6 mice to which diabetes mellitus had been induced with streptozotocin were subjected to intraperitoneal implantation of the devices and wound closure under anesthesia, and reared and observed. For 4 weeks after the implantation, the blood glucose levels were measured over time.

Table 5 shows the drug efficacy effects of the device of Example and device of Reference Example.

Here, if the blood glucose level at measurement was 200 mg/dL or more lower than that before the implantation, the animal was determined to have cured. A determination was made on curing from the blood glucose levels of each animal individual 5 days and 25 days after the implantation, and the proportion of cured animals out of all the animal individuals was calculated as the cure rate.

[Table 19]

| | Cure rate 5 days after implantation | Cure rate 25 days after implantation |
|---|---|---|
| Device of Example | 60% (N = 3/5) | 80% (N = 4/5) |
| Device of Reference Example | 66% (N = 2/3) | 33% (N = 1/3) |

As shown by the results, the device of Example was confirmed to have an effect comparable to or higher than that of the device of Reference Example with the commercially available membrane.

In particular, in contrast to the result that the device of Reference Example exhibited a cure rate of 33% 25 days after the implantation, the device of Example exhibited a high cure rate of 80% at that time.

Probably, the unevenness of the non-woven fabric surface functioned as an anchor for the hydrogel, and the maldistribution, folding, and collapse or the like of the gel in the device were thereby successfully prevented. It is expected that this resulted in an enhanced availability of cells embedded in the hydrogel, leading to a much higher therapeutic effect.

Reference Signs List

1 biocompatible device
2 biocompatible composite membrane
21 non-woven fabric layer (non-woven fabric)
211 part without any intrusion of constituent component of semi-permeable membrane
212 part with partial intrusion of constituent component of semi-permeable membrane into non-woven fabric
22 semi-permeable membrane layer (semi-permeable membrane)
3 internal space
4 cell or tissue
5 hydrogel
6 fusion site

The present invention can include the following embodiments.

[1] A biocompatible device comprising an internal space configured to accommodate a cell or tissue that secretes a biologically active substance, wherein the internal space is formed by disposing a biocompatible composite membrane including a semi-permeable membrane layer and a non-woven fabric layer in the device such that the semi-permeable membrane layer is on an outer side and the non-woven fabric layer is on an internal-space side, the semi-permeable membrane layer comprises a cellulose derivative laminated on a non-woven fabric using the non-woven fabric as a support, and the non-woven fabric layer comprises a thermoplastic non-woven fabric.
[2] The device according to [1], having immunoisolatability.
[3] The device according to [1], wherein the cellulose derivative is cellulose acetate.
[4] The device according to [1], wherein the non-woven fabric comprises one or more resins selected from the group consisting of polyethylene, polypropylene, and polyethylene terephthalate.
[5] The device according to [1], wherein the internal space is formed by partial fusion of a surface of the non-woven fabric layer that is free of cellulose derivative.
[6] The device according to [1], wherein the composite membrane has a structure with partial intrusion of the cellulose

derivative into the non-woven fabric.

[7] The device according to [1], wherein the cell that secretes a biologically active substance is an insulin-secreting cell or a pancreatic islet.

[8] The device according to [1], wherein the cell or tissue that secretes a biologically active substance is enclosed in the internal space with a hydrogel serving as a carrier.

[9] The device according to [1], wherein the composite membrane has a thickness of 10 to 1500 μm.

[10] The device according to any one of [1] to [9], for use in living-donor transplantation.

[11] A biocompatible composite membrane for producing the device according to any one of [1] to [9].

[12] A kit for living-donor transplantation, the kit comprising: a biocompatible device having an internal space formed by a biocompatible composite membrane including a semi-permeable membrane layer and a non-woven fabric layer; and a container including a cell or tissue that secretes a biologically active substance, wherein the kit is used in such a manner that the cell or tissue is enclosed in the internal space.

[13] A method of producing a biocompatible device, the method comprising a step of covering a hydrogel embedding a cell or tissue that secretes a biologically active substance with a biocompatible composite membrane including a semi-permeable membrane layer and a non-woven fabric layer, wherein, in the step, the semi-permeable membrane layer is disposed on an outer side and the non-woven fabric layer is disposed on a hydrogel side.

[14] A method of producing a biocompatible device, the method comprising a step of injecting a solution comprising a cell or tissue that secretes a biologically active substance and is mixed with a solution capable of hydrogelation into an internal space formed by a biocompatible composite membrane including a semi-permeable membrane layer and a non-woven fabric layer and gelling the solution capable of hydrogelation, wherein, in the step, the semi-permeable membrane layer is on an outer side and the non-woven fabric layer is on an internal-space side in the composite membrane.

## Claims

**1.** A biocompatible device comprising an internal space configured to accommodate a cell or tissue that secretes a biologically active substance, wherein

the internal space is formed by disposing a biocompatible composite membrane including a semi-permeable membrane layer and a non-woven fabric layer in the device such that the semi-permeable membrane layer is on an outer side and the non-woven fabric layer is on an internal-space side,
the semi-permeable membrane layer comprises a cellulose derivative laminated on a non-woven fabric using the non-woven fabric as a support, and
the non-woven fabric layer comprises a thermoplastic non-woven fabric.

**2.** The device according to claim 1, having immunoisolatability.

**3.** The device according to claim 1, wherein the cellulose derivative is cellulose acetate.

**4.** The device according to claim 1, wherein the non-woven fabric comprises one or more resins selected from the group consisting of polyethylene, polypropylene, and polyethylene terephthalate.

**5.** The device according to claim 1, wherein the internal space is formed by partial fusion of a surface of the non-woven fabric layer that is free of cellulose derivative.

**6.** The device according to claim 1, wherein the cell that secretes a biologically active substance is an insulin-secreting cell or a pancreatic islet.

**7.** The device according to claim 1, wherein the cell or tissue that secretes a biologically active substance is enclosed in the internal space with a hydrogel serving as a carrier.

**8.** The device according to claim 1, wherein the composite membrane has a thickness of 10 to 1500 μm.

**9.** The device according to any one of claims 1 to 8, for use in living-donor transplantation.

**10.** A biocompatible composite membrane for producing the device according to any one of claims 1 to 8.

**11.** A kit for living-donor transplantation, the kit comprising:

a biocompatible device having an internal space formed by a biocompatible composite membrane including a semi-permeable membrane layer and a non-woven fabric layer; and
a container including a cell or tissue that secretes a biologically active substance, wherein
the kit is used in such a manner that the cell or tissue is enclosed in the internal space.

**12.** A method of producing a biocompatible device, the method comprising a step of covering a hydrogel embedding a cell or tissue that secretes a biologically active substance with a biocompatible composite membrane including a semi-permeable membrane layer and a non-woven fabric layer, wherein, in the step, the semi-permeable membrane layer is disposed on an outer side and the non-woven fabric layer is disposed on a hydrogel side.

**13.** A method of producing a biocompatible device, the method comprising a step of injecting a solution comprising a cell or tissue that secretes a biologically active substance and is mixed with a solution capable of hydrogelation into an internal space formed by a biocompatible composite membrane including a semi-permeable membrane layer and a non-woven fabric layer and gelling the solution capable of hydrogelation, wherein, in the step, the semi-permeable membrane layer is on an outer side and the non-woven fabric layer is on an internal-space side in the composite membrane.

Fig. 1

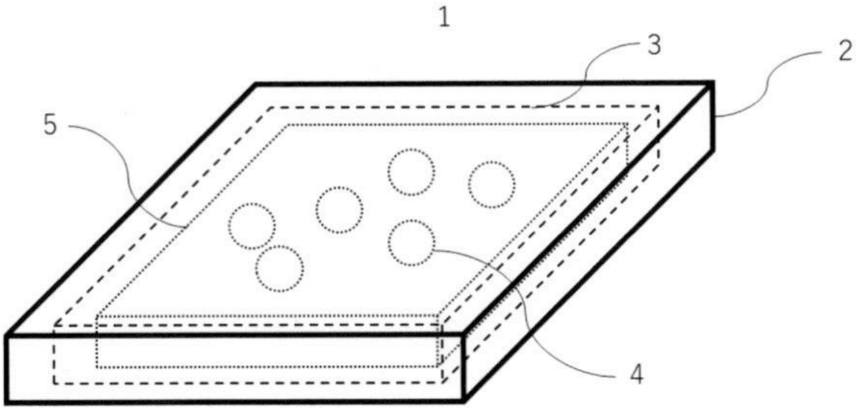


Fig. 2

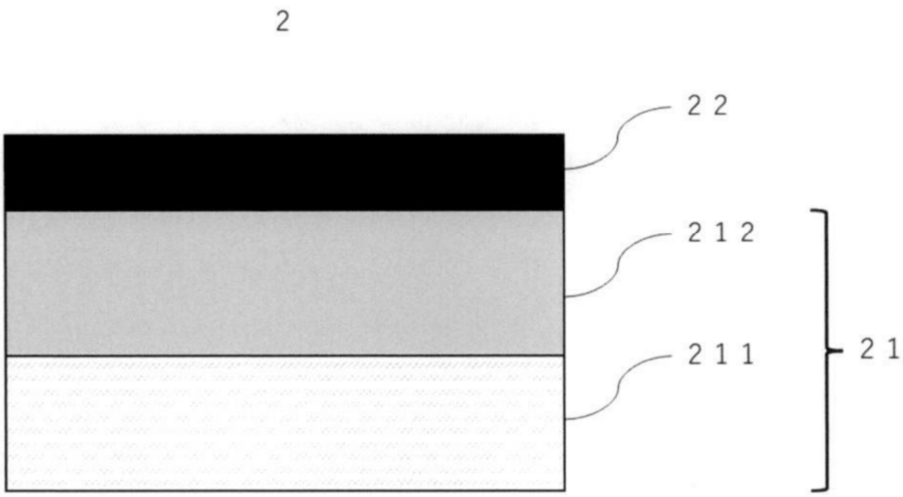


Fig. 3

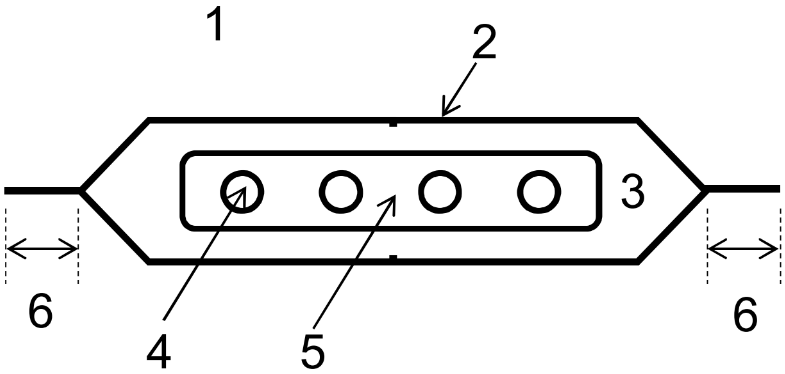

Fig. 4

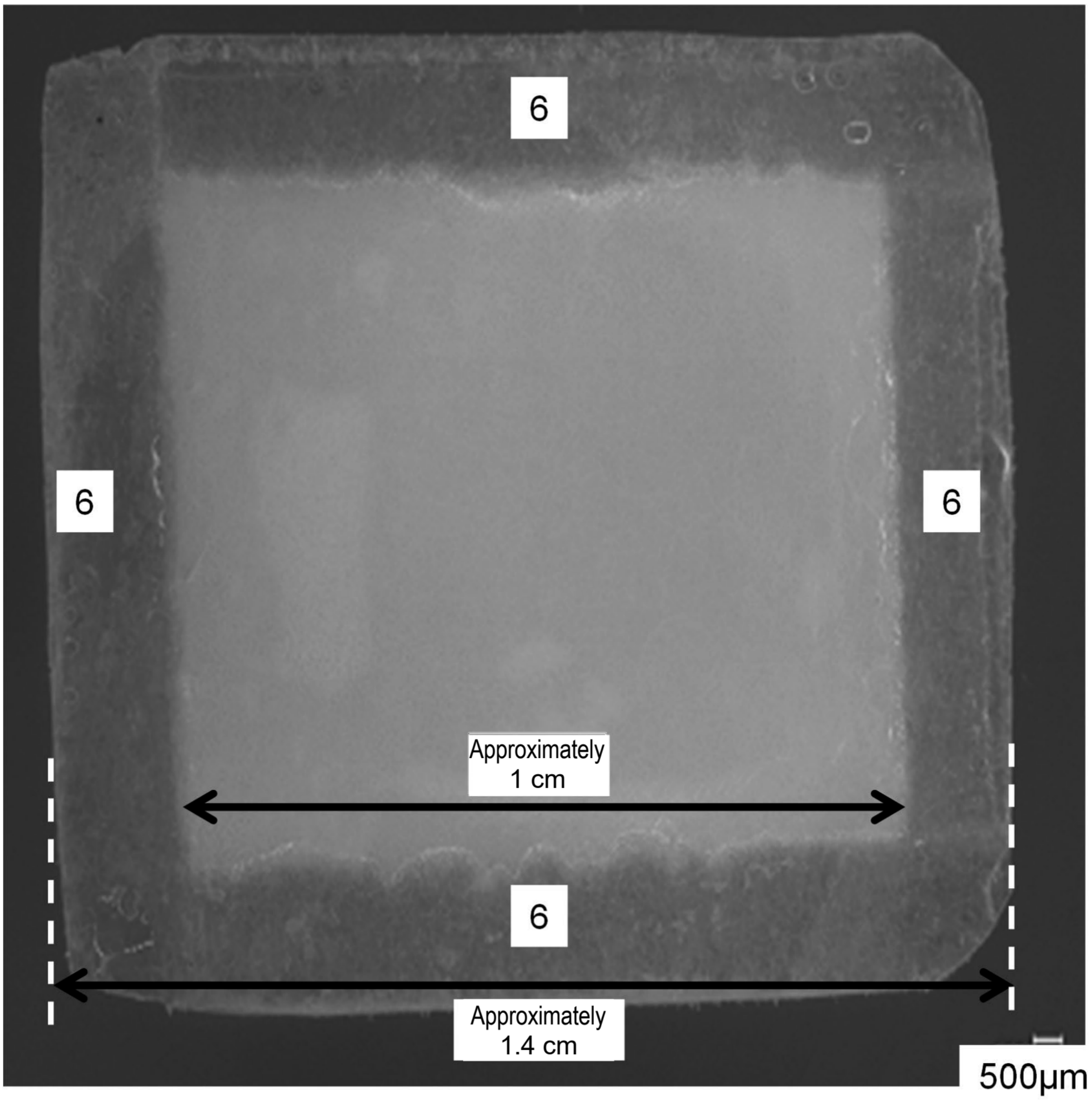

**INTERNATIONAL SEARCH REPORT**

International application No. **PCT/JP2024/023458**

**A. CLASSIFICATION OF SUBJECT MATTER**

***A61L 27/40***(2006.01)i; ***A61K 9/70***(2006.01)i; ***A61K 35/39***(2015.01)i; ***A61K 47/32***(2006.01)i; ***A61K 47/38***(2006.01)i; ***A61L 27/20***(2006.01)i; ***A61L 27/38***(2006.01)i; ***A61L 27/52***(2006.01)i; ***A61L 27/54***(2006.01)i; ***A61P 3/10***(2006.01)i
FI: A61L27/40; A61K9/70; A61K35/39; A61K47/32; A61K47/38; A61L27/20; A61L27/38; A61L27/52; A61L27/54; A61P3/10

According to International Patent Classification (IPC) or to both national classification and IPC

**B. FIELDS SEARCHED**

Minimum documentation searched (classification system followed by classification symbols)

A61L27/40; A61K9/70; A61K35/39; A61K47/32; A61K47/38; A61L27/20; A61L27/38; A61L27/52; A61L27/54; A61P3/10

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2017-502008 A (DEFYMED) 19 January 2017 (2017-01-19) claims, examples | 11 |
| Y | WO 2022/092198 A1 (KURARAY CO., LTD.) 05 May 2022 (2022-05-05) claims, paragraphs [0011], [0025]-[0026], [0042], [0063]-[0064], [0096]-[0097], [0112], fig. 3, 8 | 1-13 |
| Y | JP 2003-320227 A (DAICEL CHEM. IND. LTD.) 11 November 2003 (2003-11-11) claims | 1-13 |
| Y | JP 2000-516637 A (LABORATORIOS PHOENIX U.S.A., INC.) 12 December 2000 (2000-12-12) claims | 1-13 |
| A | JP 2018-515582 A (NEUROTECH USA INC.) 14 June 2018 (2018-06-14) claims, paragraph [0105] | 1-13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 July 2024** | **06 August 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2024/023458**

**C. DOCUMENTS CONSIDERED TO BE RELEVANT**

| Category* | Citation of document, with indication, where appropriate, of the relevant passages | Relevant to claim No. |
|---|---|---|
| A | JP 2013-537820 A (NSGENE A/S) 07 October 2013 (2013-10-07)<br>drawings | 1-13 |
| A | JP 54-57476 A (NITTO ELECTRIC IND CO.) 09 May 1979 (1979-05-09)<br>claims | 1-13 |
| A | JP 2021-527527 A (YALE UNIVERSITY) 14 October 2021 (2021-10-14)<br>claims | 1-13 |
| A | 前田葵ほか, 再生医療, バイオ人工膵臓デバイスに用いる分画膜の高性能化, 2016, vol. 15, Suppl, p. 197, (MAEDA, Aoi et al. Regenerative medicine.), non-official translation (Performance enhancement of fractionation membranes using bioartificial pancreas devices)<br>O-01-1 | 1-13 |

Form PCT/ISA/210 (second sheet) (July 2022)

**INTERNATIONAL SEARCH REPORT**
**Information on patent family members**

International application No.
**PCT/JP2024/023458**

| Patent document cited in search report | Publication date (day/month/year) | Patent family member(s) | Publication date (day/month/year) |
|---|---|---|---|
| JP 2017-502008 A | 19 January 2017 | US 2016/0310541 A1<br>claims, examples<br>WO 2015/086550 A1<br>CN 105813630 A | |
| WO 2022/092198 A1 | 05 May 2022 | US 2023/0414832 A1<br>claims, paragraphs [0043], [0057]-[0058], [0074], [0095]-[0096], [0128]-[0129], [0138]-[0140], fig. 3, 8<br>EP 4238591 A1<br>CN 116710155 A | |
| JP 2003-320227 A | 11 November 2003 | (Family: none) | |
| JP 2000-516637 A | 12 December 2000 | WO 1998/053802 A1<br>claims<br>US 6004582 A<br>CN 1228020 A | |
| JP 2018-515582 A | 14 June 2018 | US 2016/0346197 A1<br>claims, paragraph [0114]<br>WO 2016/191645 A1<br>EP 3662923 A1 | |
| JP 2013-537820 A | 07 October 2013 | US 2013/0261543 A1<br>drawings<br>WO 2012/041320 A1<br>CN 103228229 A | |
| JP 54-57476 A | 09 May 1979 | (Family: none) | |
| JP 2021-527527 A | 14 October 2021 | US 2021/0128785 A1<br>claims<br>WO 2019/246416 A1<br>CN 112566587 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022502458 W
- JP 2019097442 A
- WO 2016184872 A
- JP 2017502008 W
- JP 2018515582 W
- JP 2014200199 A
- JP 2004209228 A
- WO 2022092198 A
- WO 2020262642 A
- WO 2022137345 A
- JP 2019023478 W
- JP 2020047100 W
- JP 2019007655 W